# EUROPEAN PATENT APPLICATION

(11) **EP 4 428 291 A1**
(43) Date of publication of application: **11.09.2024**
(21) Application number: 22890365.4
(22) Date of filing: 02.11.2022
(51) Int. Cl.: D06F 58/12, D06F 58/20, D06F 58/26, D06F 73/02, A47F 7/19, E05D 7/082, E05F 15/611

(54) **CLOTHING TREATMENT APPARATUS**

(30) Priority: 02.11.2021 KR 20210148504; 26.09.2022 KR 20220121808; 01.11.2022 KR 20220143996
(71) Applicant: LG Electronics Inc., Seoul 07336 (KR)
(72) Inventor: KIM, Daewoong, Seoul 08592 (KR); LIM, Jaemyung, Seoul 08592 (KR); CHOI, Jeongguen, Seoul 08592 (KR); OH, Minkyu, Seoul 08592 (KR); JO, Yeona, Seoul 08592 (KR); JO, Yuna, Seoul 08592 (KR); KIM, Hyunki, Seoul 08592 (KR); HWANG, Sung Gul, Seoul 08592 (KR)
(74) Representative: Vossius & Partner Patentanwälte Rechtsanwälte mbB
(86) International application number: PCT/KR2022/017033
(87) International publication number: WO 2023/080634

(57) **Abstract**

The present disclosure relates to a clothing treatment apparatus comprising: a cabinet including an inlet on the front thereof; a first chamber located inside the cabinet and accommodating clothes or articles through the inlet; a hanger body unit provided inside the first chamber, extending along the front and rear directions of the cabinet, and holding the clothes; and a hanger support unit positioned above the hanger body unit to movably support the hanger body unit, wherein the arrangement of the hanger body unit and the hanger support unit may be transformed into a first arrangement in which both ends of the hanger body unit and the hanger support unit are coupled to match each other, a second arrangement in which the hanger body unit is separated from the hanger support unit and is moved forward by a predetermined distance, and a third arrangement in which the hanger body unit and the hanger support unit are vertically arranged by rotating the hanger body unit with respect to the hanger support unit.

## Description

### [Technical Field]

The present disclosure relates to a laundry treating apparatus. Specifically, the present disclosure relates to a laundry support disposed in a space where laundry or goods are accommodated.

### [Background]

A laundry treating apparatus refers to an apparatus developed for washing and drying laundry at home and in a laundry, and for removing wrinkles on the laundry. What is classified as the laundry treating apparatus includes a washing machine that washes the laundry, a dryer that dries the laundry, a washing machine/dryer that has both a washing function and a drying function, a clothes care apparatus that refreshes the laundry, a steamer that removes the wrinkles from the laundry, and the like.

In particular, the clothes care apparatus is an apparatus that helps keep the laundry fresh and clean. The clothes care apparatus may remove fine dust attached to the laundry, deodorize the laundry, dry the laundry, and add fragrance to the laundry. In addition, generation of static electricity may be prevented, wrinkles may be removed from the laundry using dehumidified air or steam, and the laundry may be sterilized.

Korean Patent Application Publication No. 10-2014-0108454 discloses a typical laundry treating apparatus. That is, in a case of the typical laundry treating apparatus, a treatment room in which the laundry is accommodated and cared for using steam and hot air is formed inside a cabinet, and a hanger bar where the laundry is hung extends in a left and right direction inside the treatment room. A machinery room containing mechanical devices necessary for the laundry caring may be located under the treatment room and may supply hot air and steam to the treatment room. Additionally, when the laundry is hung on the hanger bar using a hanger, the laundry may be disposed in a front and rear direction of the cabinet. Additionally, the treatment room may be equipped with a shelf where a bag, a hat, and other goods that require care may be mounted.

However, this is an apparatus that focuses on simply caring for the laundry. Therefore, there is no separate space to display or store laundry inside. Additionally, using the treatment room may not also be energy efficient when an amount of laundry to be cared for is small. In one example, when a front surface of the door is made of a transparent material such that a user may check the laundry hung inside, an insulation performance via the front surface of the door is reduced, making it difficult to achieve the purpose of laundry caring.

In addition, because the hanger bar may only move in the left and right direction from a fixed location, there may be inconvenience in the user having to reach inside the treatment room to hang the laundry when hanging the laundry.

In addition, with a development of clothes and accessories made of various materials, there may be a need for a space to separately store and display laundry that require delicate care, such as a leather product or a silk product, or a separate space where temperature and humidity may be adjusted on a regular basis to store and display items such as the bag or the hat.

Korean Patent Application Publication No. 10-2019-0141286 discloses a laundry treating apparatus that separately has the treatment room for accommodating therein and caring for the laundry and a storage room where laundry that has been cared for is stored and the temperature is adjustable. However, there is a problem in that the laundry treating apparatus takes up a great amount of space indoors because the treatment room and the storage room are arranged in the left and right direction. In addition, because an air conditioner for the storage room is not disposed separately, but an air conditioner for the treatment room is used in the storage room in a connected manner, independent temperature and humidity adjustment only for the laundry stored in the storage room is not available during use of the treatment room.

In one example, Korean Patent Application Publication No. 10-2019-0139400 discloses a laundry treating apparatus with the treatment room and the storage room divided from each other in the front and rear direction, but a laundry caring function is equipped only in the treatment room and there is no separate mechanical device to manage the temperature and the humidity of the storage room. When the laundry that has been treated or cared for is simply stored in the storage room, the stored laundry may be affected by changes in surrounding temperature and humidity. For example, when the laundry is stored without the temperature and humidity adjustment, a moisture content of the laundry may increase and the laundry may become damp, or surrounding odors may be absorbed again. To solve such problem, an apparatus to manage the temperature and the humidity of the laundry even during the storage is needed.

In addition, Korean Patent Application Publication No. 10-2019-0139400 discloses a laundry treating apparatus in which the treatment room on a front side and the storage room on a rear side are separated from each other and the treatment room is hinged to a side surface of the storage room. However, considering an usage environment in which the laundry treating apparatus is used, a size of the laundry treating apparatus has no choice but to consider a standard size of furniture such as a typical closet. This is because the laundry treating apparatus is disposed next to or adjacent to the closet rather than being disposed alone indoors, away from the closet. Therefore, when the treatment room and the storage room are coupled to each other in a form similar to the hinge coupling of the typical door, durability of the hinge may be a problem because of a size of the treatment room, and interference may occur with the furniture located near when the treatment room pivots relative to the storage room. To solve such problem, the laundry treating apparatus should protrude forward as much as the size of the treatment room to resolve the interference between the surrounding furniture and the hinge. However, when the laundry treating apparatus protrudes more than other furniture, sense of unity with the surrounding furniture may be ruined.

Additionally, a new hinge structure that may withstand loads of the structure for the display room and the structure for supporting the display room may be needed.

In one example, Korean Patent No. 10-2043197 shows a hanger bar disposed in a width direction to hang laundry inside. In a case of the hanger bar, there may be an inconvenience of having to reach inside to hang the laundry.

Additionally, in Korean Patent No. 10-2043197, an air discharge port and an air intake port are arranged in a front and rear direction of the cabinet. However, when a size of the treatment room that accommodates the laundry therein becomes small, there may be cases in which the laundry is not able to be hung along the width direction of the cabinet. Even in this case, when the air discharge port and the air intake port are arranged in the front and rear direction of the cabinet, air discharged from the air discharge port may not be supplied uniformly depending on a location where the laundry is hung.

### [Summary]

### [Technical Problem]

First, the present disclosure is to provide a laundry treating apparatus with a space divided into a first chamber and a second chamber for caring of laundry or goods.

Second, the present disclosure is to provide the second chamber where temperature and humidity may be adjusted independently of the first chamber where temperature and humidity may be adjusted for sterilization, deodorization, drying, and wrinkle removal of laundry or goods.

Third, the present disclosure is to provide a hinge structure that may prevent interference with adjacent furniture when opening and closing a door assembly containing a second chamber, considering a size of the second chamber.

Fourth, the present disclosure is to provide a laundry treating apparatus where a hanger bar is disposed and an air circulation direction based on the hanger bar is set, considering a size of a space of the first chamber with a length in a front and rear direction reduced by the second chamber.

Fifth, the present disclosure is to provide a door assembly that may be automatically withdrawn from a cabinet by sensing opening of the door assembly by a user.

Sixth, the present disclosure is to enable check of laundry or goods accommodated in the second chamber via a front surface of the door assembly.

Seventh, the present disclosure is to allow illuminance and color of lighting that emits light toward the second chamber to be changed depending on laundry or goods.

Eighth, the present disclosure is to provide the first chamber that executes a course to care for laundry by receiving steam or hot air based on the course selected by the user, and the second chamber that constantly monitors inside temperature and humidity for laundry accommodated inside based on user's settings.

Ninth, the present disclosure is to provide a hanger body to improve user convenience.

Tenth, the present disclosure is to uniformly supply air or steam to laundry by changing an arrangement of an air discharge port, an air intake port, and a steam discharge port based on a change in an arrangement direction of a hanger body.

### [Technical Solutions]

To solve the above-mentioned problems, provided is a laundry treating apparatus that simultaneously has a styling area for caring for laundry and a showcase area where the laundry may be stored and displayed while being constantly cared for. By dividing the styling area and the showcase area from each other, a front surface of the showcase area may be made of a transparent material, allowing interior check, while maintaining an insulation performance of the styling area.

Reflecting a size of the styling area that is reduced because of the showcase area, a hanger bar of the laundry treating apparatus may be disposed in a front and rear direction. On the other hand, an air circulation direction in a first chamber of the laundry treating apparatus may be a left and right direction.

Independently of air circulation and steam spray in the styling area, the laundry treating apparatus may include an air circulator and an air treater to adjust temperature and humidity in the showcase area.

The laundry treating apparatus may include a lighting device whose intensity and color may be adjusted to suit the laundry or items accommodated in the showcase area.

Considering a size of the showcase area, a door assembly including the showcase may be automatically extended and then slid. In addition, a separate door accessing the showcase may use a push & pull method.

More specifically, to solve above-mentioned problems, provided is a laundry treating apparatus including a cabinet including a first inlet defined in a front surface thereof, a first chamber that is located inside the cabinet and accommodates laundry or goods therein via the first inlet, an auxiliary chamber located at a lower side inside the cabinet and defining therein a space separated from the first chamber, a door assembly that opens and closes the first inlet, and a second chamber located inside the door assembly and defining therein a space separated from the first chamber and the auxiliary chamber to accommodate the laundry or the goods therein, and the first chamber includes a first area located on the auxiliary chamber; and a second area located in front of the auxiliary chamber and accommodating at least a portion of the second chamber therein when the door assembly closes the first inlet.

Along a front and rear direction of the cabinet, a front-and-rear length of the second area may be equal to or smaller than a front-and-rear length of the first area.

The laundry treating apparatus may further include a first chamber bottom surface forming a bottom surface of the first chamber, and the bottom surface of the first chamber may be formed to be stepped along the front and rear direction of the cabinet.

The first chamber bottom surface may include a first bottom surface forming a bottom surface of the first area, and a second bottom surface forming a bottom surface of the second area, and a vertical level of the first bottom surface may be higher than a vertical level of the second bottom surface.

The second bottom surface may face at least a portion of a lower portion of the door assembly when the door assembly closes the first inlet.

The door assembly may include an accommodating body including a second inlet defined in a front surface thereof to allow the second chamber formed therein to be in communication with the outside, and a door of the second chamber that opens and closes the second inlet, and at least a portion of the accommodating body may be accommodated in the first area when the door assembly closes the first inlet.

At least a portion of the door of the second chamber may be made of a material that allows visible light to pass therethrough.

A width of the accommodating body may decrease rearwards from the second inlet. That is, the accommodating body may have a tapered shape.

The laundry treating apparatus may further include a first hinge pivotably coupling the door assembly to the cabinet, and a second hinge pivotably coupling the door of the second chamber to the accommodating body.

The first hinge may include a plurality of first hinges.

The first hinge may include a first upper hinge located at an upper portion of the first chamber; and a first lower hinge located at a lower portion of the first chamber, a portion of each of the first upper hinge and the first lower hinge may be located spaced apart from both side surfaces of the first chamber, another portion of each of the first upper hinge and the first lower hinge may be pivotably coupled to a rear surface of the accommodating body so as to be spaced apart from both side surfaces of the accommodating body, and the first hinge may extend the door assembly forward of the cabinet and then move the door assembly along a width direction of the cabinet and pivot the door assembly to open the first inlet.

The second hinge may include a plurality of second hinges, and one end of each of the plurality of second hinges may be located in the accommodating body and the other end of each of the plurality of second hinges may be located on an inner surface of the door of the chamber facing the second inlet of the door of the second chamber, so that the second hinge may pivot the door such that an angle between the second inlet and the door of the second chamber increases or decreases.

The first hinge may include a first link and a second link that are pivotably coupled to the cabinet and the accommodating body, but are positioned spaced apart from each other.

The first link and the second link may move the door assembly parallel to the first inlet when opening and closing the first inlet

The first hinge may move the door assembly forward away from the first inlet via the pivoting of the first link and the second link, and then move the door assembly parallel to the first inlet.

The laundry treating apparatus may further include an opening/closing driver that is located on an inner surface of the first chamber and pushes the door assembly to separate the door assembly from the first inlet.

The opening/closing driver may include an opening/closing body that forms an outer appearance, an opening/closing motor disposed inside the opening/closing body, and an opening/closing link that is connected to the opening/closing motor and pushes the door assembly in a direction away from the first inlet when the opening/closing motor rotates.

The laundry treating apparatus may further include an air supply that is located inside the auxiliary chamber and circulates air in the first chamber, an air intake port located on a bottom surface of the first chamber, in communication with the first chamber, and sucking air from the first chamber into the air supply, and an air discharge port located on the bottom surface of the first chamber, in communication with the first chamber, and discharging air that has passed through the air supply into the first chamber.

The laundry treating apparatus may further include a first bottom surface forming a bottom surface of the first area, and a second bottom surface forming a bottom surface of the second area, and the air intake port and the air discharge port may be located on the first bottom surface.

The air intake port and the air discharge port may be disposed along the width direction of the cabinet.

The laundry treating apparatus may further include a steam supply that is located in the auxiliary chamber and generates and supplies steam to the first chamber, and a steam discharge port that is located at a bottom of the first chamber and discharges steam into the first chamber.

The steam discharge port may be located closer to the air discharge port than to the air intake port.

The laundry treating apparatus may further include a water supply tank that supplies water for generating the steam in the steam supply, and a drain tank that stores condensate generated in the air supply and the first chamber, and the water supply tank and the drain tank may be detachably inserted into the auxiliary chamber via a front surface of the auxiliary chamber.

Lengths of the water supply tank and the drain tank along the front and rear direction of the cabinet may be greater than lengths thereof along the height direction of the cabinet.

The air supply may further include a heat exchanger for dehumidifying and heating circulating air of the first chamber, and the water supply tank and the drain tank may be located above the heat exchanger.

The laundry treating apparatus may further include a hanger body that is located at an upper side inside the first chamber and hangs the laundry thereon, and the hanger body may extend along the front and rear direction of the cabinet.

The laundry treating apparatus may further include an air supply that is located in the auxiliary chamber and circulates air in the first chamber, an air intake port located at a lower portion of the first chamber, in communication with the first chamber, and sucking air from the first chamber into the air supply, and an air discharge port located at a lower portion of the first chamber, in communication with the first chamber, and discharging air that has passed through the air supply into the first chamber, and the air intake port and the air discharge port may be disposed to be spaced apart from each other in the width direction of the cabinet.

The laundry treating apparatus may further include a hanger support that supports the hanger body movably in the front and rear direction.

The laundry treating apparatus may further include a hanger bar fixing groove defined in one of the hanger support and the hanger body, and a movement preventing protrusion disposed on the other of the hanger support and the hanger body and insertable into the hanger bar fixing groove depending on whether the door assembly is opened.

The laundry treating apparatus may further include a laundry driver that generates a rotational force for a reciprocating movement of the hanger body, and a power converter connected to the laundry driver to cause the reciprocating movement of the hanger body along the front and rear direction.

The laundry treating apparatus may further include a hanger mounting portion for hanging the laundry accommodated in the second chamber, and a direction of the laundry hung on the hanger mounting portion may be the same as a direction of the laundry hung on the hanger body.

The laundry treating apparatus may further include an air circulator located inside the accommodating body to circulate air in the second chamber.

The laundry treating apparatus may further include an air treater located inside the accommodating body to circulate air in the second chamber.

The air circulator and the air treater may be operated even when the door assembly is opened.

The laundry treating apparatus may further include an air supply disposed inside the auxiliary chamber to circulate air in the first chamber, and the air circulator and the air treater may be operated independently of the operation of the air supply.

The air circulator may include a heater that heats circulating air of the second chamber to adjust a temperature of the second chamber.

The air treater may include a dehumidifier to dehumidify air circulating through the air treater.

In one example, the door assembly may further include a shelf detachably disposed in the second chamber and supporting the laundry or the goods accommodated in the second chamber.

In one example, a laundry treating apparatus includes a cabinet including a first inlet defined in a front surface thereof, a first chamber that is located inside the cabinet and accommodates laundry or goods therein via the first inlet, an auxiliary chamber located at a lower side inside the cabinet and defining a space separated from the first chamber therein, and a door assembly that opens and closes the first inlet and includes a second chamber that accommodates laundry or goods therein, and at least a portion of the second chamber is accommodated in a space defined between the first inlet and the auxiliary chamber when the door assembly closes the first inlet.

In one example, a laundry treating apparatus includes a cabinet including a first inlet defined in a front surface thereof, a first chamber that is located inside the cabinet and accommodates laundry or goods therein via the first inlet, an auxiliary chamber located at a lower side inside the cabinet and defining a space separated from the first chamber therein, a door assembly including an accommodating body including a second inlet defined in a front surface thereof and forming a second chamber therein, and a door of the second chamber that opens and closes the second inlet, wherein the door assembly opens and closes the first inlet, a first hinge that separates the door assembly from the first chamber and moves the door assembly along a width direction of the cabinet to open the first inlet, and a second hinge that moves the door of the second chamber such that an angle between the door of the second chamber and a front surface of the accommodating body increases to open the second inlet.

Further, at least a portion of the second chamber may be located in front of the auxiliary chamber inside the first chamber when the door assembly closes the first inlet.

In one example, a laundry treating apparatus includes a cabinet including a first inlet defined in a front surface thereof, a first chamber that is located inside the cabinet and accommodates laundry therein via the first inlet, an auxiliary chamber located at a lower side inside the cabinet and defining an installation space separated from the first chamber therein, a door assembly that opens and closes the first inlet, and a first hinge connecting the door assembly to the cabinet, the door assembly includes an accommodating body including a second inlet defined in a front surface thereof and at least partially accommodated inside the first chamber when the door assembly is closed, a second chamber located inside the accommodating body, formed separately from the first chamber and the auxiliary chamber, and in communication with the outside via the second inlet, a door of the second chamber that opens and closes the second inlet, and a second hinge connecting the door of the second chamber to the accommodating body, the first hinge pivots to open the first inlet by extending the door assembly forward of the cabinet and then moving the door assembly along a width direction of the cabinet, and the second hinge pivots to open the second inlet via pivoting of the door of the second chamber.

The water supply tank and the drain tank may be located upwardly of the air supply or the steam supply.

In one example, a laundry treating apparatus includes a cabinet including a first inlet defined in a front surface thereof, a first chamber that is located inside the cabinet and accommodates laundry therein via the first inlet, an auxiliary chamber located at a lower side inside the cabinet and defining an installation space separated from the first chamber therein, and a door assembly coupled to the cabinet to open and close the first inlet, and the door assembly includes an accommodating body including a second inlet defined in a front surface thereof and at least partially accommodated inside the first chamber when the door assembly is closed, a second chamber located inside the accommodating body, formed separately from the first chamber and the auxiliary chamber, and in communication via the second inlet, and a door of the second chamber that opens and closes the second inlet.

In addition, the laundry treating apparatus may further include a first hinge that pivots such that the door assembly moves along the width direction of the cabinet after being extended forward of the cabinet when the door assembly is opened, and a second hinge that pivots to open the second inlet via pivoting of the door of the second chamber when the door of the second chamber is opened.

When the door assembly is closed, the door assembly may be moved along the width direction of the cabinet to face the first inlet, and then the first inlet may be closed.

Additionally, the present disclosure provides a hanger body that is disposed in the front and rear direction of the cabinet and is extendable forward. Additionally, the present disclosure provides a laundry treating apparatus that may rotate the extended hanger body to place the hanger body in the width direction of the cabinet.

More specifically, provided is a laundry treating apparatus including a cabinet including an inlet defined in a front surface thereof, a door that opens and closes the inlet, a first chamber that is located inside the cabinet and accommodates laundry or goods therein via the inlet, a hanger body that is disposed inside the first chamber, is extending along a front and rear direction of the cabinet, and hangs the laundry thereon, and a hanger support extending parallel to the hanger body along the front and rear direction of the cabinet and supporting the hanger body to be movable along the front and rear direction of the cabinet.

The laundry treating apparatus may further include a movement guide disposed on the hanger body and connecting the hanger body with the hanger support such that the hanger body is able to be separated from the hanger support and move forward or rotate relative to the hanger support.

The movement guide may be rotatable relative to the hanger support such that the hanger body is separated from the hanger support, moved forward, and then disposed along the width direction of the cabinet.

When separated from the hanger support and moving forward, one end of the hanger body may be located between both ends of the hanger support, and the other end of the hanger body may be located forward of both ends of the hanger support.

The hanger support may include a hanger support body extending in the front and rear direction of the cabinet to support the hanger body, a first support and a second support supporting both ends of the hanger support body, and a hanger body guide extending from the hanger support body along the front and rear direction of the cabinet and defined in a shape of a groove or a hole to move the hanger body from the hanger support in the front and rear direction of the cabinet.

The hanger body may include a movement guide disposed on the hanger body and connected to the hanger body and the hanger support body such that the hanger body is movable on the hanger support body.

The movement guide may be formed in a cylindrical shape, and the hanger body may move along the hanger body guide by the movement guide or rotate relative to the hanger support body by the movement guide.

The hanger body guide may further include a hanger body guide hole extending through the hanger support body and extending along the front and rear direction of the cabinet, wherein the movement guide is inserted into the hanger body guide hole, and a coupling guide member rotatably accommodating the movement guide therein, the hanger body guide hole may extend along the front and rear direction of the cabinet such that the movement guide may move between a first hanger location on the hanger support body and a second hanger location on the hanger support body located ahead of the first hanger location along the hanger body guide hole, and the coupling guide member may be disposed at the second hanger location.

When the movement guide is located at the second hanger location, one end among both ends of the hanger body may be located between the first support and the second support.

The hanger body guide may further include a hanger bar guide member coupled to the hanger support body, and a hanger body movement hole that extends through the hanger bar guide member and overlaps the hanger body guide hole to enable movement of the hanger body between the first hanger location and the second hanger location, and the coupling guide member may be located on the hanger bar guide member.

The coupling guide member may protrude from the hanger bar guide member and may be opened along the front and rear direction of the cabinet.

The coupling guide member may rotatably accommodate the movement guide therein.

In one example, the laundry treating apparatus may further include a fixing portion disposed on at least one of the hanger support and the hanger body to detachably couple the hanger support with the hanger body.

The fixing portion may include a hanger bar fixing groove defined in one of the hanger support and the hanger body, and a movement protrusion disposed on the other one of the hanger support and the hanger body and insertable into the hanger bar fixing groove depending on whether the door is opened.

The movement preventing protrusion may be inserted into or separated from the hanger bar fixing groove depending on whether the door is opened or closed.

The fixing portion may include a hook disposed at one of a front end of the hanger body and a front end of the hanger support, and a hook coupling portion disposed at the other of the front end of the hanger body and the front end of the hanger support to be coupled with the hook.

The fixing portion may further include a hook accommodating groove defined by recessing a portion of the front end of the hanger body and a portion of the front end of the hanger support together, and the hook and the hook coupling portion may be accommodated in the hook accommodating groove when the hook is coupled to the hook coupling portion.

The hanger support may include a hanger support body that supports the hanger body and extends in the front and rear direction of the cabinet, and the hanger body may include a hanger bar that may be disposed in the front and rear direction of the cabinet to hang the laundry.

The hanger body may include a hanger bar that hangs the laundry thereon, and a plurality of hanger grooves defined in the hanger bar.

In one example, the laundry treating apparatus may further include a motor that generates a rotational force, and a power converter connected to the motor to allow the hanger body to perform a reciprocating movement along the front and rear direction.

The laundry treating apparatus may further include a driver frame supporting the motor, and the motor and the driver frame may be located between a first chamber top surface forming a top surface of the first chamber and a cabinet top surface forming a top surface of the cabinet.

The power converter may further include a rotatable portion that rotates away from a rotation shaft of the motor, and a converting member connected to the rotatable portion and extending in the width direction of the cabinet to allow the hanger support to perform the reciprocate movement along the front and rear direction of the cabinet.

The converting member may be disposed on a top surface of the hanger support, and at least a portion of the rotatable portion may extend through the driver frame and the first chamber top surface and be inserted into the first chamber to be connected to the converting member.

In one example, the laundry treating apparatus may further include an auxiliary chamber disposed beneath and separated from the first chamber, an air supply that is located inside the auxiliary chamber and circulates air in the first chamber, an air intake port that is located on a bottom surface of the first chamber, is in communication with the first chamber, and sucks air in the first chamber into the air supply, and an air discharge port that is located on the bottom surface of the first chamber, is in communication with the first chamber, and discharges air that has passed through the air supply into the first chamber.

The air intake port and the air discharge port may be arranged along the width direction of the cabinet.

In one example, the hanger body may include a hanger bar that includes a plurality of hanger grooves and extends in the front and rear direction of the cabinet, an upper hanger body spaced upwardly apart from the hanger bar and disposed in parallel with the hanger bar, and a connecting body connecting each of both ends of the upper hanger body with each of both ends of the hanger bar.

The hanger support may further include a hanger support body extending in the front and rear direction of the cabinet to support the hanger body, and a first support and a second support that support both ends of the hanger support body.

The hanger support may include a moving rail located on a bottom surface of the hanger support body to move the hanger body forward, and the hanger body may include a moving guider disposed on the upper hanger body to move the hanger body along the moving rail.

In one example, a laundry treating apparatus includes a cabinet including an inlet defined in a front surface thereof, a first chamber that is located inside the cabinet and accommodates laundry or goods therein via the inlet, a hanger body that is disposed inside the first chamber, is extending along a front and rear direction of the cabinet, and hangs the laundry thereon, and a hanger support extending parallel to the hanger body along the front and rear direction of the cabinet and supporting the hanger body to be movable, and an arrangement of the hanger body and the hanger support is switchable into a first arrangement where the hanger body and the hanger support are coupled to each other such that each of both ends of the hanger body and each of both ends of the hanger support coincide with each other, a second arrangement for separating the hanger body from the hanger support and moving the hanger body forward by a predetermined distance, and a third arrangement for rotating the hanger body relative to the hanger support to arrange the hanger body and the hanger support perpendicular to each other.

The laundry treating apparatus may further include a door that is pivotably disposed on the cabinet and opens and closes the inlet, the hanger body and the hanger support may be arranged in the first arrangement when the door is closed, and the arrangement of the hanger body and the hanger support may be switched from the first arrangement to the second arrangement and the third arrangement when the door is opened.

### [Advantageous Effects]

First, the present disclosure may provide the laundry treating apparatus with the space divided into the first chamber that accommodates the laundry or the goods therein and the second chamber that accommodates the laundry or the goods therein separately from the first chamber.

Second, the present disclosure may independently adjust the temperature and the humidity of the first chamber and the temperature and the humidity of the second chamber independently of each other.

Third, the present disclosure may prevent the interference with the adjacent furniture when the door assembly is opened and closed considering the size of the second chamber disposed inside the door assembly.

Fourth, the present disclosure may provide the laundry treating apparatus in which the air circulation direction is set based on the arrangement of the hanger bar where the laundry is hung and the arrangement direction of the hanger bar, taking into account the reduction of the space for accommodating the laundry in the first chamber.

Fifth, the present disclosure may provide the door assembly that may be automatically withdrawn from the cabinet by sensing the opening of the door assembly by the user.

Sixth, the present disclosure may check the laundry or the items accommodated in the second chamber via the front surface of the door assembly.

Seventh, the present disclosure may change the illuminance and the color of the lighting installed in the second chamber to suit the laundry or the goods.

Eighth, the present disclosure may provide the first chamber that performs the course to care for the laundry by receiving steam or hot air based on the course selected by the user, and the second chamber that constantly monitors the internal temperature and humidity of the laundry stored inside based on the settings of the user.

Ninth, the present disclosure may improve the user convenience when hanging the laundry.

Tenth, the present disclosure may uniformly supply air or steam to the laundry hung on the hanger body.

### [Brief Description of the Drawings]

FIG. 1 shows an example of an existing laundry treating apparatus.
FIG. 2 shows a laundry treating apparatus described in the present disclosure.
FIG. 3 shows a first chamber and a second chamber with a door assembly open.
(a) in FIG. 4 is an example of a mechanical device disposed inside an auxiliary chamber, (b) in FIG. 4 shows an arrangement of a compressor included in a mechanical device.
(a) in FIG. 5 is an example of an opening/closing driver and an inputter/outputter that help open a door assembly. (b) in FIG. 6 schematically shows locations of a first chamber, a second chamber, and an auxiliary chamber inside a cabinet.
(a) in FIG. 6 shows states of an opening/closing driver and a first hinge when a door assembly is closed. (b) in FIG. 6 shows states of an opening/closing driver and a first hinge when a door assembly is opened.
(a) and (b) in FIG. 7 show a problem that occurs when using a hinge coupled to one side surface of a door assembly.
FIG. 8 shows a door of a second chamber being opened with a door assembly open.
FIG. 9 shows a hanger assembly and a shelf coupling portion disposed in a second chamber.
(a) in FIG. 10 is a state of a first hinge when a door assembly is closed. (b) in FIG. 10 shows a state of a first hinge when a door assembly is opened. (c) in FIG. 10 is another example of a first hinge used when opening and closing a door assembly at the other side surface.
FIG. 11 is an example of a second hinge.
(a) in FIG. 12 is a state of a second hinge when a door of a second chamber is closed. (b) in FIG. 12 is a state of a second hinge when a door of a second chamber is opened. (c) in FIG. 12 is an exploded view of a second hinge.
FIG. 13 schematically shows a flow direction of air inside a first chamber.
FIG. 14 shows a laundry support disassembled.
FIG. 15 shows a hanger body being extended forward of a hanger support.
(a) in FIG. 16 shows one cross-section of a laundry support. (b) in FIG. 16 shows a hanger body being fixed by inserting a movement preventing protrusion of a hanger bar stopper into a hanger bar fixing groove. (c) in FIG. 16 shows a movement preventing protrusion separated from a hanger bar fixing groove.
(a) in FIG. 17 is a front view of a door assembly. (b) in FIG. 17 shows a door of a second chamber open.
FIG. 18 is another example of a laundry hanger that may be mounted in a second chamber.
FIG. 19 is an example of a second chamber top surface.
FIG. 20 is a view of an upper portion of an accommodating body cut along a height direction of an accommodating body.
FIG. 21 is an example of an air circulator.
FIG. 22 is an example of a second chamber bottom surface.
FIG. 23 is a cross-section of a lower portion of an accommodating body cut along a height direction of the accommodating body.
FIG. 24 is a cross-section of a lower portion of an accommodating body cut along a width direction of an accommodating body.
(a) in FIG. 25 is an example of a hanger assembly and a shelf connected to a second chamber, (b) in FIG. 25 is an example of a shelf.
FIG. 26 is a disassembled shelf.
FIG. 27 shows an example of a line lighting.
FIG. 28 is a cross-section of an accommodating body cut along a front and rear direction of the accommodating body.
(a) in FIG. 29 is an example of an upper lighting installed in a second chamber top surface. (b) in FIG. 29 is an example of a lower lighting of a second chamber bottom surface.
FIG. 30 is an example of a first rear line lighting and a third rear line lighting of a rear line lighting.
FIG. 31 is an example of a front line lighting and a rear line lighting.
(a) to (c) in FIG. 32 are an example of heat dissipating and moisture proof structures disposed in a lighting.
(a) to (c) in FIG. 33 are another example of heat dissipating and moisture proof structures disposed in a lighting.
(a) and (b) in FIG. 34 are another example of a laundry treating apparatus described in the present disclosure.

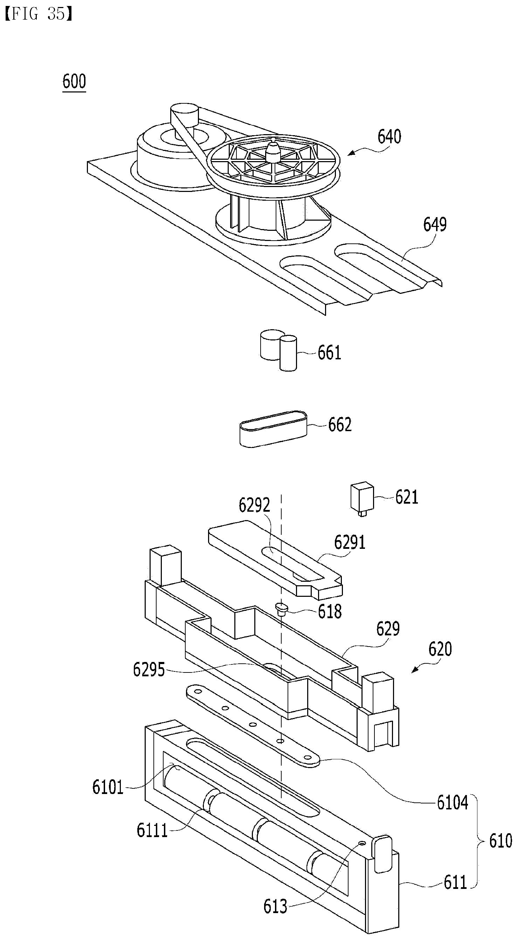

FIG. 35 is an exploded view of another example of a laundry support.

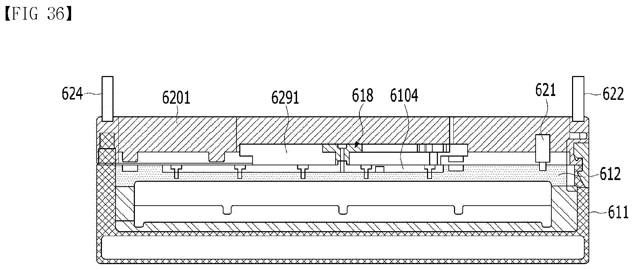

FIG. 36 shows a cross-section of a laundry support from one side.

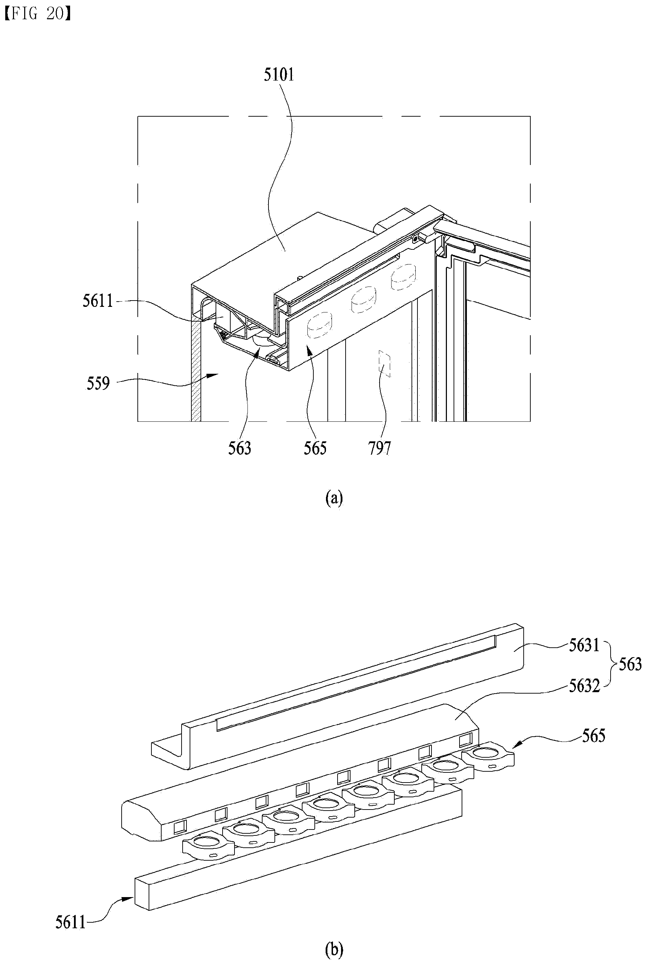

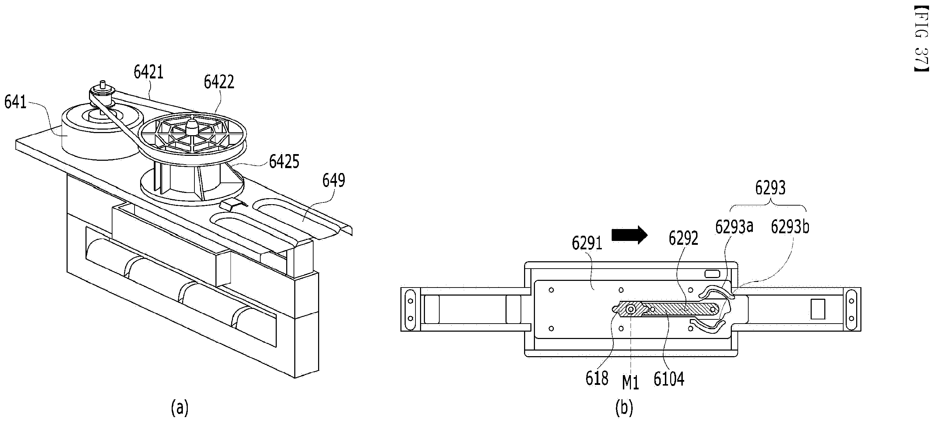

(a) in FIG. 37 shows an example in which a hanger body is fixed to a hanger support and accommodated inside a first chamber, (b) in FIG. 37 is a top view of a state in (a) in FIG. 20.

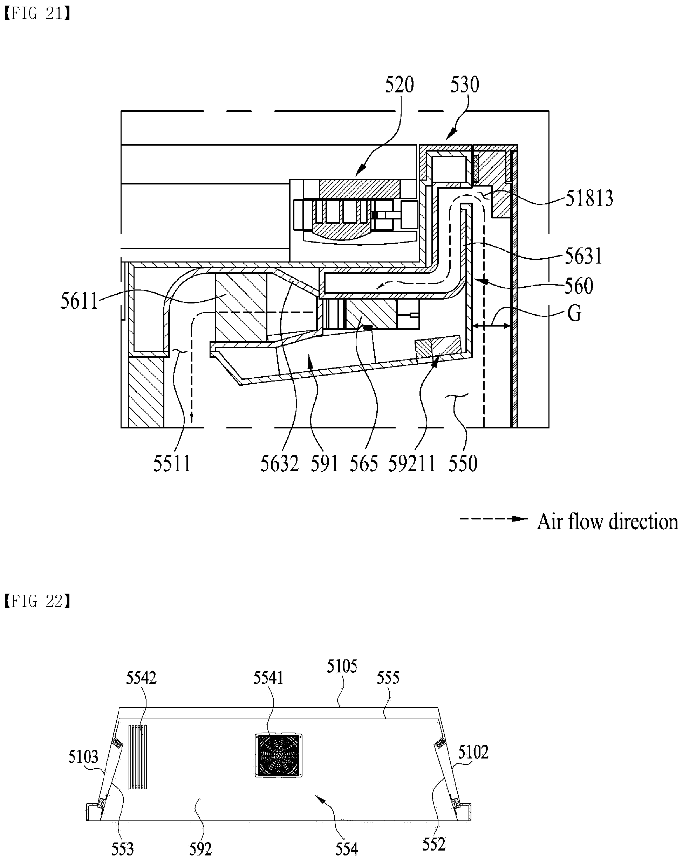

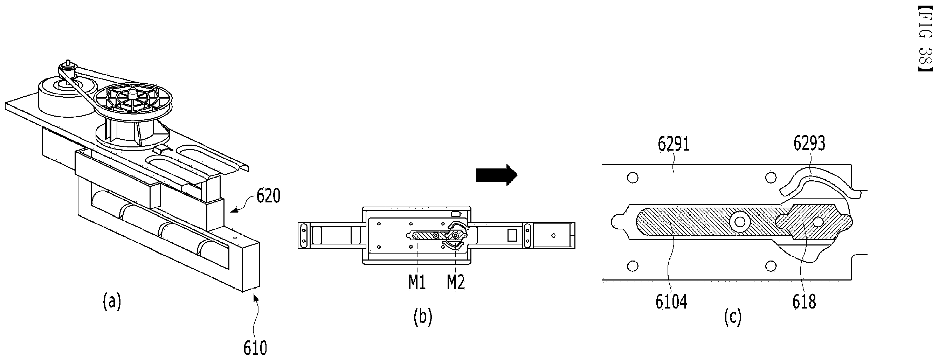

(a) in FIG. 38 shows an example in which a hanger body is extended forward from a hanger support. (b) in FIG. 38 is a top view of a state in (a) in FIG. 21. (c) in FIG. 38 is an enlarged view of a coupling guide member and a movement guide in a state in (a) in FIG. 38.

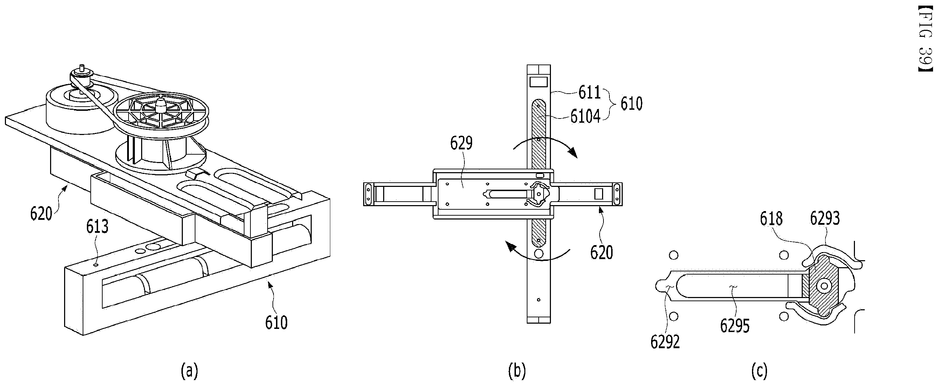

(a) in FIG. 39 shows an example in which a hanger body that has been extended forward is rotated. (b) in FIG. 39 is a top view of a state in (a) in FIG. 39. (c) in FIG. 39 is an enlarged view of a coupling guide member and a movement guide in a state in (a) in FIG. 39.

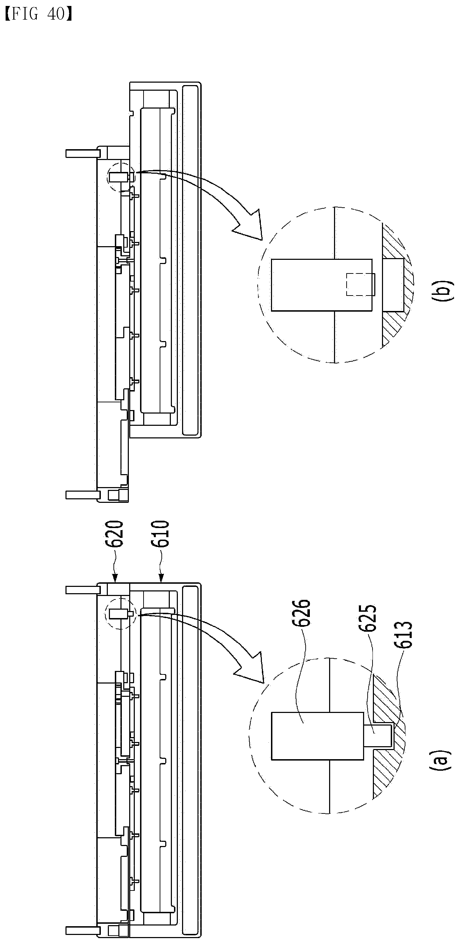

(a) in FIG. 40 shows an example in which a hanger body is fixed as a movement preventing protrusion of a hanger bar stopper is inserted into a hanger bar fixing groove. (b) in FIG. 40 is an example in which a movement preventing protrusion is separated from a hanger bar fixing groove.

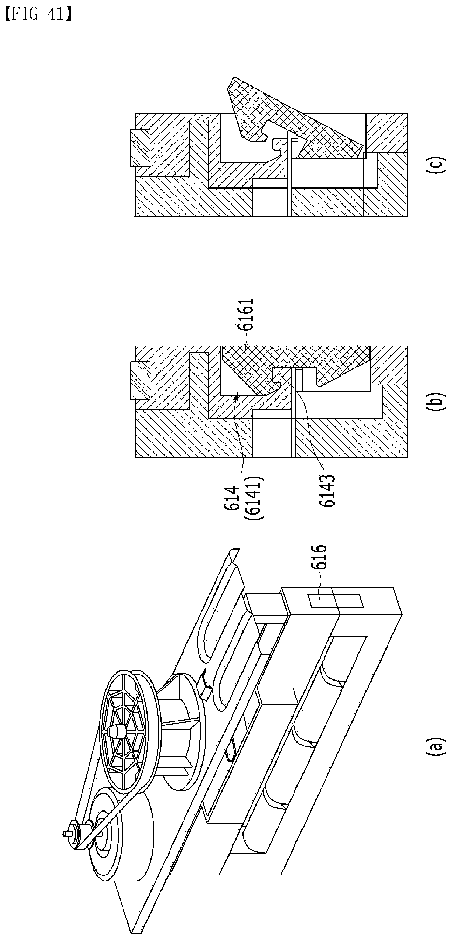

(a) in FIG. 41 is an example of a fixing hook that couples a hanger body with a hanger support. (b) in FIG. 41 is an example of a fixing hook being coupled. (c) in FIG. 41 is an example of a fixing hook separated.

### [Best Mode]

Hereinafter, a preferred embodiment of the present disclosure will be described in detail with reference to the accompanying drawings. A configuration of a device or a method for controlling the same to be described below is only for describing an embodiment of the present disclosure, not for limiting the scope of the present disclosure, and reference numerals used the same herein refer to the same components.

Specific terms used herein are only for convenience of description and are not used as a limitation of the illustrated embodiment.

For example, expressions indicating that things are in the same state, such as "same", "equal", "homogeneous", and the like, not only indicate strictly the same state, but also indicate a state in which a tolerance or a difference in a degree to which the same function is obtained exists.

For example, expressions indicating a relative or absolute arrangement such as "in a certain direction", "along a certain direction", "parallel", "orthogonal", "central", "concentric", "coaxial", or the like not only strictly indicate such arrangement, but also indicate a state in which a relative displacement is achieved with a tolerance, or an angle or a distance that achieves the same function.

In order to describe the present disclosure, the description below will be achieved on the basis of a spatial orthogonal coordinate system with an X-axis, a Y-axis, and a Z-axis orthogonal to each other. Each axial direction (an X-axis direction, a Y-axis direction, or a Z-axis direction) means both directions in which each axis extends. Adding a '+' sign in front of each axial direction (a +X-axis direction, a +Y-axis direction, or a +Z-axis direction) means a positive direction, which is one of the two directions in which each axis extends. Adding a '-' sign in front of each axial direction (a -X-axis direction, a -Y-axis direction, or a - Z-axis direction) means a negative direction, which is the other of the two directions in which each axis extends.

Expressions referring to directions such as "front (+Y)/rear (-Y)/left (+X)/right (-X)/up (+Z)/down (-Z)" to be mentioned below are defined based on a XYZ coordinate axis. However, this is to describe the present disclosure such that the present disclosure may be clearly understood. In one example, each direction may be defined differently depending on the standard.

The use of terms such as 'first, second, third' in front of the components to be mentioned below is only to avoid confusion of the components referred to, and is independent of the order, importance, or master-slave relationship between the components. For example, an invention including only the second component without the first component may also be implemented.

As used herein, singular expressions include plural expressions unless the context clearly dictates otherwise.

As used herein, laundry includes not only tops such as a dress shirt and a blouse, but also bottoms such as jeans. In addition, it is a concept that includes all laundry that requires daily care, such as a suit, a jumper, leather clothes, and the like. Therefore, it may include uniforms such as a school uniform or a company uniform. In addition, as used herein, a term 'goods' has been used as a term including items such as a hat, a scarf, a handkerchief, a bag, or a doll, excluding typical laundry, and also including things such as a watch and an accessory. However, herein, even when only one of the terms 'laundry' and 'goods' is used, such term may be interpreted to include both the laundry and the goods in the context of the present document. In other words, the two are not used to exclude each other, but to include each other.

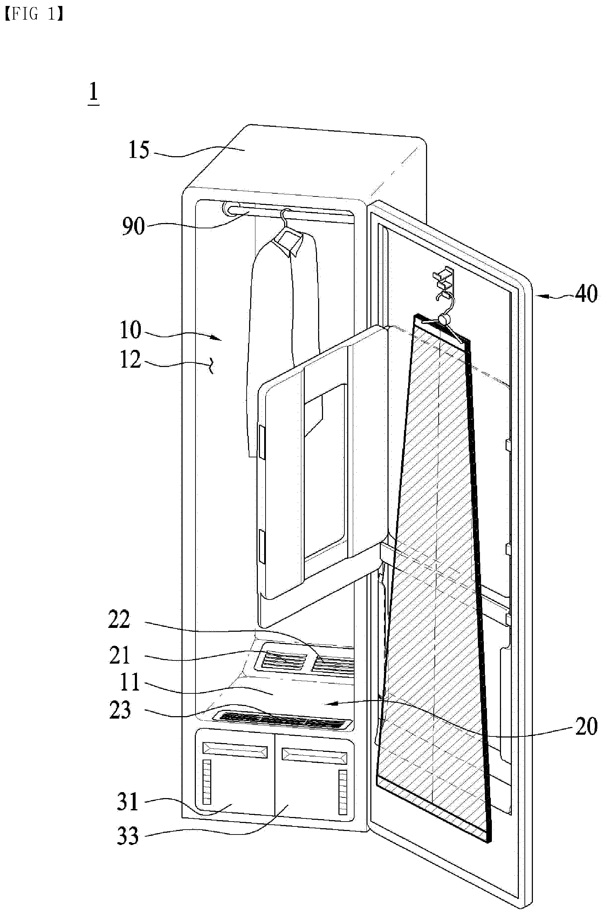

FIG. 1 shows an example of an existing laundry treating apparatus 1. The laundry treating apparatus 1 includes a cabinet 15 including an inlet 12 defined in one surface thereof, a first chamber 10 located inside the cabinet 15 to accommodate the laundry therein through the inlet 12, an auxiliary chamber 20 located under the first chamber 10 and defining therein a space separated from the first chamber 10, a steam unit (not shown) disposed inside the auxiliary chamber 20 to generate steam and supply the steam to the first chamber 10, and a door 40 pivotably coupled to the cabinet 15 to open and close the inlet 12. Considering a usage method of general users, the inlet 12 will preferably be defined in a front surface of the cabinet 15.

In addition, the laundry treating apparatus 1 may further include a blowing unit (not shown) that is located inside the auxiliary chamber 20 and sucks air from the first chamber 10, and a heat pump unit (not shown) that dehumidifies and heats the sucked air and then discharges the air to the first chamber 10.

The cabinet 15 may be made of a metal material, but may also be made of a plastic material as long as it may maintain strength. Additionally, the first chamber 10 may be formed by an inner casing manufactured by plastic injection molding. The first chamber 10 may be coupled to the cabinet 15 by a frame (not shown).

The laundry that requires the care may be placed in the first chamber 10, and the laundry may be cared for in a refreshing way via the blowing unit (not shown), the heat pump unit (not shown), and the steam unit (not shown) located inside the auxiliary chamber 20. That is, a function of sterilizing and deodorizing the laundry and removing wrinkles formed by use may be performed using steam and/or heated air via the blowing unit (not shown), the heat pump unit (not shown), and the steam unit (not shown) located inside the auxiliary chamber 20.

The first chamber 10 may include a laundry support 90 for hanging the laundry at an inner upper portion of the first chamber 10. The laundry support 90 may accommodate thereon a hanger where the laundry is hung, and may be connected to a driver (not shown) that may reciprocate the laundry support part 90 in a left and right direction. The movement of the laundry support 90 may shake the laundry, ultimately removing foreign substances, including fine dust, attached to the laundry. Additionally, while shaking the laundry mounted on the laundry support 90, the wrinkles in the laundry may be removed to some extent by being exposed to steam or moisture supplied from the auxiliary chamber 20.

That is, the laundry support 90 may allow the laundry to be hung in an unfolded state by a self-weight thereof inside the first chamber 10, so that the laundry may be evenly exposed to dehumidified and heated air and/or steam supplied from the auxiliary chamber 20.

An air supply port 21 and a steam supply port 22 for supplying steam generated by the steam unit (not shown) and air dehumidified and heated by the heat pump unit (not shown) inside the auxiliary chamber 20 to the first chamber 10, and an air intake port 23 for sucking air from the first chamber 10 by the blowing unit (not shown) may be located on a first chamber bottom surface 13.

As shown in FIG. 1, the air supply port 21 and the steam supply port 22 may be disposed on the first chamber bottom surface 11 or on a first chamber rear surface (not shown). In addition, an area where the first chamber bottom surface 11 and the first chamber rear surface (not shown) meet each other may be formed in a smoothly inclined shape, and the air supply port 21 and the steam supply port 22 may be disposed on the inclined surface.

The air intake port 23 may be located close to the inlet 12 on the first chamber bottom surface 11. Accordingly, the air supply port 21 and the air intake port 23 are arranged along a front and rear direction of the cabinet 15. On the other hand, the laundry support 90 extends along a width direction of the cabinet 15. This is to ensure that air discharged from the air supply port 21 is uniformly supplied to the laundry hanging on the laundry support 90.

Air inside the first chamber 10 will circulate by being discharged through the air supply port 21 and sucked via the air intake port 23. A water supply tank 31 and a drain tank 33 may be constructed to be independently detachable from a tank module frame (not shown). Otherwise, the water supply tank 31 and the drain tank 33 may be coupled into one and detachable at the same time.

As described above, the existing laundry treating apparatus 1 is an apparatus only for the laundry care and utility thereof as an apparatus for storage or display, including the laundry care, is not high. In addition, the existing laundry treating apparatus does not have a separate space for a case in which an amount of laundry or goods is small, so that input electrical energy may not be utilized efficiently.

In a case of expensive laundry or items, care via temperature and humidity adjustment, as well as display are required. Herein, reflecting such need, it is disclosed that the laundry treating apparatus has a space at a front side that is separate from the first chamber 10 and is able to independently adjust the temperature and the humidity.

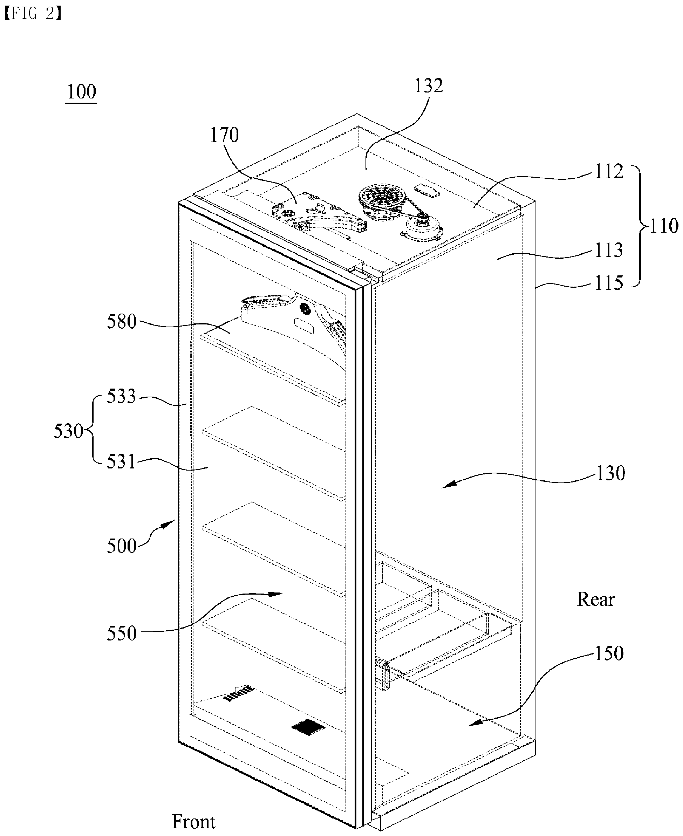

FIG. 2 shows a laundry treating apparatus 100 described in the present disclosure. Referring to FIG. 2, the laundry treating apparatus 100 includes a cabinet 110 including a first inlet 131 defined in a front surface thereof, a first chamber 130 located inside the cabinet 110 to accommodate the laundry or the goods therein through the first inlet 131, an auxiliary chamber 150 located inside the cabinet 110 at a lower side of the cabinet 110 and defining therein a space separated from the first chamber 130, a door assembly 500 that opens and closes the first inlet 131, and a second chamber 550 that is located inside the door assembly 500, defines therein a space separated from the first chamber 130 and the auxiliary chamber 150, and accommodates the laundry or the goods therein.

Because the first chamber 130 is a space for treating the accommodated laundry or goods, the first chamber 130 may also be referred to as a treatment room.

The cabinet 110 may include a cabinet top surface 112 that forms a top surface of the cabinet 110, a cabinet rear surface 115 that forms a rear surface of the cabinet 110, and a cabinet left side surface 113 and a cabinet right side surface (not shown) that form both side surfaces of the cabinet. The cabinet left side surface 113 and the cabinet right side surface (not shown) will be disposed to face each other. The cabinet top surface 112, the cabinet left side surface 113, and the cabinet right side surface 114 may be integrally formed.

The cabinet 110 includes the first inlet 131 in the front surface thereof. Therefore, the first chamber 130 is accessible via the first inlet 131. The cabinet 110 may further include the first chamber 130 and the auxiliary chamber 150 located under the first chamber 130 inside the cabinet 110. The first inlet 131 is intended to be distinguished from the second inlet 512, which will be described later. Herein, when there is no specific mention, an inlet refers to the first inlet 131. Additionally, the auxiliary chamber 150 may be referred to as a machine room, like the first chamber 130 referred to as a treatment room. Additionally, the second chamber 550 may also be referred to as a door chamber. Therefore, the second inlet 512 may be referred to as an inlet of the door chamber.

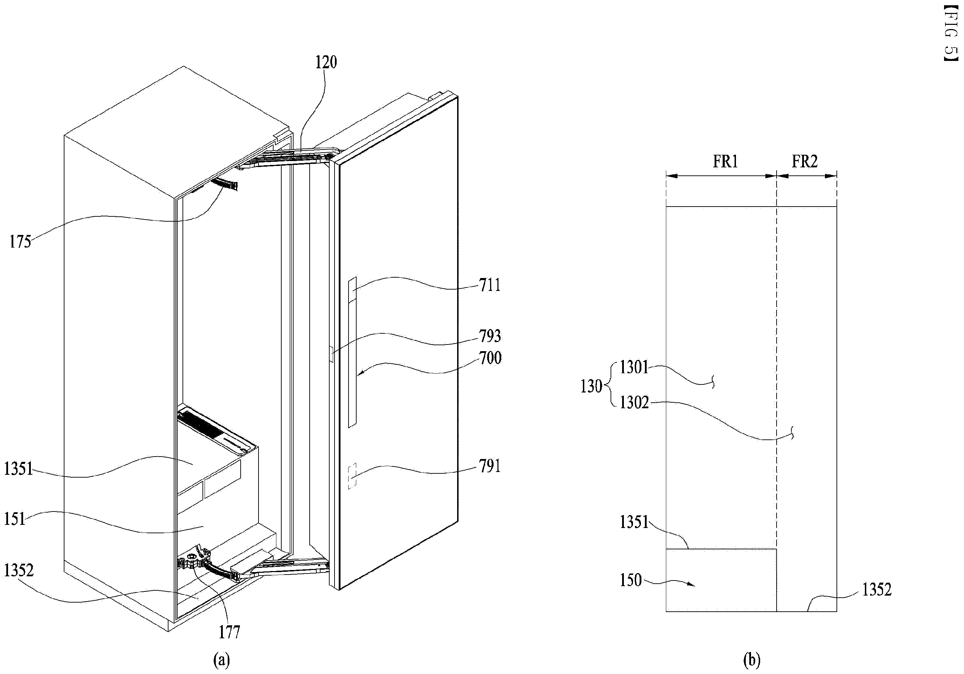

The first chamber 130 may include a first area 1301 (see (b) in FIG. 5) located on the auxiliary chamber 150, and a second area 1302 (see (b) in FIG. 5) located in front of the auxiliary chamber 150 and where at least a portion of the second chamber 550 is accommodated when the door assembly 500 closes the first inlet 131.

The first chamber 130 may be a space where the laundry is accommodated and cared for. The first chamber 130 may also be called a treatment room. The auxiliary chamber 150 may be a space where an electrical device or a mechanical device for supplying hot air or steam to the first chamber 130 is installed. The auxiliary chamber 150 may also be called a machinery room. The second chamber 550, as a space separate from the first chamber 130 and the auxiliary chamber 150, may also be called an auxiliary care room, a showcase room, or a display room.

When the door assembly 500 closes the first inlet 131, at least a portion of the second chamber 550 is located inside the first chamber 130, so that the second chamber 550 may be located in front of the auxiliary chamber 150.

The door assembly 500 may serve as a door that opens and closes the first inlet 131. A user will need to open and close the door assembly 500 to access the first chamber 130.

Additionally, the door assembly 500 may include the second chamber 550 therein for caring for, displaying, and storing the laundry or the goods. That is, the door assembly 500 may simultaneously perform the role of the door that opens and closes the first inlet 131, as well as the role of the space for caring for, storing, and displaying the laundry or the goods accommodated therein.

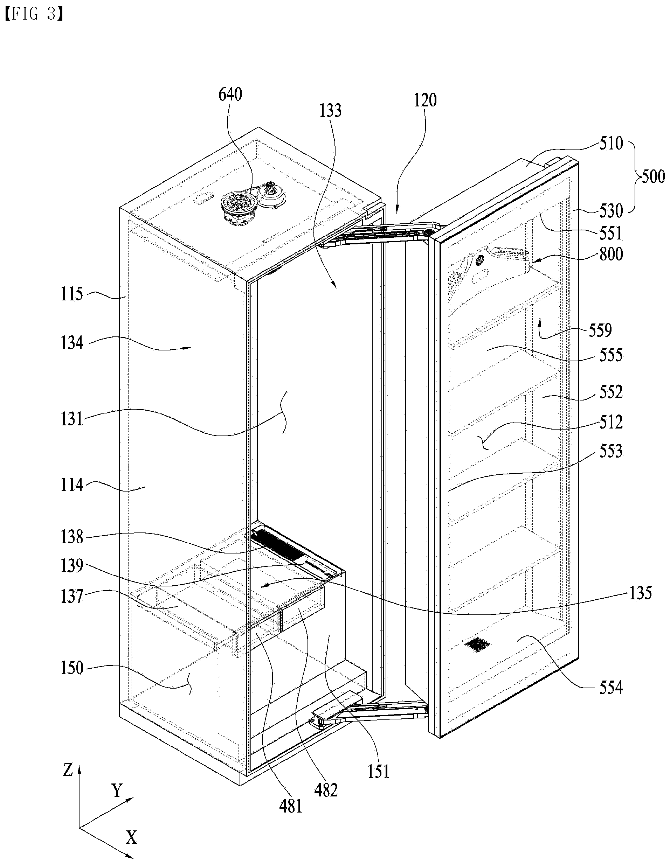

The door assembly 500 may include a second inlet 512 (see FIG. 3) in a front surface thereof, an accommodating body 510 (see FIG. 3) forming the second chamber 550 therein, and a door 530 of the second chamber that opens and closes the second inlet 512. After opening the door 530 of the second chamber, the user may put the laundry or the goods in the second chamber 550 through the second inlet 512.

Considering that, when the door assembly 500 closes the first inlet 131, at least a portion of the second chamber 550 is located in the second area 1302, which is located closer to the first inlet 131 than a rear surface of the first chamber 130 inside the first chamber 130, at least a portion of the receiving body 510 will be located in the second area 1302 when the door assembly 500 closes the first inlet 131.

At least a portion of the second chamber 550 may be accommodated in a space between the first inlet 131 and the auxiliary chamber 150 when the door assembly 500 closes the first inlet 131.

Accordingly, when the door assembly 500 closes the first inlet 131, the second chamber 550 may be located in front of the auxiliary chamber 150 inside the first chamber 130. Accordingly, the auxiliary chamber 150 may be positioned at a predetermined distance rearward from the first inlet 131 to accommodate at least a portion of the door assembly 500 inside the first chamber 130.

In one example, the first inlet 131 may be opened and closed with a door without the separate internal space, unlike the door assembly 500 that includes the second chamber 550 inside.

As shown in FIG. 2, a bottom surface of the first chamber 130 may be formed to be stepped because of the auxiliary chamber 150 forming the bottom surface.

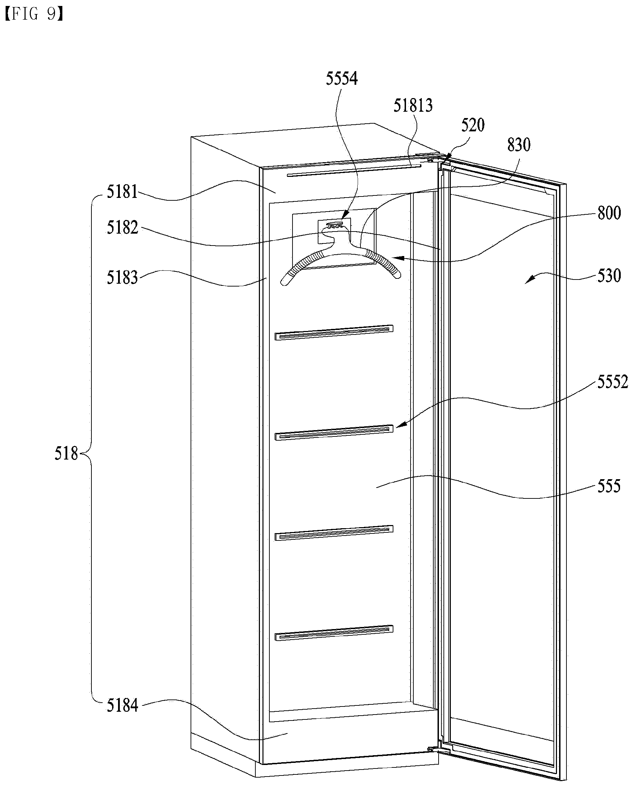

In one example, the door assembly 500 may mount a laundry hanger 830 (see FIG. 9) for accommodating the laundry in the second chamber 550. Alternatively, a shelf 580 that is detachably disposed in the second chamber 550 to support the laundry or the goods accommodated in the second chamber 550 may be further included.

As shown in FIG. 2, the door 530 of the second chamber may be made of a material that allows the interior of the second chamber 550 to be viewed. In other words, at least a portion of the door 530 of the second chamber may be made of a material that allows visible light to pass through.

When the door 530 of the second chamber is opened, the door assembly 500 may accommodate the laundry or the items in the second chamber 550 through the second inlet 512.

Accordingly, a front surface of the door 530 of the second chamber may be made of a transparent material. It may be made of a completely transparent material, but on the other hand, may also be made of a translucent material.

To this end, the door 530 of the second chamber may include an opening (not shown) in which at least a portion of a front surface is opened, and may include a door frame 533 of the second chamber forming a frame of the door 530 of the second chamber and a door window 531 of the second chamber surrounded by the door frame 533 of the second chamber and coupled to the door frame 533 of the second chamber.

At least a portion of the door window 531 of the second chamber may be made of the transparent material or may be made of the translucent material. Additionally, the door window 531 of the second chamber may be transparent but may have a color.

The door frame 533 of the second chamber may be disposed to surround an outer surface of the door window 531 of the second chamber. In contrast, the door frame 533 of the second chamber may be coupled to a rear surface of the door window 531 of the second chamber, so that only the door window 531 of the second chamber may be exposed in a forward direction of the door 530 of the second chamber.

In one example, the laundry treating apparatus 100 may further include an opening/closing driver 170 that pushes the door assembly 500 to automatically open the door assembly 500. The opening/closing driver 170 may be disposed between the cabinet top surface 112 and a first chamber top surface 132 forming a top surface of the first chamber 130.

Referring to FIG. 2, the opening/closing driver 170 may be coupled to a lower side of the cabinet top surface 112. Alternatively, the opening/closing driver 170 may be disposed at the lower side of the first chamber top surface 132.

One opening/closing driver 170 may be sufficient, but there may be a plurality of opening/closing drivers. The opening/closing driver 170 may be disposed not only between the cabinet top surface 112 and the first chamber top surface 132, but also on a first chamber bottom surface 135 forming a bottom surface of the first chamber. Alternatively, the opening/closing driver 170 may be located between the first chamber bottom surface 135 and a bottom surface of the cabinet 110.

The reason why there are the plurality of opening/closing drivers 170 is to prevent deformation of the door assembly 500 or a hinge structure that couples the door assembly 500 to the cabinet from occurring as the door assembly 500 is pushed from above or below and thus a force is concentrated on one side or unnecessary torque is generated.

Referring to FIG. 3, the door assembly 500 may further include an accommodating housing 559 located inside the accommodating body 510 to form the second chamber 550.

When the accommodating body 510 corresponds to an outer body, the accommodating housing 559 may correspond to an inner body. Ultimately, the accommodating housing 559 may form an inner surface of the second chamber 550.

A predetermined empty space may be defined between the accommodating body 510 and the accommodating housing 559, and a mechanical device for conditioning air in the second chamber 550 or various wires may be installed in the empty space.

Accordingly, the door assembly 500 may include the second inlet 512 at the front surface thereof and the accommodating housing 559 forming the second chamber 550 inside the accommodating body 510.

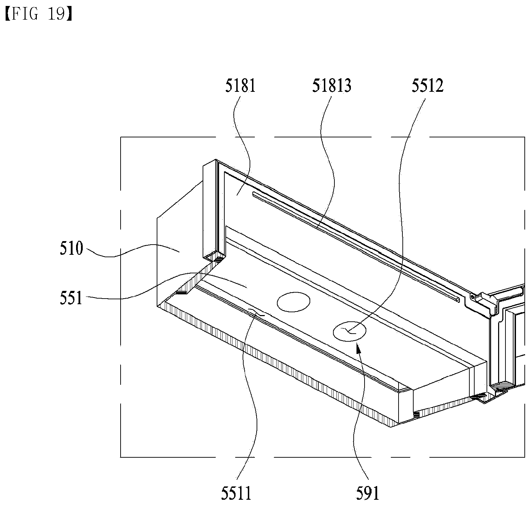

The accommodating housing 559 may include a second chamber top surface 551 (see FIG. 19) forming an upper portion of the second chamber 550, a second chamber bottom surface 554 forming a bottom surface of the second chamber 550, a second chamber rear surface 555 forming a rear surface of the second chamber and connecting a rear edge of the second chamber top surface 551 with a rear edge of the second chamber bottom surface 554, and a second chamber left side surface 552 and a second chamber right side surface 553 connecting the second chamber top surface 551, the second chamber bottom surface 554, and the second chamber rear surface 555 with each other.

In one example, the laundry treating apparatus 100 may sterilize, deodorize, remove the wrinkles from, and dry the laundry by spraying hot air or steam to the laundry accommodated in the first chamber 130. In particular, insulation of the laundry treating apparatus 100 may be important for the drying and the steam spraying.

In particular, this is because an overall insulation performance may be deteriorated when there is no insulating material because at least the portion of the door 530 of the second chamber is made of the material that allows visible light to pass therethrough. Additionally, there may be a risk of burns when the user unintentionally touches the door with hand during the steam spraying or the drying. To prevent this, the second chamber 550 should be formed separately from the first chamber 130 and the auxiliary chamber 150. In particular, in the door assembly 500, an insulating layer using the insulating material or air may be formed in the empty space between the accommodating body 510 and the accommodating housing 559.

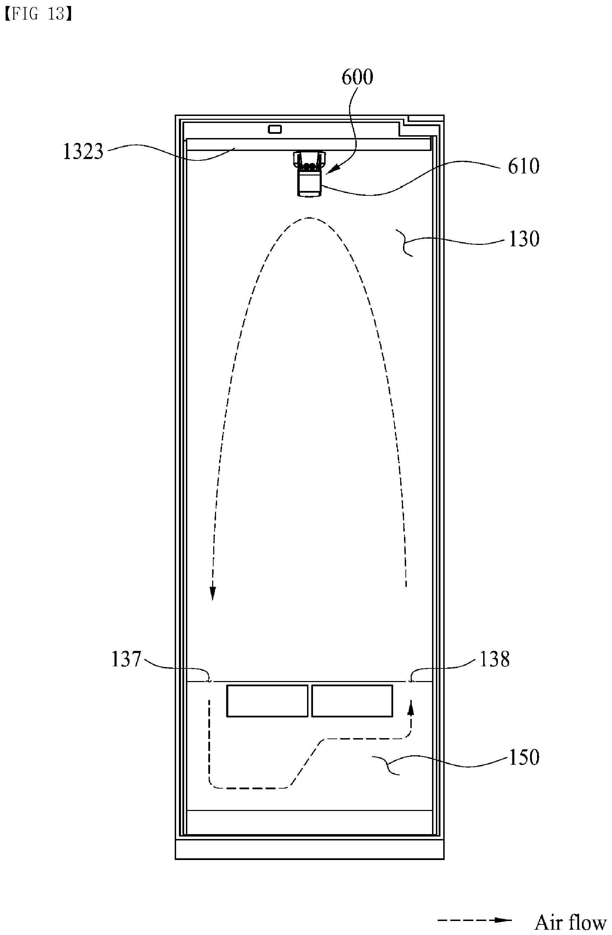

In one example, the laundry treating apparatus 100 may further include a laundry driver 640 located between the first chamber top surface 132 and the cabinet top surface 112 to reciprocate a hanger body 610 (see FIG. 13), which will be described later. This is to protect the laundry driver 640 from hot air, steam, or moisture supplied to the first chamber 130 and to prevent a complex driving device or the like such as the laundry driver 640 from being exposed to the user.

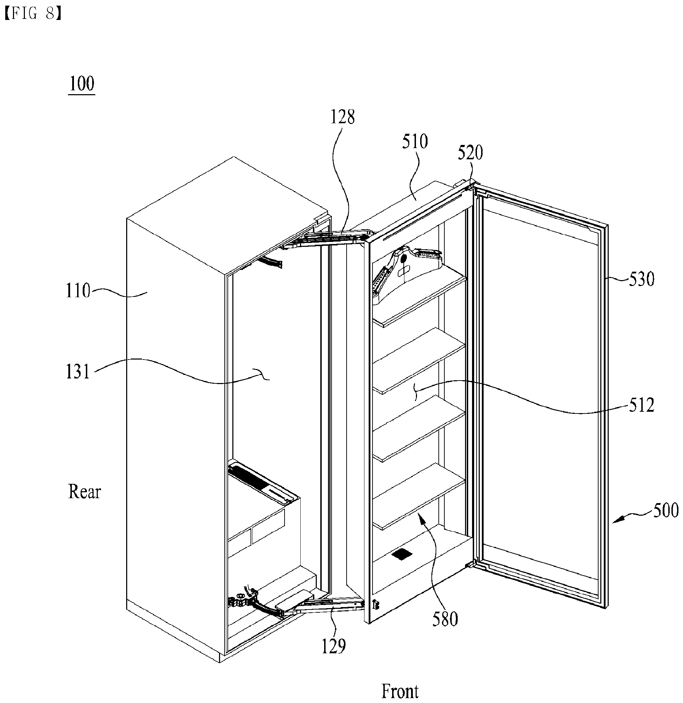

In one example, the laundry treating apparatus 100 may further include a first hinge 120 that pivots such that, when the door assembly 500 is opened, the door assembly 500 is withdrawn forward of the cabinet 110 and then moves along a width direction of the cabinet 110, and a second hinge 520 (see FIG. 8) that pivots to open the second inlet 512 via the pivoting of the door 530 of the second chamber when the door 530 of the second chamber is opened.

When the door assembly 500 closes the first inlet 131, the door assembly 500 will move in a reverse order of opening the door assembly 500.

The first hinge 120 may move the door assembly 500 while maintaining an angle between the door assembly 500 and the first inlet 131. That is, when the door assembly 500 moves, the first hinge 120 will allow the second inlet 512 to always face forward.

In other words, when the door assembly 500 is moved, an angle formed by the door assembly 500 with a front surface of the cabinet will be maintained. To this end, the first hinge 120 may separate the door assembly 500 from the first chamber 130 and then move the same along the width direction of the cabinet 110 to open the first inlet 131.

Considering an usage pattern of the users, the laundry treating apparatus 100 may not be installed in an isolated manner, but may be installed and used indoors as of a built-in type. Accordingly, a size of the laundry treating apparatus 100 may be determined by considering a standard size of a closet or furniture installed adjacent thereto.

Accordingly, while the external size of the laundry treating apparatus 100 remains the same, a size of the door assembly 500 including the second chamber 550 increases, so that a volume of the first chamber 130 may decrease. Additionally, to prevent interference with the adjacent furniture when opening and closing the door assembly 500, it is preferable that a portion of the accommodating body 510 is inserted into and accommodated inside the first chamber 130.

To this end, to prevent the interference between the first chamber 130 and the furniture adjacent to the laundry treating apparatus 100, the first hinge 120 may open and close the door assembly 500 as described above.

On the other hand, the second hinge 520, which will be described later, will open the second inlet 512 by moving the door 530 of the second chamber such that an angle between the door 530 of the second chamber and the front surface of the accommodating body 510 increases.

That is, similar to a hinge of a general door, the angle between the door 530 of the second chamber and the second inlet 512 may change while opening and closing the door 530 of the second chamber.

FIG. 3 shows the interior of the first chamber 130 with the door assembly 500 open. Additionally, the interior of the second chamber 550 with the door 530 of the second chamber made of the transparent or translucent material closed is shown.

Referring to FIG. 3, the first chamber 130 may be formed by the first chamber top surface 132 (see FIG. 2) forming the top surface of the first chamber 130, the first chamber bottom surface 135 forming the bottom surface of the first chamber 130, and a first chamber left side surface 133, a first chamber right side surface 134, and a first chamber rear surface 136 connecting the first chamber top surface 132 with the first chamber bottom surface 135 to form both side surfaces and a rear surface, respectively.

The first chamber 130 may be made of a plastic or metal material. In the case of plastic material, the first chamber 130 may be formed via injection molding.

The auxiliary chamber 150 may be disposed to be separated from the first chamber 130 at an inner lower portion of the cabinet 110. The electrical or mechanical devices necessary for spraying hot air and/or steam into the first chamber 130 may be disposed in the auxiliary chamber 150. Considering that operation of the mechanical device causes vibration or noise and considering weights of the various mechanical devices, it is preferable that the auxiliary chamber 150 is located under the first chamber 130.

In a case of the typical laundry treating apparatus 1, the first chamber bottom surface 135 may be formed extend to the first inlet 131 and the auxiliary chamber 150 may be located beneath an entirety of the first chamber bottom surface 135. However, in the present disclosure, considering that the door assembly 500 is inserted into the first chamber 130, it may be preferable for the auxiliary chamber 150 to be formed in a lower portion of the first chamber 130.

Accordingly, an auxiliary chamber front surface 151, which is a front surface of the auxiliary chamber 150 facing the door assembly 500, will be located at the rear of the first inlet 131. This may mean that a step height is formed on the first chamber bottom surface 135 in a side view of the first chamber 130.

On the other hand, in another example, the second chamber 550 may be located upward of the auxiliary chamber 150 between the first inlet 131 and the auxiliary chamber 150. However, in this case, when the door assembly 500 is closed, the second chamber 550 will protrude into the first chamber 130.

In addition, in the case of another example, because the second chamber 550 is located upward of the auxiliary chamber 150, there is no interference between the auxiliary chamber 150 and the second chamber 550, so that the first chamber bottom surface 135 may not have the step height.

That is, when the door assembly 500 is inserted into the first chamber 130, a lower portion of the accommodating body 510 will face the auxiliary chamber front surface 151 and an upper portion of the accommodating body 510 will face the first chamber rear surface 136. That is, when the door assembly 500 closes the first inlet 131, the second chamber 550 will be located in front of the auxiliary chamber 150 inside the first chamber 130.

Therefore, a length of a rear surface of the accommodating body 510 inserted into the first chamber 130 is greater than a vertical length from the first chamber top surface 132 to the first chamber bottom surface 135 along a height direction of the cabinet 110 and is smaller than a vertical length from the first chamber top surface 132 to the first chamber bottom surface 135.

Accordingly, the first chamber bottom surface 135 may be formed to be stepped. Referring to (a) in FIG. 5, the first chamber bottom surface 135 may include a first bottom surface 1351 and a second bottom surface 1352 disposed at different vertical levels.

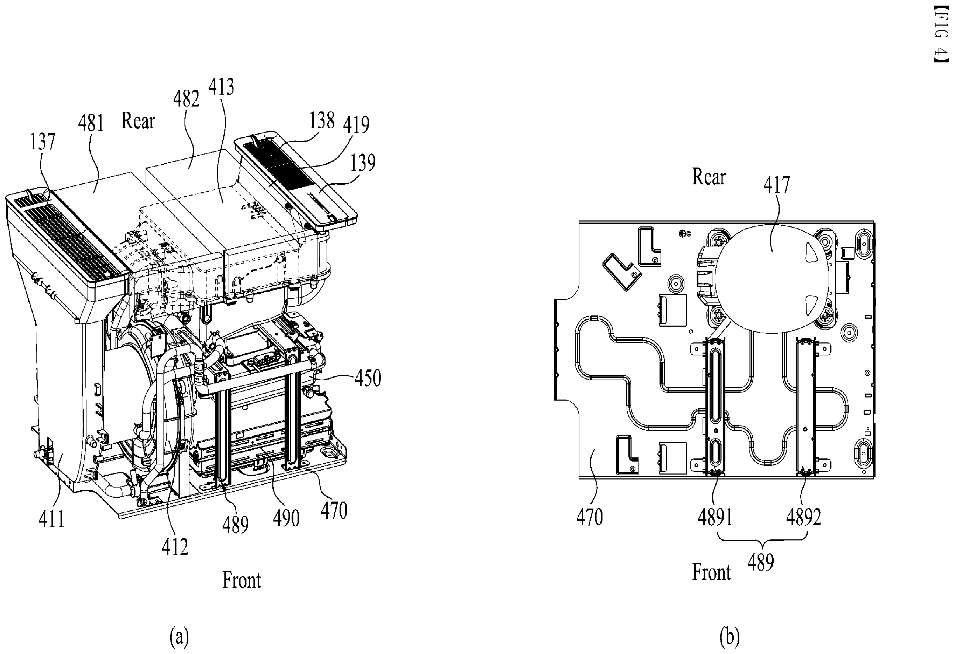

Referring to FIG. 3 and (a) in FIG. 4, the first chamber bottom surface 135 may include an air intake port 137 and an air discharge port 138 in communication with an air supply 410 that circulates or heats air of the first chamber 130. In addition, the first chamber bottom surface 135 may further include a steam discharge port 139 that is in communication with a steam supply 450 that generates steam and supplies the steam to the first chamber 130.

The auxiliary chamber 150 may be located beneath the first bottom surface 1351. Accordingly, the air intake port 137, the air discharge port 138, and the steam discharge port 139 may be located on the first bottom surface 1352 rather than on the second bottom surface 1352.

Additionally, a length from the first chamber top surface 132 to the first bottom surface 1351 may be smaller than a length from the first chamber top surface 132 to the second bottom surface 1352.

Referring to FIG. 3, the air intake port 137 and the air discharge port 138 may be disposed to be spaced apart from each other along the width direction or a left and right direction of the cabinet 110.

One of the air intake port 137 and the air discharge port 138 may be located close to one side surface of the two side surfaces 113 and 114 of the cabinet 110, and the other of the air intake port 137 and the air discharge port 138 may be located close to the other side surface of the two side surfaces 113 and 114 of the cabinet 110.

Additionally, the steam discharge port 139 may be located closer to the air discharge port 138 than the air intake port 137.

In one example, FIG. 3 and (a) in FIG. 4 show an example in which the steam discharge port 139 is located close to the first inlet 131. That is, the steam discharge port 139 may be located forwardly of the air discharge port 138. However, this is merely an example, and it may be desirable for the steam discharge port 139 to be located closer to the first inlet 131 than to the air discharge port 138 in terms of condensate treatment. This is to minimize the condensate generated on the first chamber rear surface 136.

A length in the front and rear direction of the second bottom surface 1352 may be set considering a depth at which the portion of the door assembly 500 is inserted into the first inlet 131.

For example, the depth at which the door assembly 500 is inserted into the first inlet 131 may be equal to or greater than 1/6 and equal to or smaller than 1/2 of a length in the front and rear direction of the cabinet 110. This is because the depth at which the door assembly 500 is inserted into the first inlet 131 should be 1/6 or greater considering a size of the goods accommodated in the second chamber 550, and it is preferable that the depth at which the door assembly 500 is inserted into the first inlet 131 is equal to or smaller than 1/2 for preventing overturning of the cabinet 110 considering a weight of the door assembly 500.

A water supply tank 481 for supplying water for the steam generation and a drain tank 485 for storing condensate may be inserted into the auxiliary chamber 150 through the auxiliary chamber front surface 151. That is, the auxiliary chamber front surface 151 may include a water supply tank insertion hole (not shown) and a drain tank insertion hole (not shown) into which the water supply tank 481 and the drain tank 485 are inserted, respectively, and the water supply tank 481 and the drain tank 485 may be inserted into or separated from the auxiliary chamber 150 via the water supply tank insertion hole and the drain tank insertion hole.

Referring to FIG. 3, the water supply tank 481 and the drain tank 485 may be positioned spaced apart from each other. That is, a space where the water supply tank 481 is installed and a space where the drain tank 485 is installed may be separated from each other. In a case of the typical laundry treating apparatus, they are located adjacent to each other in one tank installation space, so that the user may have difficulty in removing only one of the water supply tank 481 and the drain tank 485. To prevent this, in the laundry treating apparatus 100, the water supply tank 481 and the drain tank 485 may be arranged to be spaced apart from each other.

Additionally, the water supply tank 481 and the drain tank 485 may have lengths along the front and rear direction of the cabinet 110 that are greater than lengths along the height direction of the cabinet 110. This is to maximally utilize the compact space of the auxiliary chamber 150.

(a) in FIG. 4 shows the steam supply 450, the air supply 410, the water supply tank 481, and the drain tank 485 disposed inside the auxiliary chamber 150. The laundry treating apparatus 100 may further include the air supply 410 located inside the auxiliary chamber 150 to circulate or heat air in the first chamber 130, and the steam supply 450 that generates steam and supplies the steam to the first chamber 130.

The air supply 410 may include an air supply duct 411 that sucks air from the first chamber 130, a discharge duct 419 that discharges air to the first chamber 130, and a connecting duct 413 that connects the air supply duct 411 with the discharge duct 419 to circulate air. The air supply 410 may further include a blowing unit 412 that generates a suction force to suck air from the first chamber 130. The blowing unit 412 may be located between the connection duct 413 and the discharge duct 419 or between the air supply duct 411 and the connection duct 413.

The air supply duct 411 will be in communication with the air intake port 137, and the discharge duct 419 will be in communication with the air discharge port 138.

Considering that the air intake port 137 and the air discharge port 138 are arranged in the left and right direction of the cabinet 110, the air supply duct 411, the connecting duct 413, and the discharge duct may also be arranged along the width direction. Accordingly, air circulating in the first chamber 130 may form an air flow that circulates along the left and right direction of the cabinet 110 until it is discharged from the air discharge port 138 and introduced into the air intake port 137.

That is, air discharged from the air discharge port 138, which is located close to one side surface of both side surfaces of the cabinet 110, will ascend along said one side surface and then descend along the other side surface of both side surfaces of the cabinet 110 to be introduced into the air intake port 137.

A heat exchanger (not shown) for heat exchange with circulating air may be further included inside the connecting duct 413. The heat exchanger may be formed as a heater, but may be formed as a heat pump in terms of energy efficiency. The heat pump may include an evaporator and a condenser for heat exchange with a refrigerant.

Accordingly, the heat exchanger may cool and dehumidify air sucked via the air supply duct 411 via the heat exchange with the refrigerant circulating through the heat exchanger, and then heat the air again to create high-temperature dry air. The high-temperature dry air may be again supplied into the first chamber 130 to dry the laundry hung in the first chamber 130.

To place the electric devices or the mechanical devices in the space of the auxiliary chamber 150 as efficiently as possible, the laundry treating apparatus 100 may further include a base 470 that forms a bottom surface of the auxiliary chamber 150, and a supporter 489 that defines a predetermined installation area on the base 470.

The steam supply 450 may be located in the installation area, and the connecting duct 413 including the heat exchanger may be located above the supporter 489. The space of the auxiliary chamber 150 may be used more efficiently by being divided in the height direction of the cabinet 110.

A controller 490 may also be located inside the auxiliary chamber 150. The controller 490 may control the steam supply 450, the air supply 410, the laundry support to be described later, the opening/closing driver, various sensors and an inputter/outputter, a second chamber lighting 590, and a shelf lighting 587.

Referring to (a) in FIG. 4, it is shown that the controller 490 is located between the steam supply 450 and the base 470, but this is only an example and the controller 490 may be located elsewhere. For example, the controller 490 may be located between the cabinet top surface 112 and the first chamber top surface 132. Alternatively, the controller 490 may be disposed inside the door assembly 500. Alternatively, the controller 490 may be located close to the cabinet rear surface 115 at the rear of the auxiliary chamber 150.

The water supply tank 481 and the drain tank 485 may be located between the connecting duct 413 and the first chamber bottom surface 135. This is because an extra space is defined above the connecting duct 413 because of a vertical dimension of the discharge duct 419 and the water supply tank 481 and the drain tank 485 are able to be installed using such space.

Accordingly, inside the auxiliary chamber, the base 470, the steam supply 450, the connecting duct 413, the water supply tank 481, and the drain tank 485 may be arranged sequentially from the bottom along the height direction.

(b) in FIG. 4 shows a lay-out of a compressor 417 included in the mechanical device. This is to compress the refrigerant and circulate the same via the heat exchanger. The refrigerant will exchange heat with air circulating through the air supply 410. Air circulating in the first chamber 130 via the heat exchanger will be supplied to the first chamber 130 in the high-temperature and dry state.

The compressor 417 may be located on one side of the supporter 489. Considering a size and vibration of the compressor 417, the compressor 417 may be located closer to the cabinet rear surface 115 than to the auxiliary chamber front surface 151 or the first inlet 131.

Referring to (b) in FIG. 4, it is shown that the supporter 489 is composed of a first supporter 4891 and a second supporter 4892 to support the steam supply 450 and the air supply 410. Each of the first supporter 4891 and the second supporter 4892 may include two legs and a mounting portion connecting the two legs to each other.

In contrast, the supporter 489 is formed by a rectangular mounting portion extending in the width direction of the cabinet 110, and front and rear mounting surfaces that extend from front and rear side surfaces among side surfaces of the mounting portion and are coupled to the base 470, respectively. In other words, the supporter 489 may be formed as one member rather than as two separate members like the first supporter 4891 and the second supporter 4892. That is, one member may form a rectangular parallelepiped shape together with the base 470, and the steam supply 450 may be located inside the rectangular parallelepiped shape.

(a) in FIG. 5 shows an example in which the door assembly 500 is opened and the first chamber 130 is exposed to the outside. Additionally, (a) in FIG. 5 shows an example of an inputter/outputter 700 disposed on the front surface of the door assembly 500 or on the front surface of the door 530 of the second chamber. The inputter/outputter 700 may receive control information from the user to operate the laundry treating apparatus 100. Additionally, the inputter/outputter 700 may inform the user of a performance state (or information of a current treatment state) of the laundry treating apparatus 100 based on the control information of the user. The present disclosure shows an example in which the inputter/outputter 700 is visually displayed on the front surface of the door assembly 500, but unlike this, the inputter/outputter 700 may further include a speaker, a haptic device, and the like that may inform the user of the information also via hearing or touch.

The user may cause the laundry treating apparatus 100 to perform a desired operation via the inputter/outputter 700 by touching the inputter/outputter 700 with hand. Alternatively, the user may cause the laundry treating apparatus 100 to perform the desired operation by pressing or touching a button disposed on the inputter/outputter 700. The inputter/outputter 700 may have an inputter that receives the command of the user and an outputter that outputs the state of the laundry treating apparatus 100 separated from each other.

The controller 490 may sense and process an user input via the inputter/outputter 700, and display the state of the laundry treating apparatus 100 or results via the inputter/outputter 700.

In one example, the door assembly 500 may be relatively heavy, unlike a regular door, because of the second chamber 550 included inside. Therefore, it may be difficult for the user to open and close the door assembly 500 manually. Additionally, considering design, the door assembly 500 may not have a separate handle for manually opening and closing the door assembly 500.

To this end, the laundry treating apparatus 100 may further include the opening/closing driver 170 that pushes the door assembly 500 to open the same when the user wishes to open the door assembly 500. The controller 490 may control the opening/closing driver 170 to open the door assembly 500.

The inputter/outputter 700 may include a door opening inputter 711 that senses a door opening command of the user. For example, when the user selects a door opening menu in the inputter/outputter 700, the controller 490 may sense the same and operate the opening/closing driver 170 to open the door assembly 500.

Alternatively, the laundry treating apparatus 100 may include a proximity sensor 791 disposed in the door assembly 500. Accordingly, when the user is located within a predetermined sensing area of the proximity sensor 791, the controller 490 may sense user's approach and may operate the opening/closing driver 170 to open the door assembly 500.

The proximity sensor 791 may be located in the front surface of the door assembly 500, that is, in the door 530 of the second chamber. For example, the proximity sensor 791 may be located adjacent to the inputter/outputter 700.

Alternatively, the laundry treating apparatus 100 may open the door assembly 500 by operating the opening/closing driver 170 when the user inputs the door opening command via an app or a remote controller.

(a) in FIG. 5 shows the opening/closing driver 170 with an upper opening/closing driver 175 located at an upper portion and a lower opening/closing driver 177 located at a lower portion inside the cabinet 110. Alternatively, one opening/closing driver 170 among the upper opening/closing driver 175 and the lower opening/closing driver 177 may be implemented to open the door assembly 500.

(b) in FIG. 5 shows the internal space of the first chamber 130 divided into the first area 1301 and the second area 1302 when viewed from one side surface of the cabinet 110. (b) in FIG. 5 simply schematically shows a relative locational relationship of the first chamber 130, the second chamber 550, and the auxiliary chamber 150.

That is, considering that, when the door assembly 500 closes the first inlet 131, at least the portion of the second chamber 550 is located in the second area 1302, which is located closer to the first inlet 131 than to the rear surface of the first chamber 130 inside the first chamber 130, at least the portion of the accommodating body 510 will be located in the second area 1302 when the door assembly 500 closes the first inlet 131.

Additionally, to hang the laundry inside the first chamber 130, the user may access the first area 1301 through the first inlet 131 and the second area 1302. That is, a laundry support 600 to be described later will be located in the first area 1301 when the door assembly 500 is closed.

Accordingly, at least the portion of the second chamber 550 will be accommodated in the space between the first inlet 131 and the auxiliary chamber 150 when the door assembly 500 closes the first inlet 131.

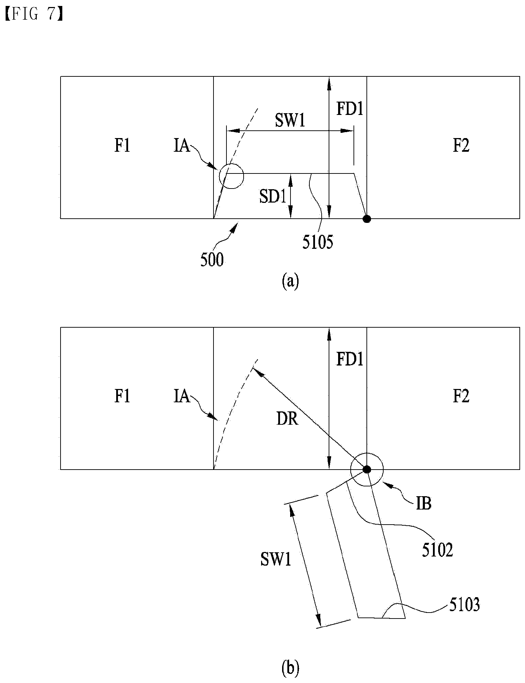

In this regard, the rear surface of the door assembly 500, that is, at least a portion of an accommodating body rear surface 5105 (see FIG. 7), specifically, a lower portion of the accommodating body rear surface 5105, may face the auxiliary chamber front surface 151. Another portion of the accommodating body rear surface 5105, specifically an upper portion of the accommodating body rear surface 5105, will face the first chamber rear surface 136.

In one example, an embodiment in which the second chamber 550 is located upward of the auxiliary chamber 150 may also be considered. However, a space located under the second chamber 550 may have a problem of defining a stagnant area in which air does not circulate well.

In addition, the first chamber 130 and the second chamber 550 may be in communication with each other, but in this case, when treating the laundry inside the first chamber 130, the laundry located inside the second chamber 550 should also be treated, so that the laundry accommodated in the second chamber 550 is not able to be separately cared for. Additionally, when the first chamber 130 and the second chamber 550 are simply in communication with each other, the condensate generated because of a temperature difference between the first chamber 130 and the second chamber 550 is not able to be treated. For example, when steam is sprayed into the first chamber 130, the steam may enter the second chamber 550 and generate the condensate in the second chamber 550, but because the first chamber 150 is operating, the condensate generated in the second chamber 550 may be treated.

In one example, a bottom surface of the first area 1301 corresponds to the first bottom surface 1351 of the stepped first chamber bottom surfaces 135, and a bottom surface of the second area 1302 corresponds to a second chamber bottom surface 1352.

Considering the weight of the door assembly 500 and the sizes of the various electrical devices or mechanical devices accommodated inside the auxiliary chamber 150, a length FR2 in the front and rear direction of the second area 1302 may be equal to or smaller than a length FR1 in the front and rear direction of the first area 1301 along the front and rear direction of the cabinet 110. This is because there is the risk of overturning of the laundry treating apparatus 100 because of the weight of the door assembly 500 when the length FR2 in the front and rear direction of the second area 1302 exceeds the length FR1 in the front and rear direction of the first area 1301.

In addition, no empty space is created between the accommodating body 510 and the auxiliary chamber 150 because of the second chamber 550, so that not only may the spaces of the first chamber 130 and the second chamber 550 be used more efficiently, but also may the stagnant area that may cause flow stagnation during the air circulation be eliminated.

In one example, a vertical dimension of the second chamber 550 may be greater than a vertical dimension of the first chamber 130. This is because the vertical level of the first bottom surface 1351 is higher than the vertical level of the second bottom surface 1352 because of a vertical dimension of the auxiliary chamber 150. Accordingly, the second chamber 550 may accommodate therein laundry relatively longer than the laundry accommodated in the first chamber 130.

Specifically, the vertical dimension of the second chamber 550 may be greater than that of the first area 1301.

Referring to (b) in FIG. 5, the vertical dimension of the first area 1301 (or a length from the first chamber top surface 132 to the first bottom surface 1351) may be smaller than the vertical dimension of the second area 1302 (or a length from the first chamber top surface 132 to the first bottom surface 1351). This is because the vertical dimension of the auxiliary chamber 550 located under the first area 1301 should be considered.

That is, the first chamber bottom surface 135 will be formed in the stepped shape by including the first bottom surface 1351 and the second bottom surface 1352, which has the relatively lower vertical level than the first bottom surface 1351 because of the auxiliary chamber 150 located under the first chamber bottom surface 135.

The second chamber 550 formed inside the door assembly 500 will be accommodated in the second area 1302. Accordingly, the length of the second chamber 550 may be greater than the length of the first area 1301. That is, the second chamber 550 may accommodate therein the laundry relatively longer than the laundry accommodated in the first chamber 130. Accordingly, the user will be able to accommodate laundry of various lengths in the laundry treating apparatus 100 and care for the laundry.

Additionally, the auxiliary chamber 150 may serve as a stopper to prevent the door assembly 500 from being pushed back any further. The door assembly 500 will be located in the second area 1302 located on the second bottom surface 1352 inside the first chamber 130. That is, the lower portion of the accommodating body 510, which forms at least a portion of an outer appearance of the door assembly 500, will face the auxiliary chamber front surface 151. Ultimately, even when an unintended external force is applied to the door assembly 500 and the door assembly 500 is pushed into the first chamber 130, the auxiliary chamber 150 may prevent the door assembly 500 from moving into the first chamber 130 by a certain depth or more.

Considering that the laundry is accommodated in the first area 1301, the vertical dimension of the first area 1301 will be greater than the vertical dimension of the auxiliary chamber 150.

Additionally, to prevent the overturning caused by the weight of the door assembly 500, a length in the front and rear direction of the first area 1301 will be equal to or greater than a length in the front and rear direction of the second area 1302.

Considering that at least the portion of the second chamber 550 is inserted into the second area 1302, an area multiplied by a width and a height of the second area 1302 will be greater than an area of the second chamber rear surface 555. That is, an open area of the first inlet 131 will be greater than the area of the second chamber rear surface 555.

A vertical dimension of the front surface of the door assembly 500 will be greater than the vertical dimension of the second area 1302. That is, a vertical dimension of the first inlet 131 will be smaller than a front surface vertical dimension of the door 530 (see FIG. 2) of the second chamber.

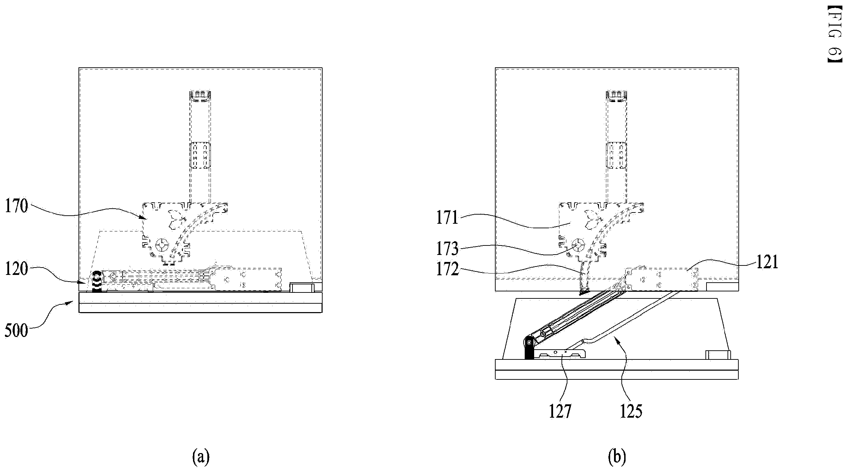

(a) and (b) in FIG. 6 illustrate an operating principle of the opening/closing driver 170. (a) in FIG. 6 shows the door assembly 500 in a closed state, and (b) in FIG. 6 shows the door assembly 500 in an open state.

The opening/closing driver 170 may push the door assembly 500 forward, causing the door assembly 500 to be withdrawn in a direction away from the first inlet 131. Thereafter, the user may pull or push a side surface of the door assembly 500 using a gap created between the door assembly 500 and the first inlet 131 to move the door assembly 500 to one side of the cabinet 110.

It may be preferable that, during the opening and the closing of the door assembly 500, the front surface of the door assembly 500 is maintained to face forward at all times. This is because, as the inputter/outputter 700 (see FIG. 5) disposed in the front surface of the door assembly 500 always faces forward, the user may easily access the inputter/outputter 700, and air inside the second chamber 550 may be circulated to care for the laundry or the goods accommodated inside the second chamber 550.

That is, even when the first chamber 130 is open, the user may execute a care mode of the second chamber 550. In other words, the door assembly 500 may be constructed to move away from the first inlet 131 such that the front surface of the door assembly 500 faces forward, and then move to one side of the cabinet 110 such that the front surface of the door assembly 500 is parallel to the first inlet 131. To this end, the laundry treating apparatus 100 according to the present disclosure may use a scheme of kinematic parallelogram four-bar linkages.

Accordingly, the user may open and close the door assembly 500 while maintaining the first inlet 131 and the door assembly 500 facing away from each other. Accordingly, the door assembly 500 may maintain the direction parallel to the first inlet 131 during the opening and the closing.

That is, an angle between the front surface of the cabinet 110 and the door assembly 500 may be maintained constant. To this end, the first hinge 120 may move the door assembly 500 forward of the first chamber 130 to separate the same from the first inlet 131 and then move the door assembly 500 along the width direction of the cabinet 110. That is, even when the location of the door assembly 500 changes, the direction the door assembly 500 faces may remain the same when opening and closing the first inlet 131. This is because when the door assembly 500 opens the first inlet 131, the front surface of the door assembly 500 moves while being directed in the forward direction of the cabinet 110, in the same manner as when the door assembly 500 closes the first inlet 131.

However, in a case of opening and closing the second inlet 512, the direction in which the front surface of the door 530 of the second chamber faces may change. Unlike the first hinge 120, when the second hinge 520 opens the door 530 of the second chamber, the second inlet 512 and the door 530 of the second chamber pivot relative to each other, so that an angle between the second inlet 512 and the door 530 of the second chamber may always change (see FIG. 8).

Referring to (a) in FIG. 6, the laundry treating apparatus 100 may further include the opening/closing driver 170 that pushes the door assembly 500 to open the door assembly 500. The opening/closing driver 170 may be disposed between the cabinet top surface 112 and the first chamber top surface 132 that forms the top surface of the first chamber 130. For example, the opening/closing driver 170 may be coupled to a lower side of the cabinet top surface 112. Alternatively, the opening/closing driver 170 may be disposed at a lower side of the first chamber top surface 132.

One opening/closing driver 170 may be sufficient, but there may be a plurality of opening/closing drivers. The opening/closing driver 170 may be disposed not only between the cabinet top surface 112 and the first chamber top surface 132, but also on the first chamber bottom surface 135 that forms the bottom surface of the first chamber. Alternatively, the opening/closing driver 170 may be located between the first chamber bottom surface 135 and the bottom surface of the cabinet 110.

The reason why there are the plurality of opening/closing drivers 170 is to push the door assembly 500 from the upper and lower sides to prevent the deformation of the door assembly 500 or the hinge structure that couples the door assembly 500 to the cabinet from occurring as the force is concentrated on one side or the unnecessary torque is generated.

The opening/closing driver 170 may not be in contact with the accommodating body 510. The laundry treating apparatus 100 may further include the first hinge 120 in the four-bar linkages scheme. (b) in FIG. 6 shows a state in which the opening/closing driver 170 pushes the door assembly 500 forward and thus the door assembly 500 is away from the first inlet 131.

The opening/closing driver 170 may include an opening/closing body 171 forming an outer appearance thereof, an opening/closing motor 173 located inside the opening/closing body 171, and an opening/closing link 172 that pushes the door assembly 500 to move the same in the direction away from the first inlet 131 using a rotational force generated by the opening/closing motor 173.

One surface of the opening/closing link 172 may have gear teeth, so that the opening/closing link 172 may move in the front and rear direction of the cabinet 110 in a rack and pinion-like manner. That is, when several gears engage and rotate with a rotation shaft of the opening/closing motor 173 to reduce a rotational speed, and some of the gears engage and rotate with the gear teeth disposed on one surface of the opening/closing link 172, as the opening/closing link 172 moves forward and protrudes, the door assembly 500 may move in the direction away from the first inlet 131.

(a) in FIG. 6 shows an example in which the opening/closing link 172 is formed in a curved shape. This is to prevent the opening/closing driver 170 from taking up a significant portion of a space inside the first chamber as a length in the front and rear direction thereof increases, considering a length that the opening/closing link 172 moves in the front and rear direction. In addition, this is because, when the door assembly 500 is withdrawn forward by the first hinge 120, both links 1251 and 1252 of the first hinge 120 may pivot and thus a spacing therebetween may increase, and at this time, a moving path of the door assembly 500 may be in a curved shape rather than a straight shape. Even in such case, the front surface of the door assembly 500 may move while always facing forward.

When the opening/closing driver 170 pushes the door assembly 500 and a gap or a predetermined separation distance (or an opening distance) is created between the first inlet 131 and the door assembly 500, the user may manually push or pull the door assembly 500 to move the same.

Accordingly, a maximum moving distance of the opening/closing link 172 may be a distance until the accommodating body 510, which was inserted into the first chamber 130, is withdrawn from the first inlet 131.

When the opening/closing link 172 moves the door assembly 500 by the predetermined opening distance, the controller 490 may rotate the opening/closing motor 173 in an opposite direction, so that the opening/closing link 172 may move to be inserted back into the opening/closing body 171. This is because, when the opening/closing link 172 does not return to an original location thereof, the door assembly 500 may collide with the opening/closing link 172 as the closing of the door assembly 500 is performed by the user, unlike the opening.

Accordingly, considering the case in which the user closes the door assembly 500, the controller 490 may return the opening/closing link 172 to the original location thereof by rotating the opening/closing motor 173 in the opposite direction.

That is, to move the opening/closing link 172 from a first location, which is an initial location, to a second location defined ahead of the first location, the controller 490 may rotate the opening/closing motor 173 in a first direction. The opening/closing link 172 is not in contact with the door assembly 500 in the first location, but will be in contact with the door assembly 500 while moving to the second location. Thereafter, the opening/closing driver 170 will move the door assembly 500 in the forward direction of the cabinet 110 by the predetermined opening distance.

Thereafter, when the opening/closing motor 173 rotates in a second direction opposite to the first direction, the opening/closing link 172 may return from the second location to the first location.

Additionally, because the opening/closing driver 170 does not need to be operated when the door assembly 500 is closed, the controller 490 will operate the opening/closing driver 170 only when the door assembly 500 is opened.

Even when the user closes the door assembly 500, the front surface of the door assembly 500 may always be maintained facing forward.

As shown in (b) in FIG. 6, the door assembly 500 may be moved with the front surface always facing forward. This is because the second chamber 550 is not only for caring for and storing the laundry or the goods accommodated, but also for displaying and exhibiting the laundry or the goods. Therefore, this is to ensure that the user may always see the second chamber 550. In addition, even when the first chamber 130 is open, the inputter/outputter 700 disposed in the front surface of the door assembly 500 always faces forward, so that the user may easily access the inputter/outputter 700. Therefore, to care for the laundry or the goods accommodated inside the second chamber 550, the user may circulate air inside the second chamber 550 via the inputter/outputter 700 regardless of whether the first chamber 130 is opened.

To this end, the laundry treating apparatus 100 may be equipped with the first hinge 120 of the parallelogram four-bar linkages type. That is, while pivotably connecting the door assembly 500 to the cabinet 110, the first hinge 120 may open and close the door assembly 500 such that the front surface of the door assembly 500 always faces forward.

(a) and (b) in FIG. 7 show the laundry treating apparatus 100 and furniture F1 and F2 disposed adjacent to the laundry treating apparatus 100. This is illustrated considering that the laundry treating apparatus 100 is disposed together with general furniture in a built-in type.

As shown, the second chamber 550 may be located in front of the first chamber 130. Additionally, because the door assembly 500 includes the second chamber 550 therein, at least the portion of the accommodating body 510 may be inserted into the first chamber 130.

Considering the built-in type, a height and a depth FD1 of the laundry treating apparatus 100 will be designed in consideration of a size of the furniture F1 and F2 disposed adjacently. Here, the depth FD1 refers to the length in the front and rear direction of the laundry treating apparatus 100. The second chamber 550 may be disposed at a side of the laundry treating apparatus 100, but in this case, the width occupied by the laundry treating apparatus 100 becomes too great, which will not be desirable.

Additionally, to utilize the second chamber 550 not only for caring for the laundry but also for the display and exhibition purposes, it will be desirable for the second chamber 550 to be located in front of the first chamber 130 and the auxiliary chamber 150.

In addition, because placing objects to overlap each other in the depth direction of the second chamber 550 is not suitable for the display and exhibition purposes, a depth SD1 of the door assembly 500 will be smaller than the depth FD1 of the laundry treating apparatus.

However, it may not be desirable for the second chamber 550 to protrude beyond the laundry treating apparatus 100 in consideration of sense of unity with the adjacent furniture. Accordingly, to not change the depth FD1 of the laundry treating apparatus, the portion of the second chamber 550 may be inserted into the first chamber 130.

When the portion of the door assembly 500 is inserted into the first chamber 130, a problem may occur when opening the door assembly 500 because of the size of the door assembly 500. (a) and (b) in FIG. 7 show an arc with a radius of DR centered on one side of the cabinet 110.

When the door assembly 500 is hinged to one side of the cabinet 110, a distance from one side of the cabinet 110 to a point indicated by IA, that is, a corner where the accommodating body rear surface 5105 and an accommodating body right side surface 5103 meet each other when the door assembly 500 pivots should be considered. This is to prevent the corner from interfering with one side surface of the first chamber 130. Ultimately, when the door assembly 500 is hinged to one side of the cabinet 110, the depth SD1 of the door assembly 500 or the width of the accommodating body rear surface 5105 should be reduced.

When the door assembly 500 is hinged to the other side of the cabinet 110, interference of an opposite corner should be considered. However, because the problem is the same, only the case in which the door assembly 500 is hinged to one side is described herein.

The depth (or a thickness) of the door assembly 500 may be equal to or greater than 1/6 and equal to or smaller than 1/2 of the length in the front and rear direction of the cabinet 110. This is in consideration of a size of the goods accommodated in the second chamber 550. For example, it may be a numeric value determined by considering a small standard size and a large standard size of bags carried by adult women.

Considering such numeric value, there may be a limit in reducing the depth SD1 of the door assembly 500 or a width SW1 of the accommodating body rear surface 5105.

In addition, referring to (b) in FIG. 7, because the hinge causes interference with the adjacent furniture F2 in an area indicated by IB, the laundry treating apparatus 100 and the adjacent furniture F2 should be spaced by a certain distance apart from each other.

Even when the door assembly 500 opens without the interference, a portion of the first inlet 131 may be obscured by the accommodating body 510, causing inconvenience to the user. Here, "obscured" refers to a case in which the angle formed by the first inlet 131 and the door assembly 500 is an acute angle.

To solve such problem, referring to FIG. 8, the laundry treating apparatus 100 may include the cabinet 110 including the first inlet 131 defined in the front surface thereof, the first chamber 130 located inside the cabinet 110 and accommodating the laundry therein through the first inlet 131, the auxiliary chamber 150 located at the lower side inside the cabinet 110 and forming the installation space therein separate from the first chamber 130, the door assembly 500 that opens and closes the first inlet 131, and the first hinge 120 that connects the door assembly 500 to the cabinet, and the door assembly 500 may include the accommodating body 510 including the second inlet 512 defined in the front surface thereof, the second chamber 550 located inside the accommodating body 510 and formed to be separated from the first chamber 130 and the auxiliary chamber 150, and accommodating the laundry therein through the second inlet 512, the door 530 of the second chamber that opens and closes the second inlet 512, and the second hinge 520 that connects the door 530 of the second chamber to the accommodating body 510.

The first hinge 120 may open and close the first inlet 131 such that the open direction of the second inlet 512 is maintained when the door assembly 500 is opened and closed, and the second hinge 520 may open and close the second inlet 512 via the pivoting of the door 530 of the second chamber.

Maintaining the opening direction of the second inlet 512 means that the door assembly 500 moves while maintaining the direction thereof when the door assembly 500 is opened or closed. When the user is located in front of the door assembly 500, the user will face the second chamber 550. Even when the user opens or closes the door assembly 500, the direction of the door assembly 500 does not change, so that the user may always check the second chamber 550.

In other words, the link disposed in the first hinge 120 pivots, but the second chamber 550 may always face forward via pivoting and translation of the door assembly 500. Accordingly, the door assembly 500 will move in the front and rear direction or the width direction of the cabinet 110 in parallel with the first inlet 131 to open and close the first inlet 131.

In other words, when the door assembly 500 has opened or has closed the first inlet 131 or is opening or closing the first inlet 131, a location and a direction from one arbitrary point of the door assembly 500 to another arbitrary point will not change.

The laundry treating apparatus 100 may include the first hinge 120 connecting the door assembly 500 to the cabinet 110 between both side surfaces of the cabinet 110. The first hinge 120 may be located at the upper portion and/or the lower portion inside the cabinet 110. Specifically. The first hinge 120 may include a first upper hinge 128 disposed on the first chamber top surface 132 and a first lower hinge 129 disposed on the first chamber bottom surface 135.

The first chamber top surface 132 may have a stepped shape. Accordingly, the first chamber top surface may include a stepped surface 1323 (see FIG. 13) resulted from a step height. This is to secure a space where the opening/closing driver 170 is installed between the cabinet top surface 112 and the first chamber top surface 132.

In this case, the first upper hinge 128 may be located in front of the stepped surface 1323 on the first chamber top surface 132.

The first lower hinge 129 may be located in front of the auxiliary chamber 150 or on the second bottom surface 1352.

Referring to FIG. 8, it may be seen that, in the first upper hinge 128 and the first lower hinge 129 included in the first hinge 120, a pivoting length of the links 1251 and 1252 connecting the cabinet with the door assembly 500 is greater than a pivoting length of connecting members 5251 and 5252 of the second hinge 520 connecting the door 530 of the second chamber with the accommodating body 510. This is to prevent the interference with the adjacent furniture by completely separating the door assembly 500 from the cabinet when opening the door assembly 500 or during the opening of the door assembly 500. In addition, this is to prevent the door assembly 500 from obscuring the first inlet 131 when the door assembly 500 is completely opened.

On the other hand, as shown in FIG. 8, when the door 530 of the second chamber is opened while the door assembly 500 is open, the interference with the adjacent furniture resulted from the door 530 of the second chamber will not occur. However, when the door 530 of the second chamber is opened while the door assembly 500 is closed, the interference with the adj acent furniture may occur. Therefore, it will be desirable for the second hinge 520 to be constructed so as not to protrude to the outside of the cabinet 110 when the door 530 of the second chamber is closed.

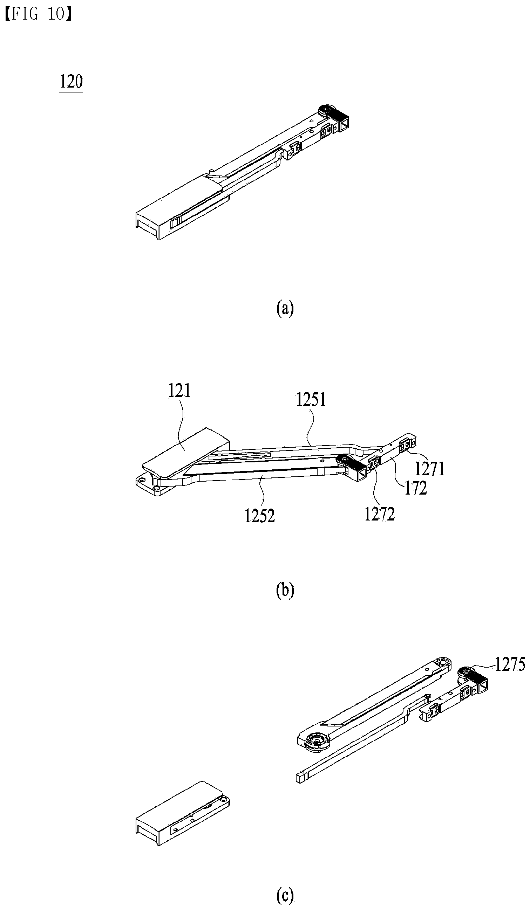

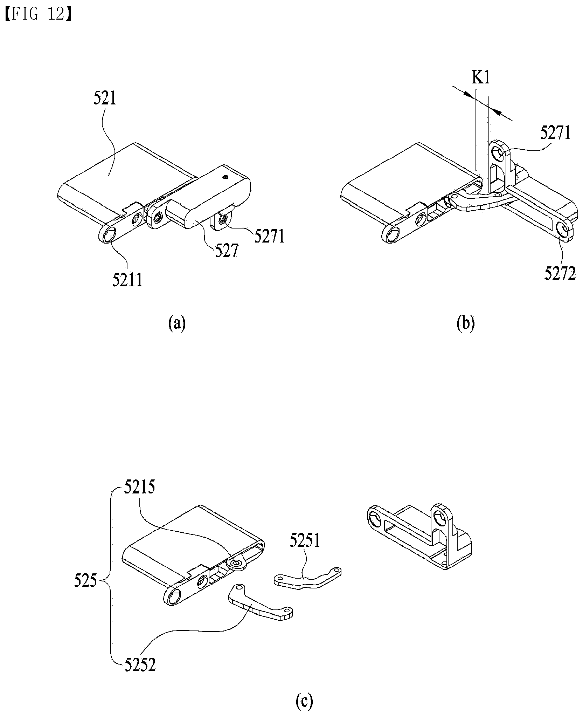

To this end, lengths of the first link 1251 (see (b) in FIG. 10) and the second link 1252 (see (b) in FIG. 10), which will be described later in FIG. 10, may be greater than lengths of a first connecting member 5251 (see (c) in FIG. 12) and a second connecting member 5252 (see (c) in FIG. 12).

Referring to FIG. 9, the door assembly 500 may include the accommodating body 510 including the second inlet 512 defined in the front surface thereof and the second chamber 550 therein, and the door 530 of the second chamber that opens and closes the second inlet 512.

More specifically, referring to FIGS. 3 and 9, the second chamber 550 may be formed by the accommodating housing 559 located inside the accommodating body 510. The user may access the second chamber 550 through the second inlet 512.

More specifically, the door assembly 500 may further include a front panel 518 coupled to the front surface of the accommodating body 510. The second inlet 512 may be defined through the front panel 518.

Accordingly, the door assembly 500 may further include the front panel 518 formed to face the door 530 of the second chamber while surrounding the second inlet 512 and coupled to the accommodating housing 559 and the accommodating body 510.

Alternatively, the front panel 518 may be formed integrally with the accommodating housing 559 and then be coupled to the accommodating body 510 with the open front surface.

Accordingly, the front panel 518 may be coupled to the front surfaces of the accommodating body 510 and the accommodating housing 559, and the laundry treating apparatus 100 may accommodate the laundry or the goods in the second chamber 550 formed inside the accommodating housing 559 through the second inlet 512 extendingg through the front panel 518.

Because the door 530 of the second chamber may have a size corresponding to a size of the front panel 518 surrounding the second inlet 512, the size of the door 530 of the second chamber may be greater than a size of the second inlet 512.

When the door 530 of the second chamber is closed, the door 530 may be coupled to the front panel 518 to close the second inlet 512.

The second hinge 520 may be coupled to the front panel 518 or the accommodating body 510.

That is, the front panel 518 may include a front upper panel 5181 that forms an upper portion of the front panel, a front lower panel 5184 that forms a lower portion of the front panel, and a front left panel 5182 and a front right panel 5183 that connect respective side surfaces of the front upper panel 5181 and respective side surfaces of the front lower panel 5184 to each other.

The second inlet 512 may be defined by the front upper panel 5181, the front lower panel 5184, the front left panel 5182, and the front right panel 5183. That is, the front panel 518 will be located surrounding the second inlet 512. This is to prevent wires, circuit boards, and various devices that may be located between the second chamber 550 and the accommodating body 510 from being exposed to the user.

The second inlet 512 may be defined in a square shape.

The front upper panel 5181 may include an air inlet 51813 defined through the front upper panel 5181. Air in the second chamber 550 may circulate through the air inlet 51813. The air inlet 51813 may be defined to extend along the width direction of the door assembly 500.

Therefore, when the door 530 of the second chamber is closed, because the air inlet 51813 is located in the front panel 518, the air inlet 51813 will face the door 530 of the second chamber.

The door 530 of the second chamber may be coupled to the front surface of the accommodating body 510, that is, the front panel 518 including the second inlet 512, to open and close the second inlet 512. That is, the door 530 of the second chamber may be coupled to the front panel 518 so as to cover at least an area including the air inlet 51813 and the second inlet 512.

That is, when the door 530 of the second chamber is closed, the second chamber 550 will be in communication with the internal space located between the accommodating body 510 and the second chamber 550 via the air inlet 51813.

Additionally, FIG. 9 shows an example of a hanger assembly 800 and a shelf mounting portion 5552 disposed in the second chamber 550. The hanger assembly 800 may include the laundry hanger 830 that may hang the laundry in the second chamber 550. The laundry hanger 830 may be mounted on or detached from the second chamber rear surface 555 that forms the rear surface of the second chamber 550.

The door assembly 500 may further include the hanger assembly 800 disposed in the second chamber 550 to hang the laundry. The hanger assembly 800 may be mounted on or coupled to a hanger mounting portion 5554 disposed in the second chamber 550. The hanger assembly 800 is a kind of accessory and is detachable from the second chamber 550.

Specifically, the door assembly 500 may further include the hanger mounting portion 5554 that may install or mount the hanger assembly 800 on the second chamber rear surface 555. The hanger mounting portion 5554 may vary depending on a shape of the hanger assembly 800. For example, in a case of the hanger assembly 800, which has an outer appearance of a general ring-shaped hanger, the hanger mounting portion 5554 may be in a shape of a hook protruding from the second chamber rear surface 555. The user may display the laundry by hanging the ring of the hanger assembly 800 on the hook.

In contrast, as shown in FIG. 9, in the case of the laundry hanger 830 coupled to the second chamber rear surface 555, the hanger mounting portion 5554 may be in a form of a groove or a hole in the second chamber rear surface 555. Accordingly, the laundry hanger 830 may be coupled to the hanger mounting portion 5554 in a detachable manner.

In one example, the laundry treating apparatus 100 may further include the shelf 580 (see FIG. 2) that is detachably disposed in the second chamber 550 and supports the goods accommodated in the second chamber 550. In addition, the laundry treating apparatus 100 may further include the shelf mounting portion 5552 for coupling with the shelf that is coupled to the second chamber rear surface 555.

Specifically, the second chamber rear surface 555 may include the shelf mounting portion 5552 in the form of the groove or the hole that extends along the width direction of the door assembly 500.

The shelf 580 may be in a shape of a rectangular sheet. The shelf 580 may include a shelf coupling portion 811 for coupling to the shelf mounting portion 5552 at a rear surface thereof. The shelf mounting portion 5552 may include a plurality of shelf mounting portions. Accordingly, the shelf 580 may also include a plurality of shelves coupled to the shelf mounting portions 5552, respectively.

The shelf mounting portion 5552 is coupled to the second chamber rear surface 555, and this is to separate both side surfaces of the shelf 580 from both side surfaces of the second chamber for air circulation in the second chamber 550, which will be described later.

FIG. 9 shows an example in which the hanger assembly 800 is coupled to one surface of the second chamber 550 and the shelf 580 is separated from the shelf mounting portion 5552. Considering a size of the laundry hung on the hanger assembly 800, only some of the plurality of shelves may be coupled to some of the plurality of shelf mounting portions 5552 located below the hanger assembly 800. Even in a state in which the hanger assembly 800 is not coupled, the user will be able to couple the shelves to the shelf mounting portions 5552 while adjusting a spacing between the plurality of shelves considering the size of the goods or the laundry accommodated in the second chamber. That is, depending on the size of the goods to be accommodated and displayed, the shelf 580 coupled to the rear surface of the second chamber 550 may be removed or coupled.

(a) to (c) in FIG. 10 show the first hinge 120. The opening and closing direction of the door assembly 500 may vary depending on whether the user is left-handed or right-handed. Alternatively, the door assembly 500 may be set to be opened and closed in a direction preferred by the user. The opening and closing direction of the door assembly 500 may be set differently depending on a direction in which the same first hinge 120 is installed. The first hinge 120 shown in (a) and (c) in FIG. 10 will move the door assembly 500 to a left side of the user, and the first hinge 120 shown in (b) in FIG. 10 will move the door assembly 500 to a right side of the user.

Referring to (b) in FIG. 10, the first hinge 120 may include a hinge coupling portion 127 coupled to the door assembly 500, a hinge receiving portion 121 coupled to the cabinet, and a link 125 pivotably connected to the hinge coupling portion 127 and the hinge receiving portion 121. The link 125 may be accommodated in the hinge receiving portion 121 when the door assembly 500 closes the first inlet 131.

The link 125 may include the first link 1251 and the second link 1252 that pivotably connect the hinge receiving portion 121 with the hinge coupling portion 127.

That is, the first hinge 120 may include the first link 1251 and the second link 1252 that are pivotably coupled to the cabinet 110 and the accommodating body 510 but are spaced apart from each other.

The first hinge 120 may move the door assembly 500 forward to be away from the first inlet 131 via the pivoting of the first link 1251 and the second link 1252 and then move the door assembly 500 parallel to the first inlet 131.

Because the first link 1251 and the second link 1252 are connected so as to be pivotable at different points of the hinge receiving portion 121 and the hinge coupling portion 127, an overall outline may be in the form of four-bar linkages. In particular, because the hinge receiving portion 121 and the hinge coupling portion 127 are respectively coupled and fixed to the cabinet 110 and the door assembly 500, only the first link 1251 and the second link 1252 may pivot. Therefore, it may have a similar mechanism to the parallelogram four-bar linkages.

That is, when the first link 1251 and the second link 1252 pivot, the hinge receiving portion 121 and the hinge coupling portion 127 are only able to move to be parallel to each other. Ultimately, the first link 1251 and the second link 1252 will also pivot in parallel with each other by being spaced apart from each other.

The door assembly 500 will move in the direction parallel to the first inlet 131.

The door assembly 500 may move with the door 530 of the second chamber always facing forward. Accordingly, the user may always see the second chamber 550 when opening and closing the door assembly 500.

Additionally, when the door assembly 500 closes the first inlet, the first link 1251 and the second link 1252 may be disposed in the direction parallel to the door assembly 500 and be in contact with the door assembly 500. This may be referred to as the first location or a folded state. When the opening/closing driver 170 pushes the door assembly 500, the door assembly 500 may pivot and be withdrawn forward simultaneously by the first hinge 120. Further, the door assembly 500 will finally be moved to one side of the laundry treating apparatus by the first hinge 120.

The first link 1251 and the second link 1252 of the first hinge 120 pivot, but because they are spaced apart from each other, the front surface of the door assembly 500 always faces forward. Accordingly, it may appear to the user that the door assembly 500 is withdrawn in the direction away from the first inlet 131 and then moves laterally.

Strictly speaking, because the first link 1251 and the second link 1252 pivot at a distance from each other, the door assembly 500 pivots by each individual link. However, because the front surface of the door assembly 500 is always fixed facing forward, the door assembly 500 may appear to be performing the translational motion.

Herein, it is described that the door assembly 500 moves forward by the predetermined distance by the opening/closing driver 170 and then moves laterally. This type of door may be called a gliding door or a swing door.

Considering lengths of the first link 1251 and the second link 1252, the first hinge 120 may open the door assembly 500 without interfering with the adjacent furniture. Additionally, when the door assembly 500 is completely opened, the door assembly 500 moves to one side of the laundry treating apparatus 100 and does not obscure the first inlet. Such state may be referred to as an unfolded state. This is to facilitate access to the inputter/outputter 700 even when the door assembly 500 is opened or moved.

The first link 1251 and the second link 1252 may pivot at an angle greater than 90 degrees (°) and smaller than 180 degrees (°) from the folded state to the unfolded state.

Referring to (c) in FIG. 10, the hinge coupling portion 127 may include an L-shaped hinge bent portion 1275 that is formed to be bent at one side. This is to prevent interference between the first link 1251 and the second link 1252 in the folded state.

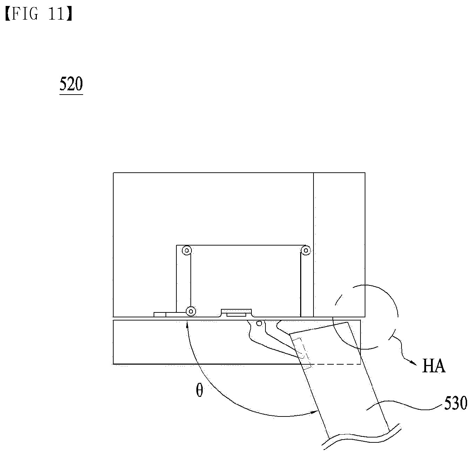

FIG. 11 is an example of the second hinge 520. The door assembly 500 may further include the second hinge 520 to pivotably couple the door 530 of the second chamber to the accommodating body 510.

Because a thickness of the door 530 of the second chamber is smaller than a thickness of the door assembly 500 and the door 530 is coupled to the front surface of the accommodating body 510, the second hinge 520 does not need to operate like the first hinge 120. Therefore, unlike the first hinge 120 moving away from the first inlet 131 and then moving in the direction parallel to the first inlet 131, the second hinge 520, like a general hinge, may be located at one side of the accommodating body 510 and pivot the door 530 of the second chamber, thereby opening and closing the door 530 of the second chamber.

However, when the second hinge 520 is constructed to be exposed to the outside, interference with the adjacent furniture may occur, so that the second hinge 520 may be installed on an inner surface of the door 530 of the second chamber, that is, a surface facing the second inlet 512 among both surfaces of the door 530 of the second chamber, and the accommodating body, so that the second hinge 520 may not be exposed to the outside when the door 530 of the second chamber is closed.

Accordingly, a portion of the second hinge 520 may be coupled to the accommodating body 510, and another portion of the second hinge may be coupled to the inner surface of the door 530 of the second chamber.

When the second hinge 520 pivots and the state of the door 530 of the second chamber is changed from the closed state to the open state, a maximum opening angle 0 of the door 530 of the second chamber may be equal to or greater than 90 degrees (°) and smaller than 180 degrees (°). When the maximum opening angle of the door 530 of the second chamber is equal to or greater than 180 degrees (°), the interference with the adjacent furniture may occur.

(a) to (c) in FIG. 12 show an example of the second hinge 520. Like the first hinge 120, the second hinge 520 may include a member receiving portion 521 coupled to the accommodating body 510, a member coupling portion 527 coupled to the door of the second chamber, and a connecting portion 525 connecting the member receiving portion 521 with the member coupling portion 527. The connecting portion 525 may include the first connecting member 5251 and the second connecting member 5252. The member receiving portion 521 may include a receiving portion fastening groove 5211 to be coupled to the accommodating body 510 using a fastening member.

Similar to the member receiving portion 521, the member coupling portion 527 may also include a first fastening hole 5271 and a second fastening hole 5272, which are fastening holes for fixing the member coupling portion 527 to the inner surface of the door 530 of the second chamber.

The second hinge 520 may include a plurality of second hinges, and may include a second upper hinge 528 connecting upper portions of the door 530 of the second chamber and the accommodating body 510 to each other, and a second lower hinge 529 connecting lower portions of the door 530 of the second chamber and the accommodating body 510 to each other. (a) in FIG. 12 is an example of the second upper hinge 528, and (b) in FIG. 12 is an example of the second lower hinge 529. Locations of the first fastening holes 5271 of the second upper hinge 528 and the second lower hinge 529 are opposite to each other. This is because a location of coupling with the door 530 of the second chamber is taken into consideration. That is, because the second upper hinge 528 is located at the upper portion of the door 530 of the second chamber, a fastening area is secured at a lower side. The second lower hinge 529 will be opposite thereto.

Referring to (b) in FIG. 12, when the second hinge 520 fully opens the door of the second chamber, a predetermined clearance distance K1 may exist between the member receiving portion 521 and the member coupling portion 527. This is to prevent interference between the door 530 of the second chamber and the accommodating body 510 in an area HA shown in FIG. 11.

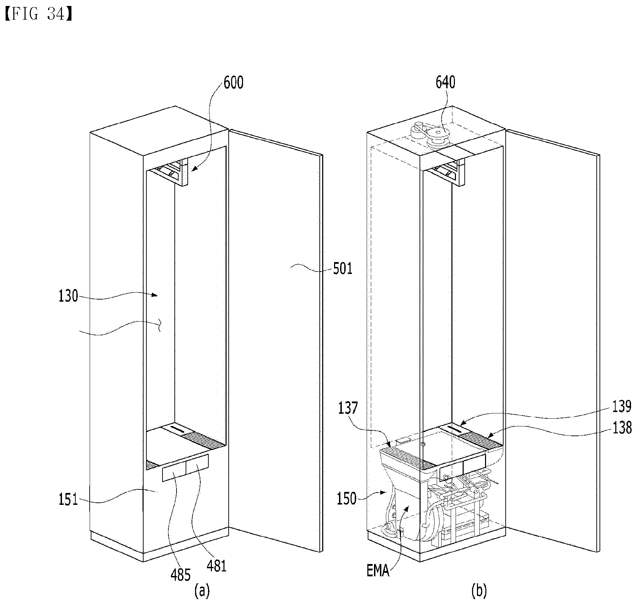

(a) and (b) in FIG. 34 are another example of the laundry treating apparatus 100 described in the present disclosure. The laundry treating apparatus 100 described above includes the door assembly 500 whose volume is increased to accommodate the second chamber 550 inside. On the other hand, the laundry treating apparatus shown in FIG. 34 shows an example of including a door 501 with a relatively small door thickness as the second chamber 550 is not included. That is, this is an example of the laundry treating apparatus 100 in which the space of the first chamber 130 is not divided into the two spaces by the step of the first chamber bottom surface 135.

That is, because the first chamber bottom surface 135 is not stepped, the door 501 may be pivotably coupled to the cabinet 110 to open and close the first inlet (or the inlet) 131. In this regard, the door 501 may have the same structure as the second hinge described above, but may be coupled to the cabinet 110 with an existing hinge. In this case, there may be a predetermined spacing between the furniture and the laundry treating apparatus 100 to prevent interference with the adjacent furniture.

Referring to (a) and (b) in FIG. 34, the laundry treating apparatus 100 according to the present disclosure includes the cabinet 110 including the inlet 131 defined in the front surface thereof, the door 501 that opens and closes the inlet 131, the first chamber 130 that is located inside the cabinet and accommodates therein the laundry or the goods via the inlet 131, the hanger body 610 that is disposed inside the first chamber 130, extends along the front and rear direction of the cabinet 110, and hangs the laundry therein, and the hanger support 620 located on the hanger body 610 and movably supporting the same.

Additionally, the hanger body 610 may be separated from the hanger support 620 and movable forward.

In addition, the auxiliary chamber 150, which is separately disposed under the first chamber 130, may insert and/or withdraw the water supply tank 481 and the drain tank 485 into and/or from the auxiliary chamber 150 via the auxiliary chamber front surface 151 forming the front surface of the auxiliary chamber 150.

(b) in FIG. 34 shows a mechanical device EMA disposed inside the auxiliary chamber 150 to spray air (heated air or unheated air) or steam into the first chamber 130, and a laundry driver 640 disposed between the first chamber top surface 132 and the cabinet top surface 112 to allow the laundry support 600 to perform a reciprocating movement in the front and rear direction of the cabinet 110.

In addition, because the laundry support 600 is disposed in the front and rear direction of the cabinet 110, the air discharge port 138 and the air intake port 137 may be arranged in the width direction of the cabinet 110 to uniformly supply air or steam to the laundry hung on the laundry support 600. In addition, the steam discharge port 139 may also be located adjacent to the air discharge port 138 and extend along the front and rear direction of the cabinet 110 like the air discharge port 138.

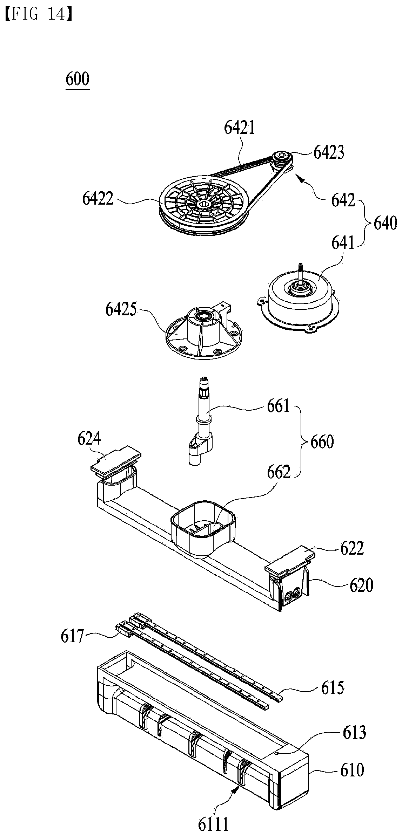

FIG. 14 briefly shows the first chamber 130 and the auxiliary chamber 150 and then schematically shows an air circulation direction. As described in FIGS. 3 and 4, at least a portion of the door assembly 500 may be inserted into the first chamber 130. Accordingly, because the length in the front and rear direction of the auxiliary chamber 150 located under the first bottom surface 1351 is reduced, the air supply duct 411 will be located closer to one of both side surfaces of the cabinet 110 to efficiently place the mechanical devices inside the auxiliary chamber 150. That is, the air supply duct 411 will be disposed in a direction facing the one side surface.

Alternatively, as described above, referring to (a) and (b) in FIG. 34, even in the case of the door 501 without the second chamber 55, to reduce the length in the front and rear direction of the cabinet 110, the laundry support 600 may be disposed in the front and rear direction of the cabinet 110. In any case, as the laundry support 600 is disposed in the front and rear direction of the cabinet 110, the air discharge port 138 and the air intake port 137 may be disposed along the width direction of the cabinet 110 perpendicular to the direction of the laundry support 600. This is to supply air or steam evenly to the laundry being hung.

Considering this, to efficiently place the mechanical device EMA inside the auxiliary chamber 150, the air supply duct 411 will be located close to one of both side surfaces of the cabinet 110. That is, the air supply duct 411 will be disposed in a direction facing the one side surface.

The connecting duct 413 may also be disposed in the left and right direction or the width direction of the cabinet 110.

The supporter 489 may also be disposed in the left and right direction of the cabinet 110.

Ultimately, the flow of air circulating through the air supply 410 may be shown as shown in FIG. 14. FIG. 14 is a view of the first chamber 130 from the first inlet (or inlet) 131.

Referring to FIG. 14, assuming that the air discharge port 138 is located on one side surface among both side surfaces of the first chamber and the air intake port 137 is located on the other side surface among both side surfaces of the first chamber 130, air discharged through the air supply 410 will flow upward toward the first chamber top surface 132 along one side surface of the first chamber through the air intake port 137. Thereafter, the flow direction of air will change by the first chamber top surface 132, and air will meet the other side surface of the first chamber 130 and flow downward again.

Ultimately, air that has flowed along the other side surface of the first chamber 130 will flow to the air supply 410 through the air intake port 137.

Air introduced into the air intake port 137 will first flow downward along the air supply duct 411. Thereafter, air will pass through the blowing unit 412 and flow upward again toward the connecting duct 413. This is because the blowing unit 412 is located on the base 470, while the connecting duct 413 is located above the supporter 489.

That is, air will flow downward and then flow upward again from the air supply duct 411 to the connecting duct 413. This is to prevent condensate that may be introduced via the air supply duct 411 from flowing into a heat exchanger 415 together. The condensate may be separated from air at a lower portion of the air supply duct before being introduced into the blowing unit 412 as the air flow direction changes.

The laundry treating apparatus 100 may further include a sump (not shown) for temporarily storing the condensate at the lower portion inside the air supply duct 411 or outside the air supply duct 411. The condensate temporarily collected in the sump (not shown) may be collected in the drain tank 485 via a drain pump (not shown).

Steam supplied via the steam supply 450 (see FIG. 4) may also be circulated in the same manner. However, instead of the air discharge port 138, steam may be discharged via the steam discharge port 139 and then enter the air supply duct 411 together with air circulating via the air intake port 137.

In one example, according to the present disclosure, as the circulation direction of air and steam in the laundry treating apparatus 100 is set to the left and right direction rather than the front and rear direction, a direction of the laundry support 600, especially the hanger body 610, for hanging the laundry may also be set differently.

To manage the laundry, air and steam should be evenly distributed throughout the laundry. When the hanger body 610 is disposed in the width direction, only laundry hung close to the air discharge port 138 and the steam discharge port 139 may be managed, and laundry hung far away may not be cared for well because of the laundry hung close to the ports. Therefore, for uniform distribution of air and steam, it will be desirable for the laundry support 600 to be disposed along the front and rear direction of the cabinet 110.

In one example, it may be inconvenient for the user to hang the laundry on the laundry support 600. This is because, as a plurality of hanger grooves 6111 defined in the laundry support 600 are arranged in the front and rear direction, distances from the first inlet 131 to the hanger grooves 6111 are all different from each other. Therefore, hanging the laundry in the deepest hanger groove may cause discomfort to the user.

To solve such problem, the laundry treating apparatus 100 may include the laundry support 600 disposed along the front and rear direction of the cabinet 110, and the hanger body 610 including the plurality of hanger grooves 6111 of the laundry support 600 may be extendable forward. Therefore, the user will be able to extend the hanger body 610 forward via the first inlet 131 and hang the laundry thereon without inconvenience of having to bend down.

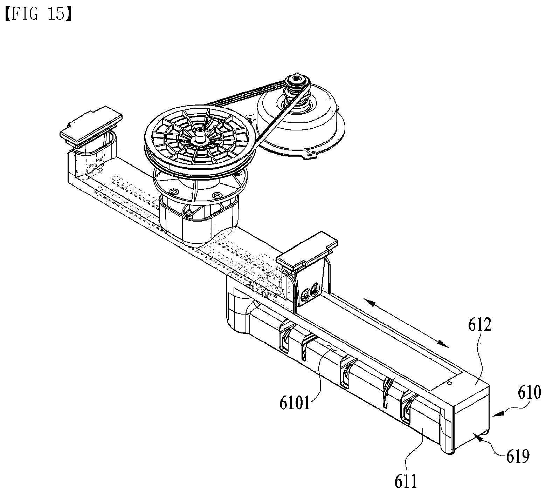

FIG. 15 is an exploded view of the laundry support 600 disposed in the first chamber 130. The laundry support 600 may include the hanger body 610 disposed along the front and rear direction of the cabinet 110 to hang the laundry thereon, and a hanger support 620 disposed along the front and rear direction of the cabinet 110 and located on the hanger body 610 to support the hanger body 610.

The laundry support 600 may further include the laundry driver 640 that generates a rotational force and a power converter 660 that converts the rotational movement of the laundry driver 640 into a reciprocating movement along the front and rear direction of the cabinet 110.

The laundry driver 640 may be located between the first chamber top surface 132 and the cabinet top surface 112 to prevent exposure to the user and to prevent breakdown caused by steam and hot air supplied to the first chamber.

Additionally, the laundry driver 640 may be supported by a hanger frame (not shown) that supports the laundry driver 640. The hanger frame may also be located between the first chamber top surface 132 and the cabinet top surface 112, and both ends of the hanger frame may be coupled to the cabinet frame (not shown) that forms a skeleton of the cabinet 110 or the cabinet 110. This is to prevent vibration of the laundry driver 640 from being transmitted into the first chamber 130.

The laundry driver 640 may include a power motor 641 and a power transmitter 642 that is connected to a rotation shaft of the power motor and transmits power to the power converter. FIG. 14 shows a driving pulley 6423, a driven pulley 6422, and a belt 6421 connecting the driving pulley 6423 with the driven pulley 6422 as an example of the power transmitter 642, but the present disclosure may not be limited thereto.

In addition, the laundry driver 640 may further include a pulley fixing member 6425 that is supported on the moving hanger frame and supports the driven pulley 6422.

The power converter 660 may include a rotatable portion 661 that is connected to the driven pulley 6422 and rotates together when the driven pulley 6422 rotates, and a converting member 662 that converts the rotation of the rotatable portion 661 into a reciprocating movement. The rotatable portion 661 may perform a circular motion of a certain radius by the rotatable portion 661 constructed to rotate at a distance from a rotation shaft direction of the driven pulley 6422.

Specifically, the rotatable portion 661 may include a rotatable connecting member connected to the rotation shaft at one end, an arm member connected to the other end of the rotatable connecting member in a direction perpendicular to the rotation shaft, and inserted rotatable member connected to the converting member 662 in a direction parallel to the rotation shaft at the other end of the arm member.

FIG. 15 shows that the converting member 662 is defined at an upper side of the hanger support 620 in a form of a groove or a hole defined along the front and rear direction of the cabinet 110, but the present disclosure is not limited thereto.

The hanger support 620 may include a first support 622 and a second support 624 that support both ends of the hanger support, respectively. The first support 622 and the second support 624 will support the hanger support 620 when the hanger support 620 performs the reciprocating movement along the front and rear direction by the converting member 662.

The laundry support 600 may include the hanger body 610 that is located below the hanger support 620 so as to be spaced apart therefrom and hangs the laundry thereon. The hanger support 620 and the hanger body 610 may extend along the front and rear direction of the cabinet 110.

The hanger support 620 and the hanger body 610 may be coupled to each other to perform the reciprocating movement by the laundry driver 640.

The hanger body 610 may include the plurality of hanger grooves 6111 where the laundry may be hung. FIG. 14 shows the plurality of hanger grooves 6111 arranged at spacings that are not uniform. This is to allow thin laundry, for example, a dress shirt to be additionally hung. For example, FIG. 14 shows five hanger grooves. Among them, first, third, and fifth hanger grooves are spaced apart from each other at an uniform spacing, and second and fourth hanger grooves are located close to the first and fifth hanger grooves, respectively. The first, third, and fifth hanger grooves are hanger grooves that are arranged considering the size of the laundry. On the other hand, the second and fourth hanger grooves may be used to additionally hang the laundry.

The hanger body 610 is coupled to the hanger support 620, but is extendable forward via the first inlet 131 when necessary. That is, the hanger support 620 may further include a moving rail 615 for moving the hanger body 610 forward. The hanger body 610 may further include a moving guider 617 that is located on the hanger body 610 and couples the hanger body 610 to the moving rail 615. When the moving guider 617 moves on the moving rail 615, the hanger body 610 may move in the front and rear direction along the moving rail 615.

Referring to FIGS. 14 and 15, the hanger body 610 may further include a hanger bar 611 including the plurality of hanger grooves 6111 for hanging the laundry. The hanger body 610 may further include an upper hanger body 612 located on the hanger bar 611 to prevent interference with the moving rail 615 or the laundry support 600 when the user hangs the laundry in the hanger grooves 6111.

The hanger bar 611 and the upper hanger body 612 may be spaced apart from each other and face away from each other. The hanger bar 611 and the upper hanger body 612 may be located to be spaced apart from each other and to be parallel with each other to define a hanging space 6101, which is a space accessible to the user for hanging the laundry. The hanger bar 611 and the upper hanger body 612 may be coupled to each other by each connecting body 619 connecting each of both ends of the hanger bar 611 with each of both ends of the upper hanger body 612.

That is, the hanging space 6101 may be defined by the hanger bar 611, the upper hanger body 612, and each connecting body 619 connecting each of both ends of the hanger bar 611 with each of both ends of the upper hanger body 612.

In other words, the hanger body 610 may include the hanging space 6101 defined through the cabinet 110 in the width direction when the hanger body 610 is disposed in the front and rear direction of the cabinet 110. The plurality of hanger grooves 6111 where the laundry may be hung may be located in a bottom surface of the hanging space 6101.

FIG. 14 shows an example in which the hanger body 610 extends forward of the hanger support 620. The user may extend the hanger body 610 forward from the hanger support 620, hang the laundry, and then retract the hanger body 610 rearwards again.

Specifically, the laundry support 600 may be located along the front and rear direction of the cabinet 110 in the first area 1301. When the laundry support 600 is located in the first area 1301, the hanger bar 611 will be fixed to the upper hanger body 612 and will perform the reciprocating movement along the front and rear direction of the cabinet 110 along with the upper hanger body 612 when the laundry driver 640 rotates. Thereafter, when the door assembly 500 is opened, the user may extend the hanger bar 611 from the upper hanger body 612 and move the same in a direction toward the first inlet 131.

That is, when the door assembly 500 is opened, the hanger bar 611 may be extended from the upper hanger body 612 toward the first inlet, and at least a portion of the hanger bar 611 may be located in the second area 1302.

In one example, when the laundry driver 640 rotates, the hanger body 610 and the hanger support 620 will perform the reciprocating movement together along the front and rear direction. Therefore, when the hanger body 610 is retracted, the hanger body 610 may unintentionally move in the front and rear direction along the moving rail 615 when the coupling with the hanger support 620 is not fixed.

This is a problem that occurs because the extension and retraction direction of the hanger body 610 is the same as the reciprocating movement direction of the hanger body 610.

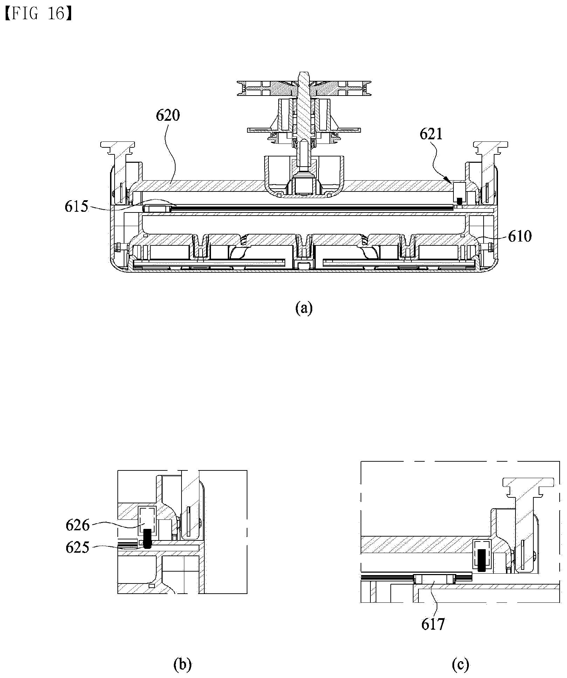

To solve such problem, referring to (a) in FIG. 16, the laundry support 600 may further include a hanger bar fixing groove 613 defined in one of the hanger support 620 and the hanger body 610, and a hanger bar stopper 621 that is disposed in the other of the hanger support 620 and the hanger body 610 and is able to insert a movement preventing protrusion 625 into the hanger bar fixing groove 613 depending on whether the door assembly 500 is opened.

(b) in FIG. 16 shows a state in which the movement preventing protrusion 625 of the hanger bar stopper 621 is inserted into the hanger bar fixing groove 613 and thus the hanger body 610 is fixed to the hanger support 620. (c) in FIG. 16 shows a state in which the movement prevention protrusion 625 is separated from the hanger bar fixing groove 613.

The hanger bar stopper 621 may include the movement preventing protrusion 625 that may be inserted into the hanger bar fixing groove 613 and a moving motor 626 for inserting the movement preventing protrusion 625 into the hanger bar fixing groove 613 or separating the same from the hanger bar fixing groove 613.

The hanger bar stopper 621 may automatically insert the movement preventing protrusion 625 into the hanger bar fixing groove 613 or separate the same from the hanger bar fixing groove 613 depending on whether the door assembly 500 is opened.

To this end, the laundry treating apparatus 100 may further include a door opening/closing sensor 793 that senses whether the door assembly 500 is opened or closed. When the door opening/closing sensor 793 senses the opening of the door assembly 500, the controller 490 may operate the moving motor 626 to separate the movement preventing protrusion 625 from the hanger bar fixing groove 613. In contrast, when the door opening/closing sensor 793 senses the closing of the door assembly 500, the controller 490 may operate the moving motor 626 in an opposite direction to insert the movement preventing protrusion 625 into the hanger bar fixing groove 613.

Referring to FIG. 5, the door opening/closing sensor 793 is shown as being located in the rear surface of the door assembly 500, but the door opening/closing sensor 793 may be located anywhere as long as it may sense whether the door assembly 500 is opened or closed. For example, the door opening/closing sensor 793 may be disposed on the first hinge 120.

That is, when the door assembly 500 closes the first inlet 131, the controller 490 will insert the movement preventing protrusion 625 into the hanger bar fixing groove 613 to couple the hanger body 610 with the hanger support 620. This is to prepare for the reciprocating movement of the laundry support 600 in the future.

When the door assembly 500 is opened, there is a high possibility that the user will extend the hanger body 610 forward, so that the controller 490 may separate the movement preventing protrusion 625 from the hanger bar fixing groove 613 in preparation therefor. Therefore, even when the user moves the hanger body 610 forward, interference between the hanger body 610 and the movement preventing protrusion 625 will not occur.

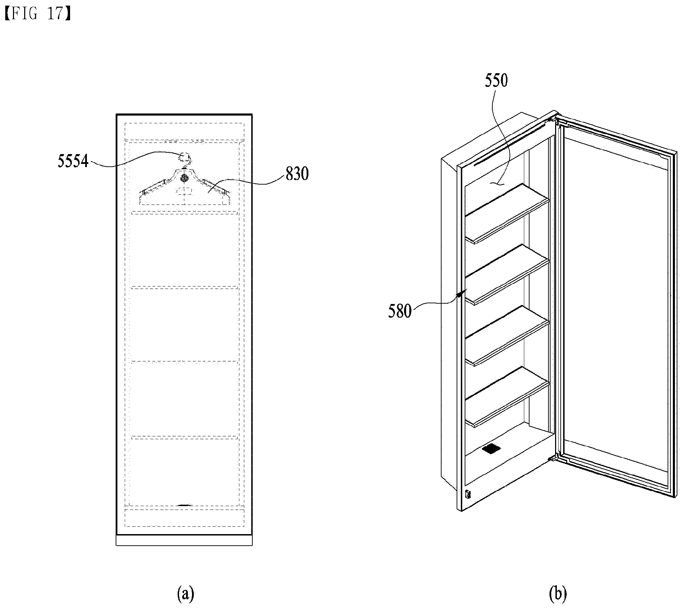

(a) in FIG. 17 is a front view of the door assembly 500. (b) in FIG. 17 shows the door 530 of the second chamber being opened. (a) in FIG. 17 shows another example in which the laundry hanger 830 is hung on the hanger mounting portion 5554. Additionally, the door 530 of the second chamber may be made of a transparent material, so that the user may check the second chamber 550 without opening the door 530 of the second chamber. Referring to (b) in FIG. 17, the door assembly 500 may further include the shelf 580 on which the laundry or the goods may be placed in the second chamber 550.

The shelf 580 may store the laundry or the goods thereon. The shelf 580 may include the plurality of shelves. Additionally, because the shelf 580 is detachable from the shelf mounting portion 5552, the spacing between the plurality of shelves may be adjusted based on the size of the goods being displayed.

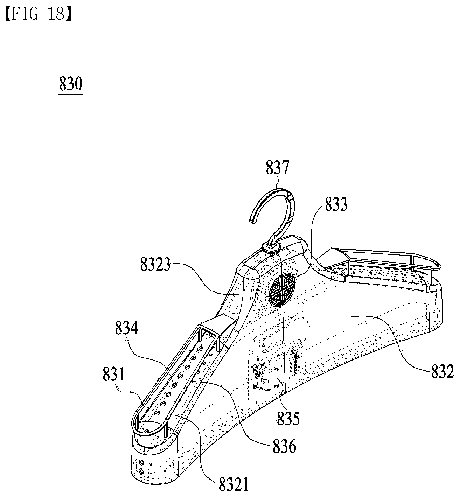

FIG. 18 discloses another example of the laundry hanger 830 in the hanger assembly 800 mounted on the hanger mounting portion 5554. The laundry hanger 830 may include a hanger housing 832 that forms an outer appearance, and a ring 837 that mounts the hanger housing 832 on the hanger mounting portion 5554.

The hanger housing 832 may include a central support 8323 located at a center of the hanger housing 832 and an extension support 8321 extending from the central support 8323 to both sides and supporting the laundry.

Top and side surfaces of the extension support 8321 and a bottom surface of the hanger housing 832, that is, the central support 8323 and a bottom surface of the extension support 8321, may include a steam supply hole 836 that may supply steam to the laundry hung on the laundry hanger 830 and an air supply hole 834 that may supply air.

The steam supply hole 836 and the air supply hole 834 may include a plurality of steam supply holes and a plurality of air supply holes, respectively. Because it is hygienic for steam and air to have different flow channels, the steam supply hole 836 and the air supply hole 834 will not be in communication with each other.

Referring to FIG. 18, the plurality of air supply holes 834 and the plurality of steam supply holes 836 may be arranged along an extension direction of the extension support 8321 in the top and side surfaces of the extension support 8321 and the bottom surface of the hanger housing 832. Accordingly, the plurality of air supply holes 834 and the plurality of steam supply holes 836 may be arranged to face each other along the front and rear direction of the hanger housing 832.

The laundry hanger 830 may further include a steam unit 835 located inside the hanger housing 832 to generate steam and supply steam to the laundry via the steam supply hole 836.

The laundry hanger 830 may further include a hanger fan 833 located in the hanger housing 832 to suck air from the outside of the hanger housing 832 and supply air via the air supply hole 834.

Specifically, an inlet hole (not shown) defined through a front surface of the hanger housing 832 and the hanger fan 833 located in the inlet hole to suck air may be further included.

The laundry hanger 830 may further include a hanger heater (not shown) located at the rear of the hanger fan 833 to heat sucked air. Sucked air may be heated via the hanger heater and then supplied via the air supply hole 834.

Accordingly, the air supply hole 834 will able to supply hot air or air at a room temperature.

The steam unit 835 and the hanger fan 833 may be located at a center of the hanger housing 832. This is to maintain balance of the laundry hanger 830 by reflecting weights of the steam unit 835 and the hanger fan 833.

The laundry hanger 830 may further include a spacer 831 to prevent the laundry hung on the laundry hanger 830 from being in contact with the extension support 8321. The spacer 831 may define a predetermined space between the laundry and the extension support 8321 to prevent the air supply hole 834 and the steam supply hole 836 located in the extension support 8321 from unintentionally clogging by being in contact with the laundry.

FIG. 19 is an example of the second chamber top surface 551. The door assembly 500 may further include an upper lighting 591 disposed on the second chamber top surface 551 to emit light to the goods or the laundry accommodated in the second chamber 550.

Because the purposes of the second chamber 550 include the display, the door assembly 500 may further include lightings 587 and 590 to make the goods or the laundry accommodated in the second chamber 550 to stand out.

The upper lighting 591 may radiate light downward on the accommodated goods. Therefore, the goods illuminated by the upper lighting 591 will be able to achieve a highlight effect that focuses attention of the user looking at the second chamber.

The door assembly 500 may further include a lower auxiliary space (not shown) located separately in a lower portion of the second chamber 550 inside the accommodating body 510. That is, the door assembly 500 may further include the lower auxiliary space (not shown) separated from the second chamber 550 between the accommodating body 510 and the second chamber 550. The lower auxiliary space may include the electrical and mechanical devices required for the door assembly 500.

Accordingly, the second chamber 550 may define a space independent of the first chamber 130. Here, the independent space means that temperature and humidity may be adjusted separately from the first chamber 130. This is to enable the temperature and humidity adjustment independently of the first chamber 130 to use the second chamber 550 for the display and care purposes.

The independent temperature and humidity adjustment of each chamber is to prevent energy from being wasted as hot air or steam is unnecessarily supplied to the other chamber when only one of the first chamber 130 and the second chamber 550 is used. Additionally, both chambers may be used with different temperatures and humidities for different purposes.

The laundry treating apparatus 100 may spray steam or supply hot air using the various electrical or mechanical devices disposed in the auxiliary chamber 150 only when it is necessary to care for the laundry accommodated in the first chamber 130. In addition, the laundry treating apparatus 100 may further include an air conditioner (not shown) separately disposed to dehumidify or heat air of the second chamber 550 to care for the laundry accommodated in the second chamber 550.

The air conditioner may include at least one of an air circulator 560 and an air treater 570, which will be described later.

Referring to FIG. 19, an upper auxiliary space (not shown) defined by the second chamber top surface 551, the accommodating body 510, and the front upper panel 5181 may be located at an upper portion of the door assembly 500. That is, the upper auxiliary space may be located separately in an upper portion of the second chamber 550 inside the accommodating body 510. Additionally, the upper lighting 591 may be located in the upper auxiliary space.

The second chamber top surface 551 may include a lighting installation hole 5512 defined through the second chamber top surface 551. The upper lighting 591 may irradiate light to the second chamber 550 via the lighting installation hole 5512.

The upper lighting 591 may emit light with a color of one of cooldaylight, incandlelight, and daylight colors.

The laundry treating apparatus 100 may include the cabinet 110 including the first inlet 131 defined in the front surface, the first chamber 130 located inside the cabinet 110 and accommodating the laundry via the first inlet 131, the auxiliary chamber 150 located at the lower side inside the cabinet 110 to define the installation space separate from the first chamber 130, the door assembly 500 coupled to the cabinet 110 to open and close the first inlet 131, the second chamber 550 located inside the door assembly 500 and formed separately from the first chamber 130 and the auxiliary chamber 150, and the air circulator 560 disposed in the door assembly 500 to circulate air in the second chamber 550.

That is, the door assembly 500 may further include the air circulator 560 located between the second chamber 550 and the accommodating body 510 to circulate air in the second chamber 550. The air circulator 560 may suck air of the second chamber 550 via the air inlet 51813 defined through the front upper panel 5181 and then discharge air that has passed through the circulator 560 to the second chamber 550 via an air outlet 5511 located in the second chamber top surface 551.

Referring to FIG. 19, the door assembly 500 may further include the air outlet 5511 in which at least a portion of a rear portion of the second chamber top surface 551 is opened along the width direction of the door assembly 500.

The second chamber 550 may be formed by an accommodating housing 559 located inside the accommodating body 510. That is, the accommodating body 510 may include the second inlet 512 in the front surface thereof and may include the accommodating housing 559 forming the second chamber 550 therein. Therefore, the accommodating housing 559 may include the second chamber top surface 551, the second chamber bottom surface 554, the second chamber left side surface 552 and the second chamber right side surface 553 connecting the second chamber top surface 551 with the second chamber bottom surface 554, and the second chamber rear surface 555.

Referring to FIG. 20, the door assembly 500 may include the air circulator 560 located between an accommodating body top surface 5101 and the second chamber top surface 551 to circulate air in the second chamber 550.

FIG. 20 show an example in which the air circulator 560 is located between the accommodating body top surface 5101 and the second chamber top surface 551.

In contrast, when air in the second chamber 550 may be circulated, the air circulator 560 may be located anywhere between the accommodating body 510 and the accommodating housing 559.

Referring to FIGS. 20 and 21, the air circulator 560 may include a circulating flow channel 563 that sucks air from the second chamber 550 and discharges the air and a heater 561 that heats air passing through the circulating flow channel 563. The heater 561 may be located inside the circulating flow channel 563. However, this is only an example and the heater 561 may be located outside the circulating flow channel.

The heater 561 may include a circulating heater 5611 that heats air passing through the circulating flow channel 563. In addition to the circulating heater 5611, the heater 561 may be implemented in other ways as long as it may heat air passing through the circulating flow channel 563. For example, the circulating heater 5611 may be formed in a film shape to surround the circulating flow channel 563.

The circulating flow channel 563 may include a first circulating duct 5631 that sucks air of the second chamber 550 and allows air to flow, and a second circulating duct 5632 that is in communication with the first circulating duct 5631 and allows air sucked into the first circulating duct 5631 to flow and discharges air to the second chamber 550.

The door assembly 500 may further include the air inlet 51813 that is located at the upper portion of the door assembly 500 and is in communication with one end of the circulating flow channel 563 to suck air from the second chamber 550, and the air outlet 5511 that is located at the upper portion of the door assembly 500 and is in communication with the other end of the circulating flow channel 563 to discharge air to the second chamber 550.

The first circulating duct 5631 may be in communication with the air inlet 51813 located in the front panel 518, and the second circulating duct 5632 may be in communication with the air outlet 5511 located in the second chamber top surface 551.

The air circulator 560 may further include a circulating fan 565 that rotates to suck air from the second chamber 550.

The circulating fan 565 may suck air from the second chamber 550 by using a motor to generate a suction force via a fan disposed on the rotation shaft of the motor.

Specifically, the circulating fan 565 may be located on the circulating flow channel. FIG. 20 shows that the circulating fan 565 is disposed inside the first circulating duct 5631, but this is only an example.

For example, the circulating fan 565 may be located adjacent to the air inlet 51813 or adjacent to the air outlet 5511. Inside the circulating flow channel 563, the circulating fan 565 may be located upstream or downstream from the heater 561.

The heater 561 may be located inside the second circulating duct 5632.

Referring to FIG. 20, the air inlet 51813 and the air outlet 5511 may be formed in a rectangular shape along the width direction of the door assembly 500.

Accordingly, a length of the circulating flow channel 563 along the width direction of the door assembly 500 may be greater than a length of the circulating flow channel 563 along the front and rear direction of the door assembly 500.

That is, lengths of the first circulating duct 5631 and the second circulating duct 5632 along the width direction of the door assembly 500 may be greater than lengths of the first circulating duct 5631 and the second circulating duct 5632 along the front and rear direction of the door assembly 500, respectively.

Additionally, the first circulating duct 5631 may be located on the second circulating duct 5632.

Additionally, the first circulating duct 5631 may be located closer to the door of the second chamber than the second circulating duct 5632. This is because circulating air rearward is more advantageous in terms of heat loss than circulating air forward.

Because of a shape of the circulating flow channel 563, the circulating fan 565 may include a plurality of circulating fans. The plurality of circulating fans 565 may be arranged along the width direction of the door assembly 500.

Likewise, the heater 561 may include a plurality of heaters (not shown). Alternatively, a coil heater that generates heat in the single heater may be formed to extend along the width direction of the door assembly 500. That is, the coil heater may be formed in a serpentine shape.

In one example, the door assembly 500 may include a circulating temperature sensor 797 that measures the temperature of the second chamber 550. A detailed description thereof will be made later with reference to FIG. 21.

Referring to FIG. 21, when the door 530 of the second chamber closes the second inlet 512, a predetermined gap G may be defined between the front panel 518 and the door 530 of the second chamber. This is because the inner surface of the door 530 of the second chamber protrudes along an edge or a sealing member (not shown) such as a gasket surrounds the edge of the inner surface of the door 530 of the second chamber. Alternatively, the front panel 518 may be formed to protrude along the edge of the front panel.

Accordingly, because the air inlet 51813 located in the front upper panel 5181 is not in contact with the door of the second chamber, the air inlet 51813 may be in communication with the second chamber 550. That is, when the door 530 of the second chamber closes the second inlet 512, air in the second chamber 550 may flow to the air circulator 560 via the air inlet 51813.

Referring to FIG. 21, the circulating flow channel 563 may be located above the lightings 587 and 590 at a location between the accommodating body top surface 5101 and the second chamber top surface 551.

In one example, the air circulator 560 may adjust the temperature of the second chamber 550 by circulating and simultaneously heating air in the second chamber 550 via the heater 561. A volume of the second chamber 550 is not only smaller than a volume of the first chamber 130, but also the second chamber 550 has a main purpose of adjusting the temperature and the humidity when displaying the laundry hung in the second chamber 550, so that the heater 561 may be sufficient with only the circulating heater 5611 unlike the heat pump module disposed inside the auxiliary chamber 150.

The heater 561 may operate only when heating air in the second chamber 550. That is, when a temperature of air in the second chamber 550 reaches a preset target temperature, the operation of the heater 561 may be stopped.

To this end, the door assembly 500 may include the circulating temperature sensor 797 (see FIG. 20) that measures the temperature of the second chamber 550. The circulating temperature sensor 797 is shown as being disposed at the upper portion of the second chamber 550, but this is only an example. The circulating temperature sensor 797 may be located elsewhere as long as the internal temperature of the second chamber 550 may be measured.

Based on the temperature measured by the circulating temperature sensor 797, the controller 490 may control the circulating fan 565 and the circulating heater 5611. When the temperature is higher than the target temperature, the controller 490 may stop the circulating heater 5611 and operate only the circulating fan 565.

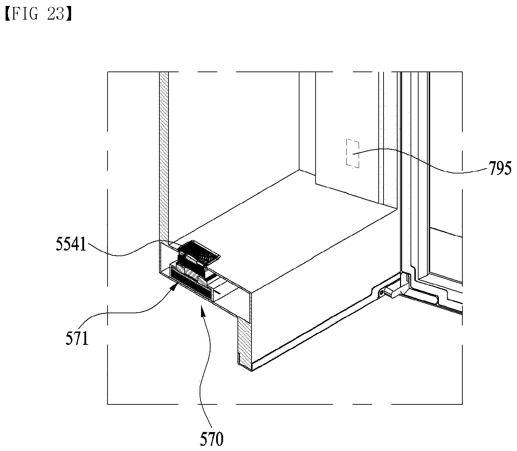

FIG. 22 shows an example of a bottom surface of the second chamber 550. FIG. 23 shows a cross-section of the air treater 570 located between the accommodating body 510 and the accommodating housing 559.

The door assembly 500 may further include the air treater 570 located between the accommodating body 510 and the second chamber bottom surface 554 to suck air from the second chamber 550 and circulate air.

The air treater 570 may circulate air in the second chamber 550 similarly to the air circulator 560. When the air treater 570 is located in the upper portion of the second chamber 550, the air circulator 560 may be located in the lower portion of the second chamber 550.

That is, the air circulator 560 and the air treater 570 may be located anywhere as long as they may purify air in the second chamber 550. Additionally, to condition air in the second chamber 550, only one of the air circulator 560 and the air treater 570 may be disposed.

Referring to FIG. 22, the second chamber bottom surface 554 forming the bottom surface of the second chamber 550 may include a bottom suction hole 5541 and a bottom discharge hole 5542 defined through the second chamber bottom surface 554.

That is, the door assembly 500 may further include the bottom suction hole 5541 located in the bottom surface of the second chamber 550 and in communication with a first treating duct 5731, and the bottom discharge hole 5542 located in the bottom surface of the second chamber 550 and in communication with a second treating duct 5732.

When the bottom suction hole 5541 is located at a center of the second chamber bottom surface 554, the bottom discharge hole 5542 may be located close to the second chamber left side surface 552 or the second chamber right side surface 553. To form a flow channel between the bottom suction hole 5541 and the bottom discharge hole 5542, the bottom discharge hole 5542 and the bottom suction hole 5541 may be arranged along the width direction of the door assembly 500 in the second chamber bottom surface 554.

The bottom suction hole 5541 may include a plurality of bottom suction holes and the plurality of bottom suction holes may be located close to both side surfaces of the second chamber 550. That is, when one bottom suction hole 5541 is close to the second chamber left side surface 552, another bottom suction hole 5541 may be located close to the second chamber right side surface 553.

A bottom suction cover (not shown) including a plurality of through-holes may be coupled to the bottom discharge hole 5541.

The bottom discharge hole 5542 may be defined to extend along the front and rear direction of the door assembly 500 to circulate air along one side surface of the second chamber 550.

Referring to FIGS. 22 and 23, the air treater 570 may include a treating flow channel 573 that defines a flow channel through which air of the second chamber 550 passes, and a dehumidifier 571 that is disposed in the treating flow channel 573 to dehumidify air passing through the treating flow channel 573.

While the air circulator 560 includes the heater 561 to supply hot air to the second chamber, the air treater 570 may include the dehumidifier 571 to dehumidify air of the second chamber 550.

The dehumidifier 571 may be implemented using a heat pump unit, but considering a size of a space where the dehumidifier is installed, the dehumidifier 571 may include a thermoelectric module 5711 utilizing the Peltier effect.

Unlike the air supply 410, which circulates and dehumidifies air in the first chamber 130, the dehumidifier 571, which circulates and dehumidifies air in the second chamber 550, circulates air in the second chamber 550 that has the volume relatively smaller than the volume of the first chamber 130, so that a bulky device such as a heat pump may not be needed.

In addition, because the second chamber 550 is to maintain the temperature and the humidity during the display, much condensate will not be generated as in a drying process of drying the laundry by operating the air supply 410 in the first chamber 130. Therefore, moisture condensed in the dehumidifier 571 may naturally evaporate.

In one example, the door assembly 500 may include a lower lighting 599 that is located on the second chamber bottom surface 554 and emits light upward. This is to irradiate light to the goods to be disposed on the second chamber bottom surface 554. Because the second chamber bottom surface 554 includes the bottom discharge hole 5542 and the bottom suction hole 5541, the lower lighting 599 may be formed in a shape of a plate in a remaining area excluding those.

The lower lighting 599 may emit light with a color of one of cooldaylight, incandlelight, and daylight colors.

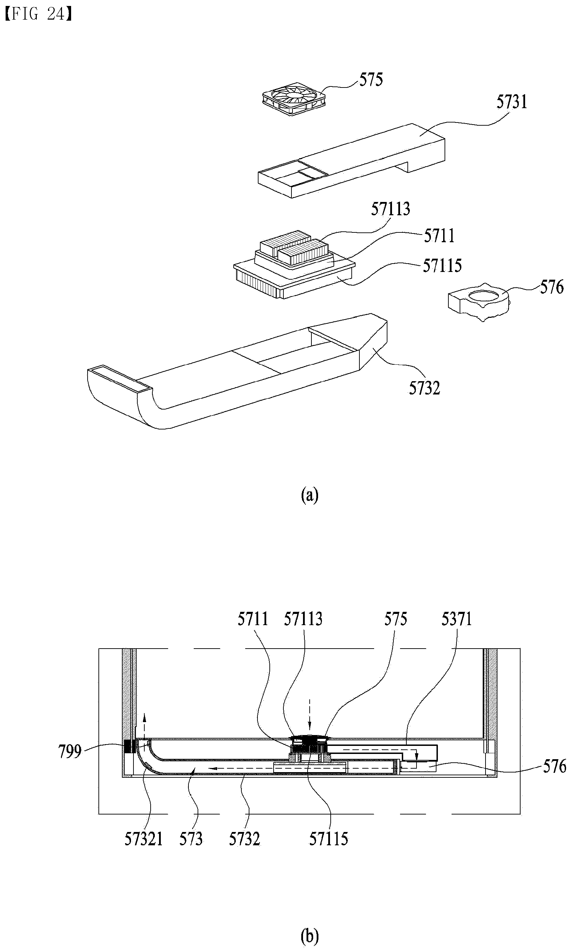

FIG. 24 shows an example of the air treater 570. The air treater 570 may include the treating flow channel 573 that circulates air in the second chamber 550 and the dehumidifier 571 that dehumidifies air passing through the treating flow channel 573.

The dehumidifier 571 may be formed in a shape of the thermoelectric module and may be located on the circulating flow channel. When electricity is applied to the thermoelectric module because of the Peltier effect, a temperature of one surface of the thermoelectric module will fall below the room temperature and a temperature of the other surface will rise above the room temperature.

Accordingly, air passing through the one surface will be cooled and dehumidified as a temperature thereof drops. On the other hand, air passing through the other surface that is heated will become high-temperature dry air as a temperature thereof rises.

The dehumidifier 571 may include the thermoelectric module 5711, a thermoelectric cooler 57113 that is coupled to the one surface, which is cooled, among the surfaces of the thermoelectric module 5711 to cool air, and a thermoelectric heater 57115 that is coupled to the other surface to heat air.

The thermoelectric cooler 57113 may be formed in a fin shape to expand a cross-sectional area of the one surface, which is cooled, of the thermoelectric module 5711, thereby enabling more effective heat exchange with air passing through the thermoelectric cooler 57113. The fin may include a plurality of fins, and may be referred to as a cooling fin.

The thermoelectric heater 57115 may also be formed in the fin shape to expand a cross-sectional area of one surface, which is heated, of the thermoelectric module 5711, thereby enabling more effective heat exchange with air passing through the thermoelectric heater 57115. The fin may include a plurality of fins, and may be referred to as a heating fin. The thermoelectric heater 57115 is originally to dissipate heat from the other surface, which is heated, of the thermoelectric module 5711 to the outside, but this means that the thermoelectric heater 57115 heats air passing through the thermoelectric heater 57115.

The treating flow channel 573 may include the first treating duct 5731 that sucks air from the second chamber 550 and allows air to flow, and the second treating duct 5732 that is in communication with the first treating duct 5731 to allow air that has passed through the first treating duct 5731 to the second chamber to be discharged.

The first treating duct 5731 and the second treating duct 5732 may include the thermoelectric cooler 57113 and the thermoelectric heater 57115 of the thermoelectric module 5711, respectively. Accordingly, air passing through the first treating duct 5731 will be dehumidified, and air passing through the second treating duct 5732 will be heated.

Specifically, air sucked in communication with the bottom suction hole 5541 will be cooled by the thermoelectric module 5711 while passing through the first treating duct 5731. Air cooled while passing through the first treating duct 5731 will be heated by the thermoelectric module 5711 while passing through the second treating duct 5732.

To this end, the thermoelectric module 5711 may be located between the first treating duct 5731 and the second treating duct 5732, so that the thermoelectric cooler 57113 may be in contact with the first treating duct 5731 or may be inserted into the first treating duct 5731, and the thermoelectric heater 57115 may be in contact with the second treating duct 5732 or may be inserted into the second treating duct 5732.

That is, the first treating duct 5731 may be located upward of the second treating duct 5732.

The first treating duct 5731, the thermoelectric module 5711, and the second treating duct 5732 may be stacked sequentially from top to bottom.

The air treater 570 may include a treating fan 575 for circulating air in the second chamber 550 via the treating flow channel 573.

In one example, to smoothly dissipate heat of the thermoelectric heater 57115 separately from the treating fan 575, the thermoelectric heater 57115 may include a heat dissipating fan 576. Additionally, the thermoelectric heater 57115 may be a plurality of heat dissipating fins or a plurality of heating fins formed in the fin shape.

That is, the air treater 570 may further include the treating fan 575 that is located in the first treating duct 5731, sucks air from the second chamber 550, and guides air to the thermoelectric cooler 57113, and the heat dissipating fan 576 that guides air that passes through the first treating duct 5731 to the second treating duct 5732.

The fin-shaped thermoelectric heater 57115 may be attached to the thermoelectric module 5711 to help dissipate heat from the thermoelectric module 5711 and effectively heat air passing through the second treating duct 5732.

The heat dissipating fan 576 may increase a speed of air passing through the treating flow channel 573 such that heat transfer occurs more effectively in the thermoelectric heater 57115. Air passing through the second treating duct 5732 will ultimately be able to more effectively exchange heat with the thermoelectric heater 57115 by the heat dissipating fan 576. To this end, the heat dissipating fan 576 may be located between the thermoelectric cooler 57113 and the thermoelectric heater 57115.

Because a space between the second chamber bottom surface 554 and an accommodating body bottom surface 5104 is narrow, the first treating duct 5731 and the second treating duct 5732 may be disposed to extend along the width direction of the door assembly 500.

In addition, as shown in FIG. 24, because one thermoelectric module 5711 performs the cooling and the heating simultaneously, the first treating duct 5731 and the second treating duct 5732 may be formed in the stacked structure. The first treating duct 5731 may be located on the second treating duct 5732.

The thermoelectric module 5711 will be located between the first treating duct 5731 and the second treating duct 5732.

Accordingly, air introduced into the first treating duct 5731 via the bottom suction hole 5541 may meet the thermoelectric cooler 57113 and be cooled, so that air will flow towards one side surface of the accommodating body 510 along the first treating duct 5731, that is, along the width direction of the door assembly 500.

Thereafter, air passing through the first treating duct 5731 will flow to the second treating duct 5732 that is in communication with one end of the first treating duct 5731. Because the second treating duct 5732 is located under the first treating duct 5731, air passing through the first treating duct 5731 will also flow downward.

Air that has entered the second treating duct 5732 will flow toward the other side surface of the accommodating body along the second treating duct 5732, that is, along the width direction of the door assembly 500.

That is, because of narrowness of the space in which the air treater 570 is mounted, when the bottom discharge hole 5542 is located closer to one side surface among both side surfaces of the accommodating body than to the other side surface around the bottom suction hole 5541, the first treating duct 5731 may be formed to extend in a direction opposite to the side on which the bottom discharge hole 5542 is located, that is, toward the other side surface of the accommodating body 510.

Accordingly, a length of the second treating duct 5732 may be greater than a length of the first treating duct 5731.

The air treater 570 may further include the treating fan 575 to smoothly suck air of the second chamber 550 in the first treating duct 5731. Additionally, the air treater 570 may further include the heat dissipating fan 576 to smoothly dissipate heat from the thermoelectric module 5711 and allow air in the treating flow channel to flow quickly.

The air treater 570 may adjust the humidity of the second chamber 550 by circulating and simultaneously dehumidifying air in the second chamber 550 via the dehumidifier 571. Not only is the volume of the second chamber 550 smaller than the volume of the first chamber 130, but also the main purpose of the second chamber 550 is the adjustment of the temperature and the humidity when displaying the laundry hung in the second chamber 550, so that the dehumidifier 571 may be sufficient with only the thermoelectric module 5711 unlike the heat pump unit disposed inside the auxiliary chamber 150.

The dehumidifier 571 may operate only when dehumidifying air in the second chamber 550. That is, when the humidity of air in the second chamber 550 reaches a preset target humidity, the operation of the dehumidifier 571 may be stopped.

To this end, the door assembly 500 may include a humidity sensor 795 (see FIG. 23) that measures the humidity of the second chamber 550. Based on the humidity measured by the humidity sensor 795, the controller 490 may control the treating fan 575, the heat dissipating fan 576, and the thermoelectric module 5711. When the humidity is lower than the target humidity, the controller 490 may stop the thermoelectric module 5711 and the heat dissipating fan 576 and operate only the treating fan 575.

The humidity sensor 795 is shown as being located at a lower portion of one of both side surfaces 552 and 553 of the second chamber 550, but the humidity sensor 795 may be located anywhere as long as it may measure the humidity of the second chamber 550.

In addition, to allow air discharged via the second treating duct 5732 to be discharged into the second chamber 550 with a temperature equal to or higher than a preset treatment temperature, the air treater 570 may further include an auxiliary heater 57321 inside the second treating duct 5732. To this end, the air treater 570 may further include a treatment temperature sensor 799 located inside the second treating duct 5732 to measure the temperature of discharged air. However, unlike this, the treatment temperature sensor 799 may be located elsewhere.

The humidity sensor 795 may be a temperature/humidity sensor that may also measure the temperature. In this case, the treatment temperature sensor 799 may be replaced by the humidity sensor 795. Accordingly, the humidity sensor 795 may be disposed in the second treating duct 5732 to measure the temperature and the humidity of air that has passed through the thermoelectric heater 57115. Accordingly, rotation speeds of the heat dissipating fan 576 and the treating fan 575 may be controlled or current flowing in the thermoelectric module may be adjusted.

The controller 490 may control the auxiliary heater 57321 to operate when the temperature measured via the treatment temperature sensor 799 is lower than the treatment temperature.

The treatment temperature may be the same as or different from the target temperature. This is because the temperature of the second chamber 550 is set to the target temperature, but the temperature of air discharged from the air treater 570 may be different therefrom. This is because an amount of air circulating via the air circulator 560 is greater than an amount of air circulating via the air treater 570.

To this end, the door assembly 500 may separately include temperature sensors 797 and 799 for the air circulator 560 and the air treater 570. Otherwise, the air circulator 560 and the air treater 570 may share the circulating temperature sensor 797.

Additionally, the air circulator 560 and the air treater 570 may operate independently of the air supply 410 and the steam supply 450 required to care for the laundry hung on the laundry support 600 of the first chamber 130. That is, the temperature and the humidity of air in the second chamber 550 may be adjusted regardless of the first chamber 130.

Additionally, when necessary, only one of the air circulator 560 and the air treater 570 may operate.

This is because the second chamber 550 has the purpose of display and storage, and thus, needs to be constantly adjusted in the temperature and the humidity. To this end, the second chamber 550 may be formed by the separate space that is separated from the first chamber 130 and the auxiliary chamber 150, and the air circulator 560 and the air treater 570 may be connected to operate independently of the air supply 410 and the steam supply 450.

Accordingly, even when the door assembly 500 opens the first inlet 131, the air circulator 560 and the air treater 570 may operate. On the other hand, when the door assembly 500 is opened, the operations of the air supply 410 and the steam supply 450 may be stopped. This is for safety.

That is, the air circulator 560 and the air treater 570 may operate even when the door assembly 500 is opened.

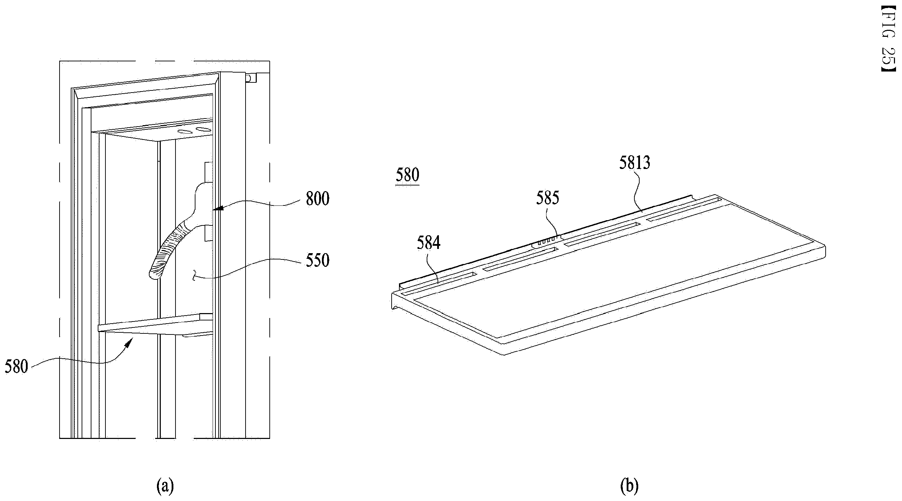

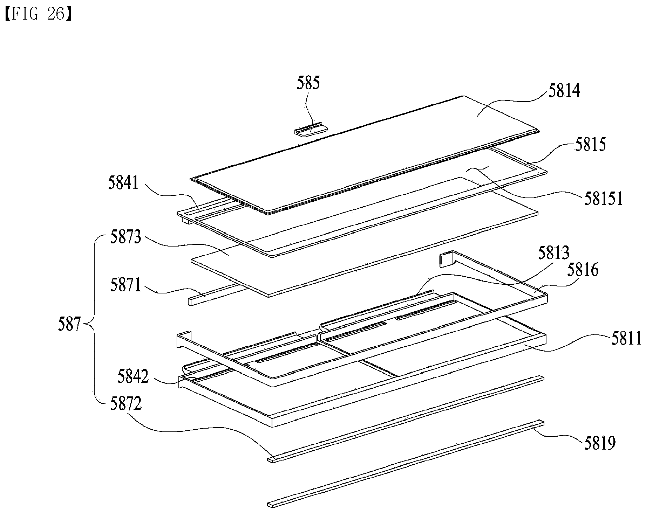

(a) in FIG. 25 shows the shelf 580 located in the second chamber 550 to support the goods accommodated in the second chamber 550. (b) in FIG. 25 is an example of the shelf 580. FIG. 26 is an example of the shelf 580 disassembled.

As shown in (a) in FIG. 25, the hanger assembly 800 for hanging the laundry and the shelf 580 for supporting the goods may be located in the second chamber 550. Because the shelf 580 may include the plurality of shelves and be detachable from the second chamber 550, when hanging the laundry on the hanger assembly 800, the user may separate some of the plurality of shelves 580.

The plurality of shelves 580 may be arranged along the height direction of the door assembly 500 at the uniform spacing. Alternatively, some of the plurality of shelves 580 may be arranged at different spacings.

(b) in FIG. 25 shows an example of the shelf 580 disposed in the second chamber 550. A width of a cross-section of the accommodating body cut at an arbitrary vertical level may decrease from the second inlet 512 toward the accommodating body rear surface 5105. This is to prevent a portion of the accommodating body 510 from interfering with the first chamber 130 when the door assembly 500 is opened and closed.

The shelf 580 may include the shelf coupling portion 811 to be coupled to the shelf mounting portion 5552 located in the second chamber rear surface 555. The shelf coupling portion 811 may be formed to protrude from the shelf 580 toward the second chamber rear surface 555 so as to be inserted into a hole or a groove defined in the shelf mounting portion 5552.

Because the shelf mounting portion 5552 extends along the width direction of the door assembly 500, the shelf coupling portion 811 may also be formed to correspond thereto.

In one example, the shelf 580 may be coupled only to the second chamber rear surface 555 and not to both side surfaces of the second chamber 550. This is because, when the shelf 580 is coupled to three surfaces, it may be difficult for the user to separate or couple the shelf 580 and air circulation in the second chamber 550 may be blocked.

Accordingly, because the shelf 580 is formed in the shape of the square plate, there may be a predetermined gap between each of both side surfaces of the second chamber 550 and each of both side surfaces of the shelf 580.

Additionally, the air circulator 560 discharges air via the air outlet 5511, which is located at the rear portion of the second chamber top surface 551. Therefore, the shelf may include a ventilating portion defined through the shelf 580 along the height direction of the door assembly 500 at a rear portion of the shelf 580 for air circulation by the air circulator 560.

The ventilating portion 584 will prevent air circulating along the height direction of the door assembly 500 from being interrupted by the shelf 580 at the rear portion of the second chamber 550.

That is, air flowing downward from the second chamber top surface 551 via the air outlet 5511 may pass through the ventilating portion 584.

That is, the door assembly 500 may further include the shelf 580 detachably disposed on the rear surface of the second chamber 550 and the ventilating portion 584 defined through the shelf 580, and the ventilating portion 584 may be located closer to the rear surface of the second chamber 550 than the first inlet 131, so that air discharged via the air circulator 560 may flow via the ventilating portion 584.

Additionally, both left and right ends of the shelf 580 may not be coupled to the second chamber 550, but may be spaced apart from both side surfaces 552 and 553 of the second chamber 550. Air discharged from the air treater 570 may circulate via the gap.

That is, air discharged via the bottom discharge hole 5542 located adjacent to one of both side surfaces 552 and 553 of the second chamber 550 will be able to circulate inside the second chamber 550 via the gap.

The shelf 580 may simply be formed in the shape of the plate. However, because the second chamber 550 is used for displaying the laundry or the goods, the door assembly 500 may further include the lightings 587 and 590 to make the laundry or the goods accommodated and displayed in the second chamber 550 stand out.

The lightings 587 and 590 may include the second chamber lighting 590 disposed in the second chamber 550 and the shelf lighting 587 disposed in the shelf 580.

In other words, the door assembly 500 may further include the shelf 580 located in the second chamber 550 to support the goods accommodated in the second chamber 550, and the shelf 580 may include a shelf body 5811 with an open top surface, an upper cover 5814 that is coupled to the open top surface of the body 5811 to support the goods, and the shelf lighting 587 disposed on the shelf body 5811 to emit light.

Additionally, the shelf lighting 587 may include an upper shelf lighting 5871 that irradiates light via the upper cover 5814 and a lower shelf lighting 5872 that irradiates light downward of the shelf body 5811.

When the upper shelf lighting 5871 is intended to illuminate the goods disposed on the upper cover 5814 upward, the lower shelf lighting 5872 is intended to illuminate the goods disposed on another shelf 580 located below downward.

FIG. 26 is an exploded view of the shelf 580. The shelf 580 may include a shelf body assembly 581 that supports the goods displayed in the second chamber 550. The shelf body assembly 581 may include the shelf body 5811 with the open top surface and the upper cover 5814 coupled to the open top surface of the shelf body 5811 to support the goods.

The shelf 580 may further include a side frame 5816 and an upper frame 5815 to reinforce strength in consideration of a weight of the goods supported by the shelf 580. The side frame 5816 may be coupled to surround front and both side surfaces of the shelf body 5811. The upper frame 5815 may be coupled to the open top surface of the shelf body portion 581 to support the upper cover 5814.

The shelf 580 may further include the ventilating portion 584 for air circulating along the height direction of the second chamber 550. The ventilating portion 584 may include a body ventilating hole 5841 located at the rear portion of the shelf body assembly 581 and defined through the shelf body 5811 and an upper ventilating hole 5842 defined through the upper frame 5815. When the shelf body 5811 and the upper frame 5815 are coupled to each other, the body ventilating hole 5841 and the upper ventilating hole 5842 will face each other.

The body ventilating hole 5841 and the upper ventilating hole 5842 may be defined to extend along the width direction of the door assembly 500. Additionally, the body ventilating hole 5841 and the upper ventilating hole 5842 may include a plurality of ventilating holes and a plurality of upper ventilating holes, respectively. Even when the body ventilation hole 5841 and the upper ventilation hole 5842 include the plurality of ventilating holes and the plurality of upper ventilating holes, respectively, a shape and the number of body ventilating holes 5841 will be the same as those of upper ventilating holes 5842, and the body ventilating hole 5841 and the upper ventilating hole 5842 will face each other.

The upper frame 5815 may include a cover installation hole 58411 defined through the upper frame. The cover installation hole 58411 may be defined to allow light from shelf lighting 587, which will be described later, to pass therethrough.

The cover installation hole 58411 may be located in front of the upper ventilating hole 5842. The upper cover 5814 may be coupled to the cover installation hole 58411. Therefore, a shape of the cover installation hole 58411 will correspond to a shape of the upper cover 5814.

The shelf 580 may further include the shelf lighting 587 disposed on the shelf body 5811 to emit light. The shelf lighting 587 may include the upper shelf lighting 5871 for illuminating light to the goods supported on the upper cover 5814 and the lower shelf lighting 5872 for illuminating light to the goods located below the shelf body 5811.

The upper shelf lighting 5871 is for illuminating light to the goods supported on the upper cover 5814, and the lower shelf lighting 5872 is for illuminating light to the goods located below the shelf 580. This is because the shelf 580 may include the plurality of shelves and another shelf 580 may be located below one shelf 580.

To improve the display effect, it will be desirable for the upper shelf lighting 5871 to emit light via an entirety of the plate-shaped upper cover. On the other hand, the lower shelf lighting 5872 may be formed in a linear form, so that light may be focused only on the goods. This is because, based on the goods, spotlight lighting is desirable from above, and soft lighting is desirable from below.

To this end, the shelf lighting 587 may include a shelf light diffuser 5873 for diffusion and reflection of the upper shelf lighting 5871. The shelf light diffuser 5873 may be made of a light diffusion material.

This is because using the light diffusion material may prevent the user from being dazzled by the lighting and make light emitted from the lighting soft and subtle. Therefore, an interior design may be better when the light diffusion material is used than when the light diffusion material is not used.

The shelf light diffuser 5873 may be located under the upper cover 5814 and diffuse light of the upper shelf lighting 5871 in a plate shape. Therefore, light from the upper shelf lighting 5871 will be diffused in the form of the plate via the shelf light diffuser 5873 and then irradiated to the goods via the upper cover 5814.

In one example, the upper cover 5814 may be made of a transparent or translucent material to allow light to pass therethrough.

Each of the upper shelf lighting 5871 and the lower shelf lighting 5872 may include a light-emitting diode (LED). The upper shelf lighting 5871 and the lower shelf lighting 5872 may be in the linear shape. Accordingly, the upper shelf lighting 5871 and the lower shelf lighting 5872 may emit light with a color of one of cooldaylight, incandlelight, and daylight colors.

The upper shelf lighting 5871 may be formed to extend along the width direction of the door assembly 500 inside the shelf body 5811.

While the upper shelf lighting 5871 irradiates light in the form of the plate via the shelf light diffuser 5873, the lower shelf lighting 5872 may irradiate light in a form of a line.

The lower shelf lighting 5872 may irradiate light downward of the shelf 580. To this end, the lower shelf lighting 5872 may be disposed under the shelf body 5811. The shelf 580 may further include a lower shelf lighting cover 5819 to cover the lower shelf lighting 5872.

The lower shelf lighting cover 5819 may be made of a transparent or translucent material.

The lower shelf lighting 5872 may be located in a lower front side of the shelf body 5811.

The lower shelf lighting 5872 may be located at a front side inside the shelf body 5811 and may irradiate light via a lower lighting through-hole (not shown) defined through the shelf body 5811. In this regard, the lower shelf lighting cover 5819 may be coupled to the lower lighting through-hole (not shown).

Accordingly, the upper shelf lighting 5871 may irradiate light forward from a rear side of the shelf body 5811. Light emitted forward will be reflected and diffused by the shelf light diffuser 5873, pass through the upper cover 5814, and travel upward of the shelf 580.

On the other hand, light emitted by the lower shelf lighting 5872 will be directed downward of the shelf 580 via the lower shelf lighting cover 5819.

The shelf 580 may include a shelf power supply 585 to supply power to the shelf lighting 587. To prevent a wire from being exposed, the shelf power supply 585 may be formed to protrude from the rear side of the shelf 580 toward the second chamber rear surface 555.

Accordingly, the shelf power supply 585 may be located at a center of the shelf coupling portion 811. Correspondingly, the shelf mounting portion 5552 may include a shelf power connecting portion 55523 into which the shelf power supply 585 is inserted.

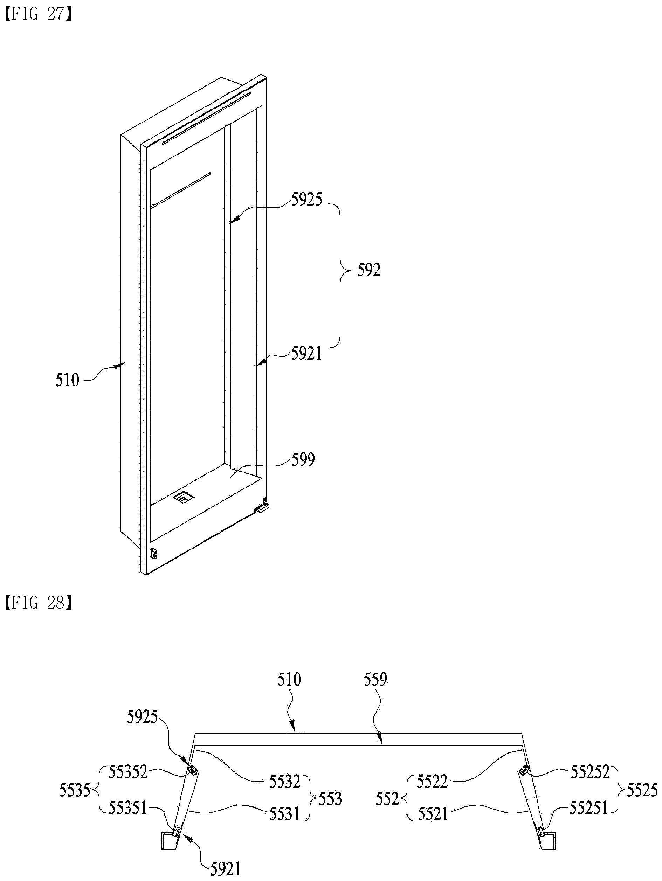

FIG. 27 is a cross-section of an accommodating body cut along a front and rear direction of the accommodating body. The door assembly 500 may include the second chamber lighting 590 that is disposed in the second chamber 550 and illuminates the interior of the second chamber 550. In addition to the upper lighting 591 and the lower lighting 599 described above, the second chamber lighting 590 may further include a line lighting 592 located in each of the second chamber top surface 551 and both side surfaces 553 and 554 of the second chamber 550.

That is, the laundry treating apparatus 100 may include the cabinet 110 including the first inlet 131 defined in the front surface thereof, the first chamber 130 located inside the cabinet 110 and accommodating the laundry therein through the first inlet 131, the auxiliary chamber 150 located at the lower side inside the cabinet 110 to define the installation space separated from the first chamber 130, and the door assembly 500 that is coupled to the cabinet 110 to open and close the first inlet 131.

In addition, the door assembly 500 may include the accommodating body 510 including the second inlet 512 defined in the front surface thereof, the accommodating housing 559 disposed inside the accommodating body 510, in communication with the outside via the second inlet 512, and forming the second chamber 550 separated from the first chamber 130 and the auxiliary chamber 150, and the line lighting 592 disposed in the accommodating housing 559 in parallel with the second inlet 512 to illuminate the second chamber 550.

As described above, since the accommodating housing 559 may form the second chamber 550, so that the accommodating housing 559 may include the second chamber top surface 551 that forms the top surface of the second chamber 550, the second chamber left side surface 552 and the second chamber right side surface 553 extending downward from both left and right ends of the second chamber top surface 551 to form the left and right side surfaces of the second chamber 550, respectively, and the second chamber rear surface 555 connected to the second chamber top surface 551, the second chamber left side surface 552, and the second chamber right side surface 553 to form the rear surface of the second chamber 550.

The line lighting 592 may be disposed along a perimeter of the second chamber 550 in a form of a line. That is, the line lighting 592 may be disposed along each of the second chamber top surface 551, the second chamber left side surface 552, and the second chamber right side surface 553.

The line lighting 592 may be disposed along the width direction of the door assembly 500 in the second chamber top surface 551. The line lighting 592 may be disposed along the height direction of the door assembly 500 in each of the second chamber left side surface 552 and the second chamber right side surface 553.

The line lighting 592 disposed along the width direction of the door assembly 500 in the second chamber top surface 551 may be disposed along the height direction of the door assembly 500 in each of the second chamber left side surface 552 and the second chamber right side surface 553.

The line lighting 592 may be arranged in two lines along the second chamber top surface 551, the second chamber left side surface 552, and the second chamber right side surface 553. The line lighting 592 may include a front line lighting 5921 located at the front side of the second chamber 550 and a rear line lighting 5925 located at the rear of the front line lighting 5921 in the second chamber 550.

That is, the line lighting 592 may include the front line lighting 5921 located closer to the second inlet 512 than the second chamber rear surface 555, and the rear line lighting 5925 located closer to the second chamber rear surface 555 than the second inlet 512.

The upper lighting 591 may be located in the second chamber top surface 551 between the front line lighting 5921 and the rear line lighting 5925.

The rear line lighting 5925 may be located between the front line lighting 5921 and the second chamber rear surface 555.

The front line lighting 5921 and the rear line lighting 5925, like the shelf lighting 587, the upper lighting 591, and the lower lighting 599, may change color depending on selection of the user.

The front line lighting 5921 may emit light with a color of one of cooldaylight, incandlelight, and daylight colors. Rather than always emitting light with the color of only one of cooldaylight, incandlelight, and daylight colors, the front line lighting 5921 may emit any color via color conversion. Additionally, the front line lighting 5921 may emit light of an RGB color. Here, the RGB color refers to a color created using trichromacy alone or in combination. This may be implemented by the LED.

Likewise, the rear line lighting 5925 may also emit light with cooldaylight, incandlelight, daylight, and RGB colors.

When the front line lighting 5921 plays a role of key lighting that creates an atmosphere of the second chamber 550, the rear line lighting 5925 may play a role of back lighting that further enhances the atmosphere created by the key lighting at the rear side of the second chamber 550.

Accordingly, the user will be able to change the color of the line lighting 592 to create a desired atmosphere to make the goods stand out in the second chamber. For example, the controller 490 may create various atmospheres using the line lighting 592, the shelf lighting 587, the upper lighting 591, and the lower lighting 599 based on the selection of the user. This is because the controller 490 is able to change intensities and colors (or wavelengths) of the line lighting 592, the shelf lighting 587, the upper lighting 591, and the lower lighting 599.

Additionally, the user may adjust intensities and colors (or wavelengths) of light emitted from the line lighting 592, the shelf lighting 587, the upper lighting 591, and the lower lighting 599 to suit preference thereof.

FIG. 28 shows a location where the line lighting 592 is installed. When the door assembly 500 is cut at an arbitrary vertical level, the door assembly 500 may include a separate space defined between the accommodating body 510 and the accommodating housing 559. In the separate space, mechanical devices, wire devices, and the like disposed for the second chamber may be disposed.

The second chamber left side surface 552 and the second chamber right side surface 553 may be inclined along the front and rear direction of the door assembly 500. Accordingly, a width between the second chamber left side surface 552 and the second chamber right side surface 553 may decrease from the second inlet 512 to the second chamber rear surface 555.

The second chamber left side surface 552 may include a first left side surface 5521 and a second left side surface 5522. The second chamber left side surface 552 may further include a left connecting surface 5523 connecting the first left side surface 5521 with the second left side surface 5522. The left connecting surface 5523 may be formed to be bent from one end of the first left side surface 5521 toward the accommodating body left side surface and may be connected to one end of the second left side surface 5522.

The second chamber right side surface 553 may include a first right side surface 5531 and a second right side surface 5532. The second chamber right side surface 553 may further include a right connecting surface 5533 connecting the first right side surface 5531 with the second right side surface 5532. The right connecting surface 5533 may be formed to be bent from one end of the first right side surface 5531 toward the accommodating body right side surface and may be connected to one end of the second left side surface 5522.

The front line lighting 5921 may include a first front line lighting 59211 installed in the second chamber top surface 551, a second front line lighting 59213 installed in the second chamber left side surface 552, and a third front line lighting 59215 installed in the second chamber right side surface 553.

In other words, the front line lighting 5921 may include the first front line lighting 59211 disposed along the width direction of the door assembly 500 in the second chamber top surface 551, the second front line lighting 59213 disposed along the height direction of the door assembly 500 in the second chamber left side surface 552, and the third front line lighting 59215 disposed along the height direction of the door assembly 500 in the second chamber right side surface 553.

The second front line lighting 59213 and the third front line lighting 59215 may be installed in the first left side surface 5521 and first right side surface 5531, respectively. Further, a second rear line lighting 59253 and a third rear line lighting 59255 may be installed in the left connecting surface 5523 and the right connecting surface 5533, respectively.

The first left side surface 5521 may include a front left installation portion 55251 defined along the height direction in the first left side surface 5521. The front left installation portion 55251 may be defined in a form of a groove or a hole, so that the second front line lighting 59213 may be inserted into the front left installation portion 55251.

The left connecting surface 5523 may include a rear left installation portion 55252 defined along the height direction in the left connecting surface 5523. The rear left installation portion 55252 may be defined in a form of a groove or a hole, so that the second rear line lighting 59253 may be inserted into the rear left installation portion 55252.

The first right side surface 5531 may include a front right installation portion 55351 defined along the height direction in the first right side surface 5531. The front right installation portion 55351 may be defined in a form of a groove or a hole, so that the third front line lighting 59215 may be inserted into the front right installation portion 55351.

The right connecting surface 5533 may include a rear right installation portion 55352 defined along the height direction in the right connecting surface 5533. The rear right installation portion 55352 may be defined in a form of a groove or a hole, so that the third rear line lighting 59253 may be inserted into the rear left installation portion 55252.

The first front line lighting 59211 and a first rear line lighting 59251 may be inserted into a front upper installation portion 5515 and a rear upper installation portion 5515 defined in the second chamber top surface 551, respectively. The front upper installation portion 5515 and the rear upper installation portion 5515 may also be defined in a form of a hole or a groove.

The front line lighting 5921 and the rear line lighting 5925 may illuminate different areas of the second chamber 550.

Considering an arrangement structure of the front line lighting 5921 and the rear line lighting 5925, the first front line lighting 59211 may irradiate light downward from the front side of the second chamber top surface 551. The second front line lighting 59213 may irradiate light from the first left side surface 5521 toward the first right side surface 5531 and the door 530 of the second chamber. The third front line lighting 59215 may irradiate light from the first right side surface 5531 toward the first left side surface 5521 and the door 530 of the second chamber. This is because the first left side surface 5521 and the first right side surface 5531 are constructed to be inclined.

The rear line lighting 5925 may include the first rear line lighting 59251 disposed along the width direction of the door assembly 500 in the second chamber top surface 551, the second rear line lighting 59253 disposed along the height direction of the door assembly 500 in the second chamber left side surface 552, and the third rear line lighting 59255 disposed along the height direction of the door assembly 500 in the second chamber right side surface 553.

The first rear line lighting 59251 may irradiate light downward from the rear side of the second chamber top surface 551. The second rear line lighting 59253 may irradiate light from the left connecting surface 5523 toward the second chamber rear surface 555. The third rear line lighting 59255 may irradiate light from the right connecting surface 5533 toward the second chamber rear surface 555. This is because, as the left connecting surface 5523 and the right connecting surface 5533 are bent toward the accommodating body from the first left side surface 5521 and the first right side surface 5531, respectively, the left connecting surface 5523 and the right connecting surface 5533 face the second chamber rear surface 555 at an angle.

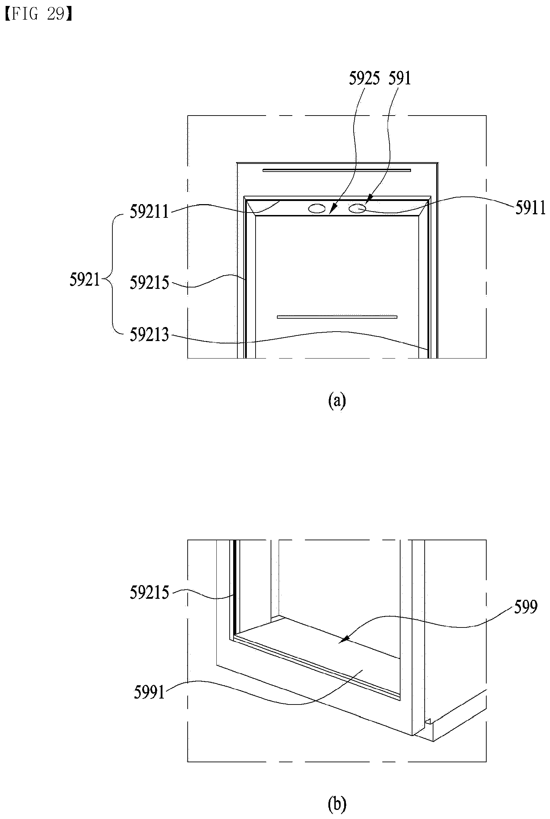

(a) in FIG. 29 is an example of the upper lighting 591 and front line lighting 5921. (b) in FIG. 29 is an example of the lower lighting 599.

The upper lighting 591 may be disposed in the lighting installation hole 5512 defined through the second chamber top surface 551. The lighting installation hole 5512 may include a plurality of lighting installation holes, and the upper lighting 591 may also include a plurality of upper lightings accordingly. The upper lighting 591 may illuminate the laundry hung on the hanger mounting portion 5554.

The upper lighting 591 may further include an upper lighting cover 5911 to prevent moisture or dust from entering the upper lighting 591.

(a) in FIG. 29 shows an example of the front line lighting 5921. The front line lighting 5921 may include the first front line lighting 59211 disposed in the second chamber top surface 551, and the second front line lighting 59213 and the third front line lighting 59215 disposed in both side surfaces of the second chamber 550, respectively.

The first front line lighting 59211 may be formed to extend along the width direction of the door assembly 500. The second front line lighting 59213 and the third front line lighting 59215 may be formed to extend along the height direction of the door assembly 500.

The rear line lighting 5925 may be located at the rear of the upper lighting 591. The rear line lighting 5925 may include the first front line lighting 59211 disposed in the second chamber top surface 551, and the second front line lighting 59213 and the third front line lighting 59215 disposed in both side surfaces of the second chamber 550, respectively.

Referring to (b) in FIG. 29, the lower lighting 599 may be formed in a shape of a plate. That is, the door assembly 500 may further include the lower lighting 599 that irradiates light upward from the second chamber bottom surface 554 toward the second chamber 550.

The lower lighting 599 may illuminate light in a form of the plate onto the bottom surface of the second chamber 550. To this end, the lower lighting 599 may include a light diffuser made of the light diffusion material, similar to the upper shelf lighting 5871 of the shelf lighting 587.

The door assembly 500 may have the lower lighting 599 that is located in the second chamber bottom surface 554 and emits light upward. This is to irradiate light to the goods to be disposed on the second chamber bottom surface 554. Because the second chamber bottom surface 554 includes the bottom discharge hole 5542 and the bottom suction hole 5541, the lower lighting 599 may be formed in the shape of the plate in a remaining area excluding those.

The lower lighting 599 and the upper lighting 591 may emit light with cooldaylight, incandlelight, and daylight colors.

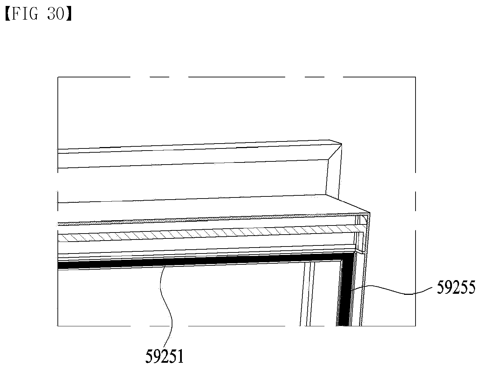

FIG. 30 is an example of the first rear line lighting 59251 and the third rear line lighting 59255 of the rear line lighting 5925. The first rear line lighting 59251 may be located between the upper lighting 591 and the air outlet 5511 in the second chamber top surface 551. The third rear line lighting 59255 may be located in the right connecting surface 5533 and be directed in an opposite direction of the second inlet 512. The second rear line lighting 59253 may also be located in the left connecting surface 5523 and be directed in the opposite direction of the second inlet 512.

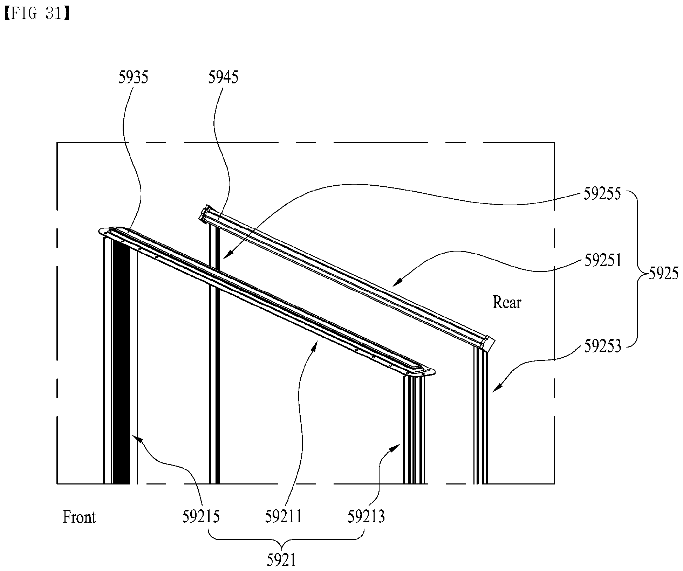

FIG. 31 shows an example of the front line lighting 5921 and the rear line lighting 5925 formed in a linear form.

The front line lighting 5921 may include the first front line lighting 59211 installed in the second chamber top surface 551, the second front line lighting 59213 installed in the first left side surface 5521, and the third front line lighting 59215 installed in the first right side surface 5531. The first front line lighting 59211 will be formed to extend along the width direction of the door assembly 500, and the second front line lighting 59213 and the third front line lighting 59215 will be formed to extend along the height direction of the door assembly 500.

The rear line lighting 5925 may include the first rear line lighting 59251 installed in the second chamber top surface 551, the second rear line lighting 59253 installed in the second chamber left side surface 552, and the third rear line lighting 59255 installed in the second chamber right side surface 553. The first rear line lighting 59251 will be formed to extend along the width direction of the door assembly 500, and the second rear line lighting 59253 and the third rear line lighting 59255 will be formed to extend along the height direction of the door assembly 500.

The first rear line lighting 59251 will be located between the upper lighting 591 and the air outlet 5511. The second chamber top surface 551 may further include a rear upper installation portion 55152 located between the lighting installation hole 5512 and the air outlet 5511 and into which the first rear line lighting 59251 is inserted.

The first shelf line lighting may be located between the upper lighting 591 and the second inlet 512. The second chamber top surface 551 may further include a front upper installation portion 55151 located between the lighting installation hole 5512 and the second inlet 512 and into which the first front line lighting 59211 is inserted.

When the line lighting 592 irradiates light toward the second chamber 550, heat is generated, so that it is necessary to dissipate the heat generated by the line lighting 592. Additionally, air in the second chamber 550 may be hot or contain a lot of moisture. Therefore, it is necessary to protect the line lighting 592 from hot air or moisture.

To this end, the line lighting 592 may be equipped with a protective cover and a heat dissipating structure. FIG. 31 shows that the front line lighting 5921 and the rear line lighting 5925 have different types of protective covers and heat dissipating structures, but alternatively, they may have the same type of protective cover and heat dissipating structure.

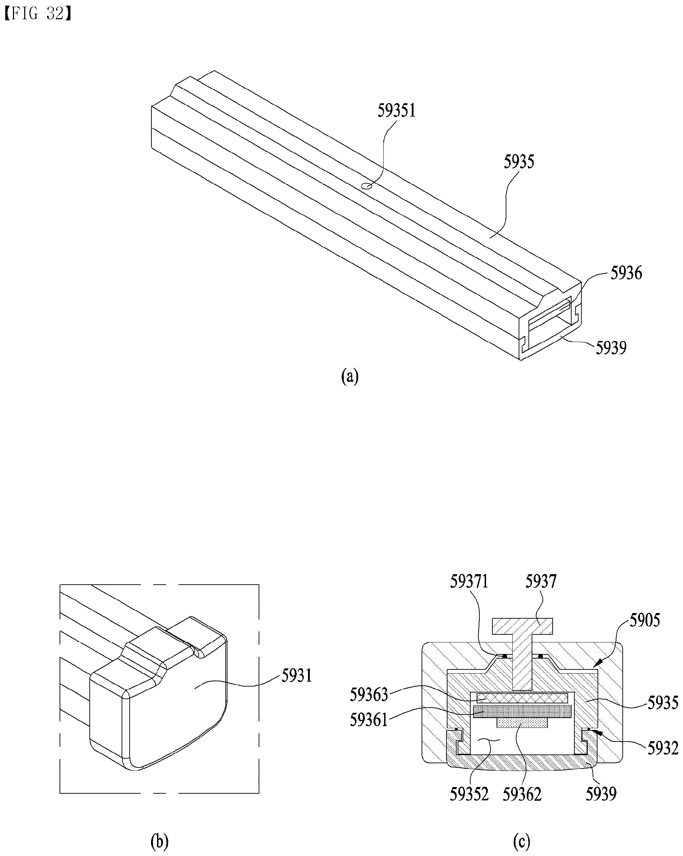

(a) to (c) in FIG. 32 show an example of a protective cover and heat dissipating and moisture proof structures disposed on the line lighting 592 when the line lighting 592 is inserted into the accommodating housing 559 in a line lighting installation portion 5905 defined in the second chamber.

Referring to (a) to (c) in FIG. 32, the line lighting 592 may include a linear heat dissipating body 5935 including an insertion hole 59352 defined in one surface thereof, an LED assembly 5936 inserted into the heat dissipating body, and a protective cover 5939 coupled to the insertion hole 59352 to protect the LED assembly 5936.

The heat dissipating body 5935 may be formed in a shape of a long line in one direction and may have a U-shaped or channel-shaped cross-section.

The protective cover 5939 may be made of the light diffusion material. Therefore, the protective cover 5939 may function as a light diffuser. Additionally, the protective cover 5939 may be made of a translucent or transparent material. The protective cover 5929 may be hooked to the heat dissipating body 5935.

Referring to (b) in FIG. 32, when the heat dissipating body 5935 and the protective cover 5939 are coupled to each other, both ends of the heat dissipating body are in communication with the outside. To prevent this, the line lighting 592 may further include an end cover 5931 coupled to each of both ends of the line lighting 592.

The heat dissipating body 5935 may be made of aluminum, which is not only resistant to moisture but also has effective heat transfer performance.

Referring to (c) in FIG. 32, the line lighting 592 may be inserted and coupled into an upper installation portion 5515, a left installation portion 5525, and a right installation portion 5535 defined in the second chamber 550. The heat dissipating body 5935 may further include a body fastening hole 59351 defined through one surface of the heat dissipating body 5935 in a direction opposite to the insertion hole.

The line lighting 592 may be coupled to an inner surface of the accommodating housing 559 by a fastening member 5937 via the body fastening hole 59351.

That is, the line lighting 592 may be coupled to each of the second chamber top surface 551, the second chamber left side surface 552, and the second chamber right side surface 553 via the body fastening hole 59351 using the fastening member 5937.

The body fastening hole 59351 may include a plurality of body fastening holes.

The door assembly 500 may further include the line lighting installation portion 5905 defined in parallel with the second inlet 512 as a portion of the inner surface of the accommodating housing 559 is recessed toward the accommodating body 510, and into which the protective cover 5939 is inserted.

In other words, the door assembly 500 may include the line lighting installation portion 5905 defined along the second chamber top surface 551 and both side surfaces of the second chamber 550.

Specifically, the line lighting installation portion 5905 may include the upper installation portion 5515 located in the second chamber top surface 551, the left installation portion 5525 located in the second chamber left side surface 552, and the right installation portion 5535 located in the second chamber right side surface 553. The line lighting installation portion 5905 may have a groove shape or a hole shape.

The upper installation portion 5515 may include the front upper installation portion 5515 and the rear upper installation portion 5515. The left installation portion 5525 may include the front left installation portion 55251 and the rear left installation portion 55252. Additionally, the right installation portion 5535 may include the front right installation portion 55351 and the rear right installation portion 55352.

The LED assembly 5936 may include an LED 59362 that emits light, and a printed circuit board (PCB) 59361 that is coupled to the LED 59362 to control the LED 59362. The LED 59362 may further include a heat transfer member 59363 that transfers heat generated from the LED 59362 and the PCB 59361 to the heat dissipating body 5935.

In one example, the line lighting 592 may further include a body sealing member 5932 disposed between the protective cover 5939 and the heat dissipating body 5935 to prevent penetration of moisture and foreign substances. That is, the body sealing member 5932 may be located between the protective cover 5939 and the heat dissipating body 5935 and prevent moisture or foreign substances from the second chamber 550 from penetrating into the heat dissipating body 5935.

In addition, an area where the heat dissipating body 5935 is coupled to the line lighting installation portion 5905 by the fastening member 5937 may further include a fastening sealing member 59371 to prevent moisture and foreign substances from penetrating.

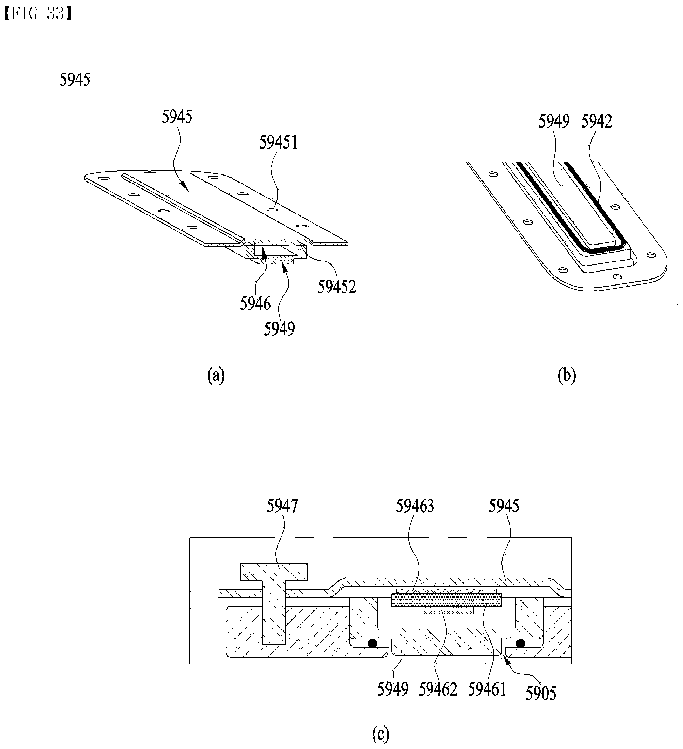

(a) to (c) in FIG. 33 show another example of a protective cover 5949 and heat dissipating and moisture proof structures disposed on the line lighting 592 when the line lighting 592 is inserted into the line lighting installation portion 5905 defined in the second chamber from the outside of the accommodating housing 559.

FIG. 32 shows an example in which the line lighting installation portion 5905 is defined in the shape of the groove, while FIG. 33 shows another example in which the line lighting installation portion 5905 is defined in the shape of the hole. Accordingly, the line lighting 592 in the type to be inserted shown in FIG. 32 may be coupled to the line lighting installation portion 5905 inside the accommodating housing 559. On the other hand, the line lighting 592 in the type to be fixed shown in FIG. 33 may be coupled to the line lighting installation portion 5905 in the form of the hole in communication with the inside of the second chamber 550 on the outside of the accommodating housing 559. That is, the line lighting 592 shown in FIG. 32 may be coupled to the inner surface of the accommodating housing 559 forming the second chamber 550, while the line lighting 592 shown in FIG. 33 may be coupled to an outer surface of the accommodating housing 559 forming the second chamber 550 and partially inserted into the second chamber 550.

Referring to (a) in FIG. 33, the line lighting 592 may include a plate-shaped heat dissipating body 5945 including a recessed portion 59452 on one surface, an LED assembly 5946 inserted into the heat dissipating body, and the protective cover 5949 that is coupled to the heat dissipating body 5945 and protects the LED assembly 5946.

The heat dissipating body 5945 may be formed to extend in one direction, and the recessed portion 59452 may be recessed in a direction toward the accommodating body 510 when the heat dissipating body 5945 is coupled to the accommodating housing 559.

The protective cover 5949 may be formed in an angled U-shape or a channel shape and may define a space in which the LED assembly 5946 may be accommodated when coupled to the plate-shaped heat dissipating body 5945. That is, the protective cover 5949 may cover the LED assembly 5946.

The protective cover 5949 may be made of the light diffusion material.

The protective cover 5949 may function as a light diffuser. Additionally, the protective cover 5949 may be made of a translucent or transparent material. The protective cover 5929 may be formed in the U-shape or the channel shape and may be coupled to the heat dissipating body 5935.

The heat dissipating body 5945 may include a body fastening hole 59451 defined therethrough along a perimeter of the recessed portion 59452. The heat dissipating body 5945 may be made of aluminum, which is not only resistant to moisture but also has effective heat transfer performance.

The body fastening hole 59451 may be include a plurality of body fastening holes.

Referring to (b) in FIG. 33, the LED assembly 5946 and the protective cover 5949 may be located in the recessed portion 59452 and coupled to the heat dissipating body 5945. In addition, a body sealing member 5942 to prevent penetration of moisture and foreign substances when the heat dissipating body 5945 is coupled to the line lighting installation portion 5905 may be further included along a perimeter of the protective cover 5949.

The protective cover 5949 may have a stepped top surface that emits light. The body sealing member 5942 may be located on the stepped surface formed around the top surface of the protective cover 5949. In this regard, while the body sealing member 5942 is coupled to the outer surface of the accommodating housing 559, a portion of the top surface of the protective cover 5949 may be inserted into the second chamber 550.

The line lighting installation portion 5905 may be defined through the inner surface of the accommodating housing 559 in parallel with the second inlet 512 along the inner surface of the accommodating housing 559 such that the protective cover 5949 is inserted thereinto. Accordingly, in the line lighting 592, when the plate-shaped heat dissipating body 5945 is coupled to the outer surface of the accommodating housing 559, the portion of the top surface of the protective cover 5949 may be inserted into the second chamber.

The heat dissipating body 5945 may be coupled to the line lighting installation portion 5905 using a fastening member 5947. When the protective cover 5949 is inserted into the second chamber 550 via the line lighting installation portion 5905, the protective cover 5949 and the inner surface of the accommodating housing 559 may form a smooth surface without a step.

Referring to (b) and (c) in FIG. 33, the line lighting 592 may be inserted into and coupled to the line lighting installation portion 5905 defined in the second chamber 550.

The line lighting installation portion 5905 may include the upper installation portion 5515 located in the second chamber top surface 551, the left installation portion 5525 located in the second chamber left side surface 552, and the right installation portion 5535 located in the second chamber right side surface 553.

The upper installation portion 5515 may include the front upper installation portion 5515 and the rear upper installation portion 5515. The left installation portion 5525 may include the front left installation portion 55251 and the rear left installation portion 55252. Additionally, the right installation portion 5535 may include the front right installation portion 55351 and the rear right installation portion 55352.

Accordingly, the line lighting 592 may be coupled to the second chamber top surface 551, the second chamber left side surface 552, and the second chamber right side surface 553 via the body fastening hole 59451 using a fastening member 5947.

The LED assembly 5946 may include an LED 59462 that emits light, and a printed circuit board (PCB) 59361 that is coupled to the LED 59462 to control the LED 59462. The LED 59462 may further include a heat transfer member 59463 that transfers heat generated from the LED 59462 and the PCB 59461 to the heat dissipating body 5945.

FIG. 35 is an exploded view of another example of the laundry support 600 disposed in the laundry treating apparatus 100. As described above, after the hanger body 610 is extended forward, the user will hang the laundry on the extended hanger body 610 from a lateral direction of the cabinet 110. In this case, the user may have an inconvenience of not being able to hang the laundry in front of the laundry treating apparatus 100. To solve such problem, in the example of the laundry support 600 shown in FIG. 35, the hanger body 610 is extended forward from the hanger support 620 and then rotated so as to be disposed again along the width direction of the cabinet.

That is, the hanger body 610 may be separated from the hanger support 620, moved forward, and then rotated relative to the hanger support 620 so as to be disposed along the width direction of the cabinet 110.

Referring to FIG. 35, the laundry support 600 may include the hanger body 610 disposed along the front and rear direction of the cabinet 110 to hang the laundry thereon, and the hanger support 620 disposed along the front and rear direction of the cabinet 110, located on the hanger body 610, and supporting the hanger body 610.

The laundry support 600 may further include the laundry driver 640 that generates the rotational force, and the rotatable portion 661 and the converting member 662 that convert the rotational movement of the laundry driver 640 into a reciprocating movement along the front and rear direction of the cabinet 110. When the rotatable portion 661 rotates away from a rotation shaft of the motor, the converting member 662 will convert the rotational movement into the reciprocating linear movement in the front and rear direction. This uses a so-called Scotch-York mechanism.

The converting member 662 is shown to disposed on the hanger support 620 in a form of a groove or a hole defined along the front and rear direction of the cabinet 110, but the present disclosure is not limited thereto.

Referring to (b) in FIG. 34, the laundry driver 640 may be located between the first chamber top surface 132 and the cabinet top surface 112 to prevent exposure to the user and to prevent failure caused by steam and hot air supplied to the first chamber.

Additionally, the laundry driver 640 may be supported by a driver frame 649 that supports the laundry driver 640. The driver frame 649 may also be located between the first chamber top surface 132 and the cabinet top surface 112, and both ends of the driver frame 649 may be coupled to the cabinet frame (not shown) that forms the skeleton of the cabinet 110 or the cabinet 110. This is to prevent vibration of the laundry driver 640 from being transmitted into the first chamber 130.

The laundry support 600 may include the hanger body 610 that is spaced downwardly apart from the hanger support 620 and hangs the laundry thereon. The hanger support 620 and the hanger body 610 may extend along the front and rear direction of the cabinet 110.

The hanger support 620 and the hanger body 610 may be coupled with each other to perform the reciprocating movement by the laundry driver 640.

The hanger body 610 may include the hanger bar 611 that may hang the laundry thereon. Further, the hanger bar 611 may include the plurality of hanger grooves 6111 defined along the hanger bar 611. FIG. 35 shows the plurality of hanger grooves 6111 arranged at the uniform spacing, but the spacings between the plurality of hanger grooves 6111 may not be uniform.

The hanger body 610 is coupled to the hanger support 620, but is able to be extended forward via the first inlet 131 when necessary. That is, the hanger body 610 may further include a movement guide 618 for moving the hanger body 610 forward relative to the hanger support 620 and rotating the hanger body 610.

The movement guide 618 may be connected to the hanger support 620 to move the hanger body 610, and the hanger body 610 coupled with the movement guide 618 may be moved in the front and rear direction of the cabinet 110 by the movement guide 618. That is, the movement guide 618 may move and rotate in determined directions by a hanger body guide 629 disposed in the hanger support 620.

That is, the movement guide 618 may protrude from the hanger body 610 and be coupled with the hanger body guide 619.

The hanger body guide 629 may include a hanger body guide hole 6295 extending through a hanger support body 6201 and extending along the front and rear direction of the cabinet 110, and a coupling guide member 6293 disposed at one end of the hanger body guide hole 6295 and rotatably accommodating the movement guide 618 therein. As the movement guide 618 is inserted into and movably coupled to the hanger body guide hole 6295, the hanger body 610 may move along the front and rear direction of the cabinet 110. In addition, when the movement guide 618 is accommodated in the coupling guide member 6293 located at one end of the hanger body guide hole 6295, the user may rotate the hanger body 610, so that the hanger body 610 is disposed in the width direction of the cabinet 110. This is described in detail in FIGS. 37 to 39.

Referring to FIGS. 35 and 36, the hanger support 620 may include a hanger bar guide member 6291 coupled to the hanger support body 8201 at a location corresponding to the hanger body guide hole 6295. The hanger bar guide member 6291 may further include a hanger body movement hole 6292 that extends through the hanger bar guide member 6291 in the front and rear direction of the cabinet 110 and overlaps at least a portion of the hanger body guide hole 6295. Additionally, the coupling guide member 6293 may be located at one end of the hanger body moving hole 6292. This is to improve durability because the hanger support 620 and the hanger body 610 are components to be operated.

Accordingly, the coupling guide member 6293 may be located on the hanger support body 6201 or on the hanger bar guide member 6291.

Likewise, the hanger body 610 may further include a hanger bar moving member 6104 made of a material different from that of the hanger bar 611 to improve rigidity and durability. The movement guide 618 may be coupled to the hanger bar moving member 6104.

The hanger body 610 and the hanger support 620 may be coupled to each other by the hanger bar stopper 621. This is to prevent the hanger body 610 and the hanger support 620 from being separated from each other during the reciprocating movement in the front and rear direction of the cabinet 110.

As described above, when the door 501 is closed, the hanger body 610 and the hanger support 620 may be fixed by the hanger bar stopper 621 so as to be prevented from moving relative to each other. because this is merely a specific example, the hanger body 610 and the hanger support 620 may be fixed relative to each other in another way.

FIG. 36 shows an example in which the hanger body 610 and the hanger support 620 are coupled to each other. When the hanger body 610 performs the function of hanging the laundry thereon, the hanger support 620 may support the hanger body 610 so as to be movable, and may perform the reciprocating movement in the front and rear direction of the cabinet 110 by the laundry driver 640 together with the hanger body 610.

The hanger support 620 may further include a hanger support body 6201 disposed in parallel with and spaced upwardly apart from the hanger bar 611, and a first support 622 and a second support 624 supporting both ends of the hanger support body 6201. The first support 622 and the second support 624 will support the hanger support 620 when the hanger support 620 performs the reciprocating movement along the front and rear direction by the converting member 662.

Referring to FIG. 36, the hanger body 610 may further include the upper hanger body 612 (see FIG. 15) located on the hanger bar 611 to prevent interference with the hanger support 620 when the user hangs the laundry in the hanger groove 6111.

The hanger bar 611 and the upper hanger body 612 may be spaced apart from each other and face each other. The hanger bar 611 and the upper hanger body 612 may be spaced apart from each other and located in parallel with each other to define the hanging space 6101 (see FIG. 35), which is the space accessible to the user to hang the laundry, and may be coupled to each other by each connecting body 619 connecting each of both ends of the hanger bar 611 with each of both ends of the upper hanger body 612.

That is, the hanging space 6101 may be defined by the hanger bar 611, the upper hanger body 612, and each connecting body 619 connecting each of both ends of the hanger bar 611 with each of both ends of the upper hanger body 612.

In other words, the hanger body 610 may include the hanging space 6101 defined therethrough in the width direction of the cabinet 110 when being disposed in the front and rear direction of the cabinet 110. The plurality of hanger grooves 6111 where the laundry may be hung may be located in a bottom surface of the hanging space 6101.

Referring to FIG. 35 and \ in FIG. 37, the laundry driver 640 may include the power motor 641 and the power transmitter 642 that is connected to the rotation shaft of the power motor and transmits power to the power converter. FIG. 15 shows the driving pulley 6423, the driven pulley 6422, and the belt 6421 connecting the driving pulley 6423 with the driven pulley 6422 as an example of the power transmitter 642, but the present disclosure is not limited thereto.

Additionally, the laundry driver 640 may further include the pulley fixing member 6425 that is supported on the moving hanger frame and supports the driven pulley 6422.

The power converter 660 may include the rotatable portion 661 that is connected to the driven pulley 6422 and rotates together when the driven pulley 6422 rotates, and the converting member 662 that converts the rotation of the rotatable portion 661 into the reciprocating movement. The rotatable portion 661 will be able to perform a circular movement with a certain radius by the rotatable portion 661 disposed to be spaced apart from a rotation shaft direction of the driven pulley 6422 to rotate.

Specifically, the rotatable portion 661 may include a rotatable connecting member connected to the rotation shaft at one end, an arm member connected to the other end of the rotatable connecting member in a direction perpendicular to the rotation shaft, and an inserted rotatable member connected to the converting member 662 in a direction parallel to the rotation shaft at the other end of the arm member.

The laundry driver 640 may be supported by a driver frame 649 that supports the laundry driver 640. The driver frame 649 may also be located between the first chamber top surface 132 and the cabinet top surface 112, and both ends of the driver frame 649 may be coupled to the cabinet frame (not shown) that forms the skeleton of the cabinet 110 or the cabinet 110. This is to prevent the vibration of the laundry driver 640 from being transmitted into the first chamber 130.

Referring to (a) in FIG. 37, the hanger body 610 and the hanger support 620 overlap each other in the height direction of the cabinet 110. An arranged state in which a center of the hanger body 610 coincides with a center of the hanger support 620 may be referred to as a first arrangement.

Referring to (b) in FIG. 37, when lengths of the hanger body 610 and the hanger support 620 are the same in the first arrangement, both ends of the hanger body 610 and both ends of the hanger support 620 will coincide with each other. Even when the lengths of the hanger body 610 and the hanger support 620 are different from each other, the centers of the hanger body 610 and the hanger support 620 will coincide with each other based on the front and rear direction of the cabinet. At this time, it may be said that the hanger body 610 is located at a first hanger location M1 of the hanger support 620.

(b) in FIG. 37 is an enlarged view of the hanger body movement hole 6292, the hanger body guide hole 6295, the coupling guide member 6293, and the hanger bar moving member 6104 and the movement guide 618 disposed in the hanger body 610. The hanger bar guide member 6291 is disposed such that at least a portion of the hanger body movement hole 6292 overlaps the hanger body guide hole 6295. Therefore, (b) in FIG. 37 shows an example in which the hanger body movement hole 6292 and the hanger body guide hole 6295 overlap each other and a portion of the hanger bar moving member 6104 is exposed via the hanger body movement hole 6292 and the hanger body guide hole 6295. In this regard, the movement guide 618 may be located at one (specifically, a rear end) of both ends of the hanger body movement hole 6292.

The hanger body movement hole 6292 extends along the front and rear direction of the cabinet 110. When the movement guide 618 is located at a rear end of the hanger body movement hole 6292, a corresponding location may be referred to as the first location. Further, at the first hanger location M1, the hanger body 610 and the hanger support 620 may be arranged in the first arrangement.

The coupling guide member 6293 may be made of an elastic material and may fix or movably accommodate the movement guide 618. The coupling guide member 6293 may be formed in a shape of being opened in the front and rear direction of the cabinet 110. That is, the coupling guide member 6293 may be located at a front end of the hanger body movement hole 6292, specifically a second hanger location M2, which will be described later, to avoid interference with the hanger body movement hole 6292. Because the cabinet 110 is opened in the front and rear direction, the coupling guide member 6293 may include a first guide wall 6293a and a second guide wall 6293b. The first guide wall 6293a and the second guide wall 6293b may be formed as curved surfaces and may be arranged along the hanger body movement hole 6292.

(a) in FIG. 38 shows a state while the user is opening the door 501 and extending the hanger body 610 forward, (b) in FIG. 38 shows a state in which the movement guide 618 has moved from one end of the hanger body movement hole 6292 to the other end (specifically, the front end). (c) in FIG. 38 is an enlarged view of the hanger body movement hole 6292, the hanger body guide hole 6295, the coupling guide member 6293, and the movement guide 618 and the hanger bar moving member 6104 disposed in the hanger body 610 when the hanger body 610 has moved forward relative to the hanger support 620.

Referring to (b) in FIG. 38, an arrangement of the hanger body 610 and the hanger support 620 will be referred to as a second arrangement, and it may be said that the hanger body 610 moves from the first hanger location M1 to the second hanger location M2 on the hanger support 620.

That is, the first arrangement means that the hanger body 610 and the hanger support 620 overlap each other such that the center of the hanger body 610 and the center of the hanger support 620 coincide with each other based on the front and rear direction of the cabinet 110. On the other hand, the second arrangement means that the hanger body 610 moves forward of the hanger support 620 based on the front and rear direction of the cabinet 110 and a portion of the hanger body 610 and the hanger support 620 overlap each other.

In the second arrangement, one end (specifically, the rear end) of the hanger body 610 is located between both ends of the hanger support 620, while the other end (specifically, the front end) of the hanger body 610 is positioned forwardly of both ends of the hanger support 620.

The second hanger location M2 will be located ahead of the first hanger location M1. Preferably, the movement guide 618 will be located in a middle area of the hanger bar moving member 6104 based on the front and rear direction of the cabinet 110. Accordingly, when the movement guide 618 is located at the second hanger location M2, another portion of the hanger bar moving member 6104 may be exposed via the hanger body movement hole 6292 and the hanger body guide hole 6295.

Because the coupling guide member 6293 is disposed at the second hanger location M2, the movement guide 618 may be accommodated inside the coupling guide member 6293 at the second location M2. Because the coupling guide member 6293 is made of an elastic material, the coupling guide member 6293 will be able to stably support the movement guide 618. When the movement guide 618 reaches the second hanger location M2, the movement guide 618 enters the coupling guide member 6293. Because both wings disposed on an outer surface of the movement guide 618 are located between the first guide wall 6293a and the second guide wall 6293b, the movement guide 618 is not able to move in the width direction of the cabinet 110 and is not able to rotate unless there is an external force.

Directions of both wings may be the same as the front and rear direction of the cabinet 110, that is, the front and rear direction of the hanger body 610. Accordingly, when the movement guide 618 reaches the second hanger location M2, the first guide wall 6293a and the second guide wall 6293b may fix the movement guide 618.

(a) in FIG. 39 shows a state in which the user has extended the hanger body 610 forward and then has rotated the hanger body 610 90 degrees (°). (b) in FIG. 39 shows a state in which the movement guide 618 rotates at the second hanger location M2. (c) in FIG. 39 is an enlarged view of the hanger body movement hole 6292, the hanger body guide hole 6295, the coupling guide member 6293, and the movement guide 618 and the hanger bar moving member 6104 disposed in the hanger body 610 when the hanger body 610 rotates 90 degrees (°) with respect to the hanger support 620.

When the hanger body 610 is extended only forward as shown in FIG. 13, the hanger body 610 will be extended in the front and rear direction. Therefore, considering that in a case of a general wardrobe, the hanger bar is disposed in the width direction, it may be preferable to place the hanger body 610 in the width direction of the cabinet 110 for user convenience.

To this end, the hanger body 610 may be disposed in the width direction of the cabinet 110 via the rotation at the second hanger location M2. A case in which the hanger body 610 and the hanger support 620 are arranged in different directions may be referred to as a third arrangement. The hanger body 610 may be disposed in the pivoting direction of the cabinet 110 via the rotation at the second hanger location M2, that is, inside the coupling guide member 6293.

When the movement guide 618 rotates by the user's external force, the coupling guide member 6293 may stably fix the movement guide 618 again by elasticity.

The movement guide 618 may be cylindrical, but may have the wings at radially opposite locations. The coupling guide member 6293 may stably fix the movement guide 618 like a kind of hook by the wing.

Referring to (c) in FIG. 39, it may be seen that, because the hanger body 610 has rotated by 90 degrees, the hanger bar moving member 6104 is not visible via the hanger body guide hole 6295 and the hanger body movement hole 6292, at least partially overlapping each other.

The first arrangement is an arrangement that may be achieved regardless of whether the door 501 is open or closed, while the second arrangement and the third arrangement are arrangements that may be achieved only when the door 501 is open. This is to prevent interference between the door 501 and the laundry support 600, especially the hanger body 610. Therefore, when the door 501 is closed, only the first arrangement will be able to be achieved.

When the hanger body 610 rotates by 90 degrees (°) again, the hanger body 610 will return to the second arrangement in the form of moving forward of the hanger support 620. In this regard, when the hanger body 610 is moved rearward again, the hanger body 610 will return to the first arrangement. In other words, to change the arrangement of the hanger body 610 from the third arrangement to the first arrangement, the above-described order may be reversed.

When the laundry driver 640 rotates, the hanger body 610 and the hanger support 620 will perform the reciprocating movement along the front and rear direction. Therefore, when the hanger body 610 is retracted, the hanger body 610 may unintentionally move in the front and rear direction along the moving rail 615 when the coupling with the hanger support 620 is not fixed.

This is a problem that occurs because the extension and retraction direction of the hanger body 610 is the same as the reciprocating movement direction of the hanger body 610.

To solve this problem, referring to (a) in FIG. 40, shown is a state in which the hanger body 610 is fixed to the hanger support 620 as the movement preventing protrusion 625 of the hanger bar stopper 621 is inserted into the hanger bar fixing groove 613. (b) in FIG. 40 is an enlarged view of a case in which the movement preventing protrusion 625 is separated from the hanger bar fixing groove 613.

The laundry support 600 may further include a fixing portion 616 disposed on one of the hanger support 620 and the hanger body 610 to detachably couple the hanger support 620 and the hanger body 610 to each other.

The hanger bar stopper 621 is an example of the fixing portion. That is, the laundry support 600 may further include the hanger bar fixing groove 613 defined in one of the hanger support 620 and the hanger body 610, and the hanger bar stopper 621 disposed on the other of the hanger support 620 and the hanger body 610 and capable of inserting the movement preventing protrusion 625 into the hanger bar fixing groove 613 depending on whether the door assembly 500 is opened.

The hanger bar stopper 621 may include the movement preventing protrusion 625 that may be inserted into the hanger bar fixing groove 613, and the moving motor 626 (see FIG. 17) for inserting the movement preventing protrusion 625 into the hanger bar fixing groove 613 or separating the same from the hanger bar fixing groove 613.

The hanger bar stopper 621 may automatically insert the preventing movement protrusion 625 into the hanger bar fixing groove 613 or separate the same from the hanger bar fixing groove 613 depending on whether the door assembly 500 or the door 501 is opened.

To this end, the laundry treating apparatus 100 may further include the door opening/closing sensor 793 (see FIG. 5) that senses whether the door assembly 500 or the door 501 is opened or closed. When the door opening/closing sensor 793 senses that the door assembly 500 or the door 501 is opened, the controller 490 will operate the moving motor 626 to separate the movement preventing protrusion 625 from the hanger bar fixing groove 613. In contrast, when the door opening/closing sensor 793 senses that the door assembly 500 or the door 501 is closed, the controller 490 will operate the moving motor 626 in an opposite direction to insert the movement preventing protrusion 625 into the hanger bar fixing groove 613.

That is, when the door 501 closes the inlet 131, the controller 490 will insert the movement preventing protrusion 625 into the hanger bar fixing groove 613 to couple the hanger body 610 with the hanger support 620. This is to prepare for the reciprocating movement of the laundry support 600 in the future.

When the door 501 is opened, there is a high possibility that the user will extend the hanger body 610 forward, so that in preparation therefor, the controller 490 may separate the movement preventing protrusion 625 from the hanger bar fixing groove 613. Therefore, even when the user moves the hanger body 610 forward, interference between the hanger body 610 and the movement preventing protrusion 625 will not occur.

FIG. 41 shows another example of the fixing portion 616. Referring to (a) in FIG. 41, instead of the hanger bar stopper 621, to fix the hanger body 610 and the hanger support 620, the laundry support 600 may include a hook accommodating portion 614 defined at portions of front ends of the hanger body 610 and the hanger support 620 and defined when the hanger body 610 and the hanger support 620 are in the first arrangement, and a hook 6161 accommodated in the hook accommodating portion 614 and maintaining the first arrangement of the hanger body 610 and the hanger support 620.

The hook accommodating portion 614 may have various shapes, but (b) in FIG. 41 shows an example in which the hook accommodating portion 614 is defined as a hook accommodating groove 6141 in a form of a groove.

When the hook 6161 couples the hanger body 610 with the hanger support 620, the hook 6161 and both front ends of the hanger body 610 and the hanger support 620 may form a coplanar surface with each other. in this regard, the hook 6161 will be coupled to a hook coupling portion 6143 disposed in the hook accommodating groove 6151.

The hook 6161 may further include an elastic member (not shown) so as to be separated from the hook coupling portion 6153 and protrude forward when touched again.

(c) in FIG. 41 shows an example in which the hook 6161 is separated. When the hook 6161 is separated, the arrangement of the hanger body 610 may be switched into the second arrangement and the third arrangement.

The present disclosure may be modified and implemented in various forms, so that the scope of rights thereof is not limited to the above-described embodiments. Therefore, when the modified embodiment includes components of the patent claims of the present disclosure, it should be regarded as falling within the scope of the rights of the present disclosure.

## Claims

1. A laundry treating apparatus comprising:
a cabinet including a first inlet defined in a front surface thereof;
a first chamber located inside the cabinet and configured to accommodate laundry or goods therein via the first inlet;
an auxiliary chamber located at a lower side inside the cabinet and defining a space separated from the first chamber therein;
a door assembly configured to open and close the first inlet; and
a second chamber located inside the door assembly and defining therein a space separated from the first chamber and the auxiliary chamber to store laundry or goods therein,
wherein the first chamber includes:
a first area located on top of the auxiliary chamber; and
a second area located in front of the auxiliary chamber and configured to accommodate at least a portion of the second chamber when the door assembly closes the first inlet.

2. The laundry treating apparatus of claim 1, wherein along a front and rear direction of the cabinet, a length in the front and rear direction of the second area is equal to or smaller than a length in the front and rear direction of the first area.

3. The laundry treating apparatus of claim 1, further comprising a first chamber bottom surface forming a bottom surface of the first chamber,
wherein the bottom surface of the first chamber is formed to be stepped along a front and rear direction of the cabinet.

4. The laundry treating apparatus of claim 3, wherein the first chamber bottom surface includes:
a first bottom surface forming a bottom surface of the first area; and
a second bottom surface forming a bottom surface of the second area,
wherein a vertical level of the first bottom surface is higher than a vertical level of the second bottom surface.

5. The laundry treating apparatus of claim 4, wherein the second bottom surface faces at least a portion of a lower portion of the door assembly when the door assembly closes the first inlet.

6. The laundry treating apparatus of claim 1, wherein the door assembly includes:
an accommodating body including a second inlet defined in a front surface thereof to allow the second chamber defined therein to be in communication with the outside; and
a door of the second chamber configured to open and close the second inlet,
wherein at least a portion of the accommodating body is accommodated in the first area when the door assembly closes the first inlet.

7. The laundry treating apparatus of claim 6, wherein at least a portion of the door of the second chamber is made of a material allowing visible light to pass therethrough.

8. The laundry treating apparatus of claim 6, wherein a width of the accommodating body decreases rearwards from the second inlet.

9. The laundry treating apparatus of claim 6, further comprising:
a first hinge pivotably coupling the door assembly to the cabinet; and
a second hinge pivotably coupling the door of the second chamber to the accommodating body.

10. The laundry treating apparatus of claim 9, wherein the first hinge includes:
a first upper hinge located at an upper portion of the first chamber; and
a first lower hinge located at a lower portion of the first chamber,
wherein a portion of each of the first upper hinge and the first lower hinge is located to be spaced apart from both side surfaces of the first chamber,
wherein another portion of each of the first upper hinge and the first lower hinge is pivotably coupled to a rear surface of the accommodating body, spaced apart from both side surfaces of the accommodating body,
wherein the first hinge pivots to open the first inlet by extending the door assembly forward of the cabinet and then moving the door assembly along a width direction of the cabinet.

11. The laundry treating apparatus of claim 10, wherein the second hinge includes a plurality of second hinges,
wherein one end of each of the plurality of second hinges is located in the accommodating body,
wherein the other end of each of the plurality of second hinges is located on an inner surface of the door of the second chamber facing the second inlet in the door of the second chamber,
wherein the second hinge pivots such that an angle between the second inlet and the door of the second chamber increases or decreases.

12. The laundry treating apparatus of claim 9, wherein the first hinge includes a first link and a second link pivotably coupled to the cabinet and the accommodating body, but spaced apart from each other.

13. The laundry treating apparatus of claim 12, wherein the first link and the second link move the door assembly parallel to the first inlet when opening and closing the first inlet.

14. The laundry treating apparatus of claim 13, wherein the first hinge moves the door assembly forward to be away from the first inlet via pivoting of the first link and the second link, and then moves the door assembly parallel to the first inlet.

15. The laundry treating apparatus of claim 1, further comprising an opening/closing driver located on an inner surface of the first chamber and configured to push the door assembly to separate the door assembly from the first inlet.

16. The laundry treating apparatus of claim 15, wherein the opening/closing driver includes:
an opening/closing body forming an outer appearance;
an opening/closing motor disposed inside the opening/closing body; and
an opening/closing link connected to the opening/closing motor and configured to push the door assembly in a direction away from the first inlet when the opening/closing motor rotates.

17. The laundry treating apparatus of claim 1, further comprising:
an air supply located inside the auxiliary chamber and configured to circulate air in the first chamber;
an air intake port located on a bottom surface of the first chamber, in communication with the first chamber, and configured to suck air in the first chamber into the air supply; and
an air discharge port located on the bottom surface of the first chamber, in communication with the first chamber, and configured to discharge air that has passed through the air supply into the first chamber.

18. The laundry treating apparatus of claim 17, further comprising:
a first bottom surface forming a bottom surface of the first area; and
a second bottom surface forming a bottom surface of the second area,
wherein the air intake port and the air discharge port are located on the first bottom surface.

19. The laundry treating apparatus of claim 17, wherein the air intake port and the air discharge port are arranged along a width direction of the cabinet.

20. The laundry treating apparatus of claim 17, further comprising:
a steam supply located in the auxiliary chamber and configured to generate steam and supply steam to the first chamber; and
a steam discharge port located at a lower portion of the first chamber and configured to discharge steam into the first chamber.

21. The laundry treating apparatus of claim 20, wherein the steam discharge port is located closer to the air discharge port than to the air intake port.

22. The laundry treating apparatus of claim 20, further comprising:
a water supply tank configured to supply water to generate steam to the steam supply; and
a drain tank configured to store condensate generated in the air supply and the first chamber therein,
wherein the water supply tank and the drain tank are detachably inserted into the auxiliary chamber via a front surface of the auxiliary chamber.

23. The laundry treating apparatus of claim 22, wherein the water supply tank and the drain tank have lengths along a front and rear direction of the cabinet greater than lengths along a height direction of the cabinet.

24. The laundry treating apparatus of claim 22, wherein the air supply further includes a heat exchanger for dehumidifying and heating circulating air in the first chamber,
wherein the water supply tank and the drain tank are located upwardly of the heat exchanger.

25. The laundry treating apparatus of claim 1, further comprising a hanger body located at an upper side inside the first chamber and configured to hang the laundry thereon,
wherein the hanger body extends along a front and rear direction of the cabinet.

26. The laundry treating apparatus of claim 25, further comprising:
an air supply located in the auxiliary chamber and configured to circulate air in the first chamber;
an air intake port located at a lower portion of the first chamber, in communication with the first chamber, and configured to suck air in the first chamber into the air supply; and
an air discharge port located at the lower portion of the first chamber, in communication with the first chamber, and configured to discharge air that has passed through the air supply into the first chamber,
wherein the air intake port and the air discharge port are arranged to be spaced apart from each other in a width direction of the cabinet.

27. The laundry treating apparatus of claim 25, further comprising a hanger support supporting the hanger body to be movable in the front and rear direction.

28. The laundry treating apparatus of claim 27, further comprising:
a hanger bar fixing groove defined in one of the hanger support and the hanger body; and
a movement preventing protrusion disposed on the other of the hanger support and the hanger body and insertable into the hanger bar fixing groove depending on whether the door assembly is opened.

29. The laundry treating apparatus of claim 25, further comprising:
a laundry driver configured to generate a rotational force for a reciprocating movement of the hanger body; and
a power converter connected to the laundry driver and configured to allow the hanger body to perform the reciprocating movement along the front and rear direction.

30. The laundry treating apparatus of claim 25, further comprising a hanger mounting portion for hanging the laundry accommodated in the second chamber,
wherein a direction of the laundry hung on the hanger mounting portion is the same as a direction of the laundry hung on the hanger body.

31. The laundry treating apparatus of claim 6, further comprising an air circulator located inside the accommodating body and configured to circulate air in the second chamber.

32. The laundry treating apparatus of claim 31, further comprising an air treater located inside the accommodating body and configured to circulate air in the second chamber.

33. The laundry treating apparatus of claim 32, wherein the air circulator and the air treater are operatable even when the door assembly is opened.

34. The laundry treating apparatus of claim 32, further comprising an air supply disposed inside the auxiliary chamber and configured to circulate air in the first chamber,
wherein the air circulator and the air treater operate independently of an operation of the air supply.

35. The laundry treating apparatus of claim 31, wherein the air circulator includes a heater configured to heat circulating air in the second chamber to adjust a temperature of the second chamber.

36. The laundry treating apparatus of claim 32, wherein the air treater includes a dehumidifier for dehumidifying air circulating via the air treater.

37. The laundry treating apparatus of claim 1, wherein the door assembly further includes a shelf detachably disposed in the second chamber and supporting the laundry or the goods accommodated in the second chamber.

38. The laundry treating apparatus of claim 1, wherein a vertical dimension of the second area is greater than a vertical dimension of the first area.

39. The laundry treating apparatus of claim 1, wherein a vertical dimension of the first area is greater than a vertical dimension of the auxiliary chamber.

40. The laundry treating apparatus of claim 1, wherein a vertical dimension of a front surface of the door assembly is greater than a vertical dimension of the second area.

41. A laundry treating apparatus comprising:
a cabinet including a first inlet defined in a front surface thereof;
a first chamber located inside the cabinet and configured to accommodate laundry or goods therein via the first inlet;
an auxiliary chamber located at a lower side inside the cabinet and defining a space separated from the first chamber therein; and
a door assembly configured to open and close the first inlet and including a second chamber configured to accommodate laundry or goods therein,
wherein at least a portion of the second chamber is accommodated in a space defined between the first inlet and the auxiliary chamber when the door assembly closes the first inlet.

42. A laundry treating apparatus comprising:
a cabinet including a first inlet defined in a front surface thereof;
a first chamber located inside the cabinet and configured to accommodate laundry or goods therein via the first inlet;
an auxiliary chamber located at a lower side inside the cabinet and defining a space separated from the first chamber therein;
a door assembly including an accommodating body including a second inlet defined in a front surface thereof and forming a second chamber therein, and a door of the second chamber configured to open and close the second inlet, wherein the door assembly opens and closes the first inlet;
a first hinge configured to separate the door assembly from the first chamber and move the door assembly along a width direction of the cabinet to open the first inlet; and
a second hinge configured to move the door of the second chamber such that an angle between the door of the second chamber and a front surface of the accommodating body increases to open the second inlet.

43. The laundry treating apparatus of claim 42, wherein at least a portion of the second chamber is located in front of the auxiliary chamber inside the first chamber when the door assembly closes the first inlet.

44. A laundry treating apparatus comprising:
a cabinet including an inlet defined in a front surface thereof;
a door configured to open and close the inlet;
a first chamber located inside the cabinet and configured to accommodate laundry or goods therein via the inlet;
a hanger body disposed inside the first chamber, extending along a front and rear direction of the cabinet, and configured to hang the laundry thereon; and
a hanger support extending parallel to the hanger body along the front and rear direction of the cabinet and supporting the hanger body to be movable along the front and rear direction of the cabinet.

45. A laundry treating apparatus comprising:
a cabinet including an inlet defined in a front surface thereof;
a first chamber located inside the cabinet and configured to accommodate laundry or goods therein via the inlet;
a hanger body disposed inside the first chamber, extending along a front and rear direction of the cabinet, and configured to hang the laundry thereon; and
a hanger support extending parallel to the hanger body along the front and rear direction of the cabinet and supporting the hanger body to be movable,
wherein an arrangement of the hanger body and the hanger support is switchable into:
a first arrangement where the hanger body and the hanger support are coupled to each other such that each of both ends of the hanger body and each of both ends of the hanger support coincide with each other;
a second arrangement for separating the hanger body from the hanger support and moving the hanger body forward by a predetermined distance; and
a third arrangement for rotating the hanger body relative to the hanger support to arrange the hanger body and the hanger support perpendicular to each other.
